(19) Europäisches Patentamt
European Patent Office
Office européen des brevets

(11) EP 4 692 371 A1

(12) **EUROPEAN PATENT APPLICATION**
published in accordance with Art. 153(4) EPC

(43) Date of publication:
**11.02.2026 Bulletin 2026/07**

(21) Application number: **24779923.2**

(22) Date of filing: **22.03.2024**

(51) International Patent Classification (IPC):
***C12Q 1/6851*** *(2018.01)* ***A61K 39/395*** *(2006.01)*
***A61K 45/00*** *(2006.01)* ***A61P 35/00*** *(2006.01)*
***C07K 16/28*** *(2006.01)* ***C12Q 1/686*** *(2018.01)*
***C12Q 1/6869*** *(2018.01)* ***G01N 33/50*** *(2006.01)*
***G16H 20/10*** *(2018.01)*

(52) Cooperative Patent Classification (CPC):
**A61K 39/395; A61K 45/00; A61P 35/00; C07K 16/28; C12Q 1/6851; C12Q 1/686; C12Q 1/6869; G01N 33/50; G16H 20/10**

(86) International application number:
**PCT/JP2024/011202**

(87) International publication number:
**WO 2024/203804 (03.10.2024 Gazette 2024/40)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **24.03.2023 JP 2023047877**

(71) Applicants:
- **Zenick.Lab Corporation**
  **Tokyo, 142-0064 (JP)**
- **Showa Medical University**
  **Tokyo, 142-8555 (JP)**

(72) Inventors:
- **SUGINO Toru**
  **Tokyo 142-0064 (JP)**
- **TAKAYANAGI Daisuke**
  **Tokyo 142-0064 (JP)**
- **WADA Satoshi**
  **Tokyo 142-8555 (JP)**
- **TSUNODA Takuya**
  **Tokyo 142-8555 (JP)**

(74) Representative: **Klöckner, Christoph**
**df-mp Patentanwälte Rechtsanwälte PartG mbB**
**Theatinerstraße 16**
**80333 München (DE)**

# (54) METHOD FOR PREDICTING EFFICACY OF TREATMENT WITH IMMUNE CHECKPOINT INHIBITOR (ICI) ON SUBJECT, AND METHOD FOR TREATING TUMOR IN SUBJECT

(57) The present disclosure provides a method of predicting the effectiveness of treatment with an immune checkpoint inhibitor (ICI) in a subject. The present disclosure also provides a method of treating a tumor in a subject determined to have effectiveness. The effectiveness prediction can be performed on a blood sample from the subject.

EP 4 692 371 A1

## Description

TECHNICAL FIELD

The present disclosure relates to a method of predicting the effectiveness of treatment with an immune checkpoint inhibitor (ICI) in a subject. The present disclosure also relates to a method of treating a tumor in a subject determined to have effectiveness.

BACKGROUND ART

The application of immune checkpoint inhibitors to tumor treatment has brought about a revolutionary development in tumor treatment. However, there is a group of patients for whom a sufficient therapeutic effect is not observed even with immune checkpoint inhibitors. Since immune checkpoint inhibitors are expensive, if it were possible to identify patients who are expected to show a therapeutic effect and administer immune checkpoint inhibitors mainly to such patients, it would contribute to healthcare economics. Patent Document 1 discloses a method of predicting the effectiveness of an immune checkpoint inhibitor based on evaluation items consisting of a combination of PD-1 expression intensity and PD-1 expression ratio in Treg cells and CD8+ T cells. Patent Document 2 discloses a method of predicting the effectiveness of an immune checkpoint inhibitor based on RNA-Seq of a tumor.

PRIOR ART DOCUMENTS

PATENT DOCUMENTS

[PATENT DOCUMENT 1] WO2019/230919
[PATENT DOCUMENT 2] WO2018/231772

SUMMARY OF THE INVENTION

The present disclosure provides a method of predicting the effectiveness of a treatment with an immune checkpoint inhibitor (ICI) in a subject. In the present disclosure, the effectiveness of treatment with an ICI for the subject can be predicted based on gene expression in cells (e.g., peripheral blood mononuclear cells and monocytes) contained in the subject's peripheral blood. The present disclosure also provides a method of treating a tumor in a subject determined to have effectiveness.

According to the present disclosure, for example, the following inventions are provided:

(1) A method of predicting the effect of tumor treatment with an immune checkpoint inhibitor (ICI) in a subject, comprising:

determining the expression level of a gene including one or more genes selected from the gene group described in any of Tables 1 to 7 (preferably Table 1) in a sample (e.g., a sample containing monocytes such as peripheral blood mononuclear cells) obtained from the subject; and
using a prediction model constructed to be able to predict the therapeutic effect, or based on the determined expression level, predicting the effect of treatment with an ICI from the determined expression level,
wherein the prediction model is constructed based on the expression level of the gene including the one or more genes in a sample (e.g., a sample containing monocytes such as peripheral blood mononuclear cells) obtained from each of a patient group for whom ICI treatment is effective and a patient group for whom ICI treatment is not effective, and information regarding the effectiveness of ICI treatment.

(2) The method according to the above (1), wherein the one or more genes selected from the gene group described in Table 1 include any one or more genes from the gene group described in FIGS. 4A to 400.
(3) The method according to the above (1) or (2), wherein the one or more genes selected from the gene group described in Table 1 include one or more or all genes selected from the group consisting of CLEC12A, TMEM176A, and TMEM176B.
(4) The method according to any one of the above (1) to (3), wherein the one or more genes selected from the gene group described in Table 1 include five or more genes.
(5) The method according to any one of the above (1) to (4), wherein the one or more genes selected from the gene group described in Table 1 include 10 or more genes.

(6) The method according to any one of the above (1) to (5), wherein the one or more genes selected from the gene group described in Table 1 include 20 or more genes.

(7) The method according to any one of the above (1) to (6), wherein the prediction model is constructed by machine learning.

(8) The method according to any one of the above (1) to (6), wherein the prediction model is constructed by logistic regression.

(9) A method of treating a tumor in a subject, comprising:

administering a therapeutically effective amount of an immune checkpoint inhibitor (ICI) to the subject, wherein the subject is a subject for whom a therapeutic effect of an immune checkpoint inhibitor (ICI) is predicted to be effective by the method according to any one of the above (1) to (8).

(10) The method according to any one of the above (1) to (9), wherein the immune checkpoint inhibitor (ICI) is an antibody or a PD-1 binding fragment of an antibody that can bind to PD-1 and neutralize the binding between PD-1 and PD-L1.

(11) The method according to any one of the above (1) to (9), wherein the immune checkpoint inhibitor (ICI) is an antibody or a PD-1 binding fragment of an antibody that can bind to PD-L1 and neutralize the binding between PD-1 and PD-L1.

(12) An immune checkpoint inhibitor (ICI) or a pharmaceutical composition including an immune checkpoint inhibitor, for use in the method according to any one of the above (9) to (11).

(13) The invention according to any one of the preceding claims, wherein one or more genes selected from the gene group described in Table 1 include the gene groups described in any one of FIGS. 19 to 23.

(14) A method of predicting the action of a candidate substance on a patient's treatment responsiveness to immune checkpoint inhibitor treatment (ICI treatment), comprising:

determining the expression level of one or more genes, including those selected from the gene groups described in any of Tables 1 to 7, in a first sample obtained from the patient before administration of the candidate substance and a second sample obtained from the patient after administration of the candidate substance; and predicting the effect of the ICI treatment from the determined expression levels using a predictive model (e.g., that can be constructed by the above (1)) constructed to be able to predict the effect of the immune checkpoint inhibitor treatment;

wherein the predictive model is constructed based on the expression level of the one or more genes in a sample obtained from a patient group in which the ICI treatment is effective and a patient group in which the ICI treatment is not effective, and information regarding the effectiveness of the ICI treatment;

wherein an increase in the prediction result of the effectiveness of the ICI treatment in the first sample and the second sample indicates a possibility that the candidate substance enhances the effectiveness of the ICI treatment, and a decrease in the prediction result of the effectiveness of the ICI treatment in the first sample and the second sample indicates a possibility that the candidate substance reduces the effectiveness of the ICI treatment.

(15) The method according to (14) above, wherein the patient is a patient before ICI treatment.

(16) The method according to (14) above, wherein the patient is a patient during ICI treatment.

(17) The method according to any one of (14) to (16) above, wherein the candidate substance has an anti-tumor effect.

(18) The method according to any one of (14) to (16) above, wherein the candidate substance does not have an anti-tumor effect.

BRIEF EXPLANATION OF DRAWINGS

FIG. 1: The relationship between the prognosis of each patient treated with an immune checkpoint inhibitor (ICI) and the ratio of classical monocytes (Panel A) or non-classical monocytes (Panel B) in peripheral blood mononuclear cells is shown.

FIG. 2: A cytogram obtained by separating peripheral blood mononuclear cells based on the expression level of CD14 and the expression level of CD16 is shown.

FIG. 3A: The results of predicting the effectiveness of treatment with an ICI with a prediction model constructed by logistic regression analysis from the expression level of a single gene are shown.

FIG. 3B: Same as above.
FIG. 4A: The results of predicting the effectiveness of treatment with an ICI with a prediction model constructed by logistic regression analysis from the expression level of a single gene are shown.
FIG. 4B: Same as above.
FIG. 4C: Same as above.
FIG. 4D: Same as above.
FIG. 4E: Same as above.
FIG. 4F: Same as above.
FIG. 4G: Same as above.
FIG. 4H: Same as above.
FIG. 4I: Same as above.
FIG. 4J: Same as above.
FIG. 4K: Same as above.
FIG. 4L: Same as above.
FIG. 4M: Same as above.
FIG. 4N: Same as above.
FIG. 4O: Same as above.
FIG. 4P: Same as above.
FIG. 4Q: Same as above.
FIG. 4R: Same as above.
FIG. 4S: Same as above.
FIG. 4T: Same as above.
FIG. 4U: Same as above.
FIG. 4V: Same as above.
FIG. 4W: Same as above.
FIG. 4X: Same as above.
FIG. 4Y: Same as above.
FIG. 4Z: Same as above.
FIG. 4AA: Same as above.
FIG. 4BB: Same as above.
FIG. 4CC: Same as above.
FIG. 4DD: Same as above.
FIG. 4EE: Same as above.
FIG. 4FF: Same as above.
FIG. 4GG: Same as above.
FIG. 4HH: Same as above.
FIG. 4II: Same as above.
FIG. 4JJ: Same as above.
FIG. 4KK: Same as above.
FIG. 4LL: Same as above.
FIG. 4MM: Same as above.
FIG. 4NN: Same as above.
FIG. 5: The receiver operating characteristic curve (ROC), area under the curve (AUC), and other information for a prediction model constructed by logistic regression analysis based on the expression level of the three indicated genes are shown.
FIG. 6: The receiver operating characteristic curve (ROC), area under the curve (AUC), and other information for a prediction model constructed by logistic regression analysis based on the expression level of the ten indicated genes are shown.
FIG. 7: The receiver operating characteristic curve (ROC), area under the curve (AUC), and other information for a prediction model constructed by logistic regression analysis based on the expression level of the 20 indicated genes are shown.
FIG. 8: The receiver operating characteristic curve (ROC), area under the curve (AUC), and other information for a prediction model constructed by logistic regression analysis based on the expression level of all 160 genes are shown.
FIG. 9: The receiver operating characteristic curve (ROC), area under the curve (AUC), and other information for a prediction model constructed by decision tree analysis are shown.
FIG. 10: The receiver operating characteristic curve (ROC), area under the curve (AUC), and validation results for a prediction model constructed by a neural network are shown.
FIG. 11: The receiver operating characteristic curve (ROC), area under the curve (AUC), and other information for a prediction model constructed by the random forest method are shown.

FIG. 12: The receiver operating characteristic curve (ROC), area under the curve (AUC), and validation results for a prediction model constructed by the random forest method are shown.

FIG. 13: The receiver operating characteristic curve (ROC), area under the curve (AUC), and other information for a prediction model constructed by logistic regression analysis based on the expression level of the 30 indicated genes are shown.

FIG. 14: The receiver operating characteristic curve (ROC), area under the curve (AUC), and other information for a prediction model constructed by logistic regression analysis based on the expression level of the 40 indicated genes are shown.

FIG. 15: The receiver operating characteristic curve (ROC), area under the curve (AUC), and other information for a prediction model constructed by logistic regression analysis based on the expression level of the 45 indicated genes are shown.

FIG. 16: The receiver operating characteristic curve (ROC), area under the curve (AUC), and other information for a prediction model constructed by logistic regression analysis based on the expression level of the 50 indicated genes are shown.

FIG. 17: The receiver operating characteristic curve (ROC), area under the curve (AUC), and other information for a prediction model constructed by logistic regression analysis based on the expression level of the 55 indicated genes are shown.

FIG. 18: The receiver operating characteristic curve (ROC), area under the curve (AUC), and other information for a prediction model constructed by logistic regression analysis based on the expression level of the 60 indicated genes are shown.

FIG. 19: The extraction of genes from the 160 genes by the stepwise method and the receiver operating characteristic curve (ROC), area under the curve (AUC), and other information for a prediction model constructed by the extracted 10 genes are shown.

FIG. 20: The extraction of genes from the 160 genes by LASSO regression and the receiver operating characteristic curve (ROC), area under the curve (AUC), and other information for a prediction model constructed by the extracted 47 genes are shown.

FIG. 21: A list of the 27 genes showing a p-value less than 0.0001 among the 47 genes extracted by LASSO regression, and the receiver operating characteristic curve (ROC), area under the curve (AUC), and other information for a prediction model constructed by the 27 genes are shown.

FIG. 22: The extraction of genes from the 160 genes by elastic net regression and the receiver operating characteristic curve (ROC), area under the curve (AUC), and other information for a prediction model constructed by the extracted 45 genes are shown.

FIG. 23: A list of the 17 genes showing a p-value less than 0.0001 among the 45 genes extracted by elastic net regression, and the receiver operating characteristic curve (ROC), area under the curve (AUC), and other information for a prediction model constructed by the 17 genes are shown.

[EMBODIMENT OF THE INVENTION]

As used herein, the term "subject" refers to an individual who undergoes a blood test. The subject may be an animal. The subject may be a fish, a bird, a reptile, a mammal, or an amphibian. The mammal may be a dog, cat, cow, horse, sheep, pig, hamster, mouse, squirrel, goat, mule, camel, alpaca, and primates such as a monkey, gorilla, chimpanzee, bonobo, or human. The subject is preferably a human.

As used herein, "antibody" refers to a protein that has a structure in which two heavy chains (H chains) and two light chains (L chains) are associated and stabilized by a pair of disulfide bonds. A heavy chain consists of a heavy chain variable region VH, heavy chain constant regions CH1, CH2, CH3, and a hinge region located between CH1 and CH2. A light chain consists of a light chain variable region VL and a light chain constant region CL. Among these, the variable region fragment (Fv) consisting of VH and VL directly participates in antigen binding and is the region that gives diversity to the antibody. The antigen-binding region consisting of VL, CL, VH, and CH1 is called the Fab region, and the region consisting of the hinge region, CH2, and CH3 is called the Fc region.

Among the variable regions, the regions that directly contact the antigen are particularly highly variable and are called complementarity-determining regions (CDRs). The parts other than the CDRs that have relatively few mutations are called framework regions (FRs). The variable regions of the light chain and heavy chain each have three CDRs, which are called heavy chain CDRs 1 to 3 and light chain CDRs 1 to 3, respectively, in order from the N-terminal side. As used herein, "anti-A antibody" means an antibody that binds to A. An anti-A antibody can specifically bind to A. As used herein, "specifically binds to A" means that the antibody has a stronger binding affinity for A than for other proteins.

The antibody may be a monoclonal antibody or a polyclonal antibody. The antibody may also be any isotype of IgG, IgM, IgA, IgD, or IgE. It may be produced by immunizing a non-human animal such as a mouse, rat, hamster, guinea pig, rabbit, goat, sheep, horse, cow, donkey, camel, llama, alpaca, or chicken, or it may be a recombinant antibody, a chimeric antibody, a humanized antibody, a fully humanized antibody, or the like. A "chimeric antibody" refers to an antibody in which fragments of antibodies derived from different species are linked.

A "humanized antibody" refers to an antibody in which a corresponding position of a human antibody is replaced with an amino acid sequence characteristic of a non-human-derived antibody. Examples include an antibody that has heavy chain CDRs 1 to 3 and light chain CDRs 1 to 3 of an antibody produced by immunizing a mouse or rat, and all other regions including the four framework regions (FRs) of the heavy chain and light chain are derived from a human antibody. Such an antibody is sometimes called a CDR-grafted antibody. The term "humanized antibody" may also include a human chimeric antibody.

A "human chimeric antibody" is an antibody in which the constant region of a non-human-derived antibody is replaced with the constant region of a human antibody. In a human chimeric antibody, from the viewpoint of enhancing ADCC activity, for example, the subtype of the human antibody used for the constant region can be IgG1.

As used herein, an "antigen-binding fragment" refers to a fragment of an antibody that has antigen-binding affinity. Specifically, it includes, but is not limited to, Fab consisting of VL, VH, CL, and CH1 regions; F(ab')2 in which two Fabs are linked by a disulfide bond in the hinge region; Fv consisting of VL and VH; scFv which is a single-chain antibody in which VL and VH are linked by an artificial polypeptide linker; and bispecific antibodies such as diabody type, scDb type, tandem scFv type, and leucine zipper type.

As used herein, an "immune checkpoint inhibitor" means a drug that activates immunity by releasing the suppression of immune cells caused by immune checkpoint molecules. Examples of immune checkpoint molecules include programmed cell death-1 (PD-1), CTLA-4, T-cell immunoglobulin domain and mucin domain-3 (TIM-3), lymphocyte activation gene 3 (LAG-3), and V-type immunoglobulin domain-containing suppressor of T-cell activation (VISTA). The immune checkpoints they are responsible for are called the PD-1 immune checkpoint system, the CTLA-4 immune checkpoint system, the TIM-3 immune checkpoint system, the LAG-3 immune checkpoint system, and the VISTA immune checkpoint system. The immune checkpoint inhibitor can, for example, bind to an immune checkpoint molecule or its ligand to inhibit the function of the immune checkpoint. For example, the PD-1 immune checkpoint can be inhibited by inhibiting the binding of PD-1 to PD-L1 or PD-L2. Also, the CTLA-4 immune checkpoint can be inhibited by inhibiting the binding of CTLA-4 to CD80 or CD86. Also, the TIM-3 immune checkpoint can be inhibited by inhibiting the binding of TIM-3 to galectin-9. Also, the LAG-3 immune checkpoint can be inhibited by inhibiting the binding of LAG-3 to MHC class II molecules. Also, the VISTA immune checkpoint can be inhibited by inhibiting the binding of VISTA to VSIG-3/IGSF11. In this way, one or more immune checkpoints selected from the group consisting of the PD-1 immune checkpoint system, the CTLA-4 immune checkpoint system, the TIM-3 immune checkpoint system, the LAG-3 immune checkpoint system, and the VISTA immune checkpoint system can be inhibited. An antibody that inhibits the binding of two proteins can bind to a receptor or a ligand. For example, an antibody that inhibits the PD-1 immune checkpoint can be an antibody selected from the group consisting of anti-PD-1 antibodies, anti-PD-L1 antibodies, and anti-PD-L2 antibodies (e.g., nivolumab, pembrolizumab, avelumab, atezolizumab, and durvalumab). Also, an antibody that inhibits the CTLA-4 immune checkpoint can be an antibody selected from the group consisting of anti-CTLA-4 antibodies, anti-CD80 antibodies, and anti-CD86 antibodies (e.g., ipilimumab and tremelimumab). Also, an antibody that inhibits the TIM-3 immune checkpoint can be an antibody selected from the group consisting of anti-TIM-3 antibodies and anti-galectin-9 antibodies (e.g., MGB453). Also, an antibody that inhibits the VISTA immune checkpoint can be an antibody selected from the group consisting of anti-VISTA antibodies and anti-VSIG-3/IGSF11 antibodies (e.g., JNJ-61610588). As an immune checkpoint inhibitor, in addition to an antibody, an antigen-binding fragment of an antibody can also be used.

As used herein, "cancer" means a malignant tumor. A tumor includes a benign tumor and a malignant tumor. Examples of tumors include solid tumors (e.g., epithelial tumors, non-epithelial tumors) and tumors in hematopoietic tissues. More specifically, solid tumors that can be treated by the pharmaceutical of the present disclosure include, for example, gastrointestinal cancer (e.g., esophageal cancer, stomach cancer, colon cancer, rectal cancer), lung cancer (e.g., small cell carcinoma, non-small cell carcinoma), splenic cancer, kidney cancer, liver cancer, pancreatic cancer, bile duct cancer, thymic cancer, thyroid cancer, adrenal gland cancer, prostate cancer, bladder cancer, ureter cancer, ovarian cancer, uterine cancer (e.g., endometrial cancer, cervical cancer), bone cancer, skin cancer, sarcoma (e.g., Kaposi's sarcoma), melanoma, neurocytoma, blastoma (e.g., neuroblastoma), brain tumor, cancer of unknown primary origin, and cancers due to recurrence and metastasis of these solid tumors. Tumors that can be treated by the pharmaceutical of the present disclosure also include adenocarcinoma, squamous cell carcinoma, and non-squamous cell carcinoma. Tumors in hematopoietic tissues that can be treated by the pharmaceutical of the present disclosure include leukemia (e.g., acute myeloid leukemia (AML), chronic myeloid leukemia (CML), acute lymphoblastic leukemia (ALL), chronic lymphocytic leukemia (CLL), adult T-cell leukemia-lymphoma (ATL), myelodysplastic syndrome (MDS)), lymphoma (e.g., T-lymphoma, B-lymphoma, Hodgkin's lymphoma, non-Hodgkin's lymphoma), myeloma (multiple myeloma), and cancers due to recurrence and metastasis of these tumors.

As used herein, a "predictive model" is a tool created based on statistical analysis of data to predict the outcome of an event to be predicted. A predictive model for treatment effectiveness is obtained from features in a sample obtained from a subject and information regarding treatment effectiveness. In the construction of a predictive model, it is often performed to formulate the relationship between the features in a sample obtained from a subject and information regarding treatment effectiveness. In a predictive model utilizing machine learning, treatment effectiveness in a subject can be predicted using a moving average method, an exponential smoothing method, or the like. When the gene expression level used for prediction is a single gene, the predictive model may simply determine the effectiveness by whether or not it exceeds a specific reference value. When the gene expression level used for prediction is two or more genes, the predictive model may be constructed by, for example, logistic regression. In a certain embodiment, being effective for treatment may mean that the progression-free survival (PFS) is 2 years or more. In a certain embodiment, being effective for treatment may mean that complete response (CR), partial response (PR), and stable disease (SD) are shown by the treatment. This classification can be based on RECIST (e.g., RECIST 1.1). In a certain embodiment, not being effective for treatment may mean that progression (PD) is shown for the treatment. However, whether it is effective or not may be defined appropriately by a person skilled in the art. For example, only CR may be evaluated as effective, or CR and PR may be evaluated as effective. In a certain embodiment, not being effective for treatment may mean that the progression-free survival (PFS) is less than 6 months. In a certain embodiment, not being effective for treatment may be any case other than being effective. The definition of treatment effectiveness may differ between solid tumors and blood tumors.

The present specification, "peripheral blood mononuclear cell" (PBMC) means a mononuclear cell separated from the subject's peripheral blood, and typically includes monocytes and lymphocytes. PBMCs can generally be obtained by removing plasma components, red blood cells, platelets, and granulocytes, etc., from collected peripheral blood.

In the present specification, prediction can be performed by a prediction model with an area under the receiver operating characteristic curve (ROC) (AUC) for the effectiveness of ICI treatment of more than 0.5. The ROC can be drawn on a two-dimensional graph with sensitivity on the vertical axis and 1-specificity (false positive rate) on the horizontal axis. The prediction model is preferably constructed such that sensitivity > (1-specificity) at any false positive rate. The prediction model is not particularly limited, but examples include a prediction model based on a simple threshold, a prediction model based on weighted scoring, a prediction model based on logistic regression analysis, and a prediction model based on machine learning (based on a prediction model by a neural network, a prediction model by decision tree analysis, a prediction model by a random forest method, a prediction model by logistic regression analysis, etc.). By inputting gene expression level data from peripheral blood or PBMC to the obtained prediction model, a determination result regarding the effectiveness of ICI treatment can be obtained. The prediction model may establish a reference value and predict effectiveness by comparing with the reference value. The prediction model may be a weighted scoring model, that is, a model where the expression level of each individual gene (or a value obtained by standardizing the expression level) is multiplied by a weighting coefficient and summed to form a score. The prediction model may predict effectiveness by comparing the score with a reference value.

An example of scoring in the prediction model:

[MATH 1]

$$S = \sum_{t=0}^{n} A_t \times Ex_t \quad \cdots \text{(Formula 1)}$$

In the above (Formula 1), Ext is the expression level of the t-th gene among the n types of genes to be measured, or a value obtained by standardizing or normalizing the expression level; At is the weighting coefficient for the expression level of the t-th gene; and S is the score. The weighting coefficient can be a positive or negative numerical value. Standardization means scaling the data so that the mean is 0 and the variance is 1. Specifically, the standardized score is calculated as (expression level - mean value) / variance. Normalization means scaling so that the minimum value is 0 and the maximum value is 1. Specifically, the normalized score is calculated as (expression level - minimum value) / (maximum value - minimum value) .

The reference value can be determined by various methods. When it is predicted that effectiveness is high if the expression level or score exceeds a reference value, increasing the reference value increases specificity but decreases sensitivity, whereas decreasing the reference value decreases specificity and increases sensitivity. Therefore, the reference value should be determined as appropriate depending on the purpose and situation, etc., and the preferred reference value may change from time to time. For example, if you want to limit ICI administration to as few patients as possible, you can increase the reference value, and if you want to administer ICI widely to as many patients as possible, you can decrease the reference value.

In a certain embodiment, when it is predicted that effectiveness is high if the expression level or score exceeds a first reference value, the first reference value can be any value selected from the group consisting of the mean, the top 40%, the top 33%, the top 25%, the top 20%, the top 15%, the top 10%, and the top 5% of the corresponding expression level or score in the patient group for whom ICI treatment is not effective, and values between these two values. Also, the first reference value can be any value selected from the group consisting of the mean, the top 60%, the top 70%, the top 80%, and the top 90% of the corresponding expression level or score in the patient group for whom ICI treatment is effective, and values between these two values. In a certain embodiment, the first reference value can be a value between the mean in the patient group for whom ICI treatment is effective and the mean in the patient group for whom ICI treatment is not effective.

In a certain embodiment, when it is predicted that effectiveness is high if the expression level or score is lower than a second reference value, the second reference value can be any value selected from the group consisting of the mean, the top 40%, the top 33%, the top 25%, the top 20%, the top 15%, the top 10%, and the top 5% of the corresponding expression level or score in the patient group for whom ICI treatment is effective, and values between these two values. Also, the second reference value can be any value selected from the group consisting of the mean, the top 60%, the top 70%, the top 80%, and the top 90% of the corresponding expression level or score in the patient group for whom ICI treatment is not effective, and values between these two values. In a certain embodiment, the second reference value can be a value between the mean in the patient group for whom ICI treatment is effective and the mean in the patient group for whom ICI treatment is not effective.

In a certain embodiment, when it is predicted that effectiveness is low if the expression level or score exceeds a third reference value, the third reference value can be any value selected from the group consisting of the mean, the top 40%, the top 33%, the top 25%, the top 20%, the top 15%, the top 10%, and the top 5% of the corresponding expression level or score in the patient group for whom ICI treatment is effective, and values between these two values. Also, the third reference value can be any value selected from the group consisting of the mean, the top 60%, the top 70%, the top 80%, and the top 90% of the corresponding expression level or score in the patient group for whom ICI treatment is not effective, and values between these two values. In a certain embodiment, the third reference value can be a value between the mean in the patient group for whom ICI treatment is effective and the mean in the patient group for whom ICI treatment is not effective.

In a certain embodiment, when it is predicted that effectiveness is low if the expression level or score is lower than a fourth reference value, the fourth reference value can be any value selected from the group consisting of the mean, the top 40%, the top 33%, the top 25%, the top 20%, the top 15%, the top 10%, and the top 5% of the corresponding expression level or score in the patient group for whom ICI treatment is not effective, and values between these two values. Also, the fourth reference value can be any value selected from the group consisting of the mean, the top 60%, the top 70%, the top 80%, and the top 90% of the corresponding expression level or score in the patient group for whom ICI treatment is effective, and values between these two values. In a certain embodiment, the fourth reference value can be a value between the mean in the patient group for whom ICI treatment is effective and the mean in the patient group for whom ICI treatment is not effective.

In this way, by comparing the expression level or score with a reference value, the responsiveness of the subject to ICI treatment and the effectiveness of ICI treatment in the subject can be evaluated. It is possible that a subject predicted to be effective for ICI treatment by one method of the present disclosure may be predicted to be ineffective (low effectiveness) by another method. In such a case, a doctor, etc., can decide whether or not to carry out ICI treatment on the subject depending on the situation.

According to the present disclosure, a method of predicting the effect of tumor treatment with an immune checkpoint inhibitor (ICI) in a subject is provided. According to the present disclosure, a method for predicting the effect of tumor treatment with an immune checkpoint inhibitor (ICI) in a subject is provided. According to the present disclosure, a method for predicting the effect of tumor treatment with an immune checkpoint inhibitor (ICI) in a subject {provided that medical acts on humans are excluded} is provided. In a certain embodiment, the prediction method of the present disclosure can predict the therapeutic effect using a prediction model on an electronic computer. The prediction method of the present disclosure can be a method that is industrially applicable. The prediction method of the present disclosure belongs to in vitro diagnostics. The method of the present disclosure can be performed either or both in vitro and in silico. The method of the present disclosure can include determining the expression level of a gene including one or more genes selected from the gene group described in Table 1 in a sample obtained from the subject. In a certain preferred embodiment, the sample includes monocytes.

The prediction method of the present disclosure can include determining the expression level of a gene including one or more genes selected from the gene group described in Table 1 in a sample (e.g., a sample comprising monocytes), for example, a sample comprising monocytes, such as blood cells, e.g., peripheral blood mononuclear cells, e.g., monocytes (e.g., classical monocytes and non-classical monocytes and intermediate monocytes), obtained from the subject. Blood cells, e.g., peripheral blood mononuclear cells, e.g., monocytes (e.g., classical monocytes and non-classical monocytes and intermediate monocytes) are contained in peripheral blood. Blood cells, e.g., peripheral blood mononuclear cells, e.g., monocytes (e.g., classical monocytes and non-classical monocytes and intermediate monocytes) are preferably isolated from peripheral blood before measuring the expression level. The expression level of the gene is preferably the expression level of messenger RNA (mRNA). The expression level of mRNA can be quantified by a conventional method. The expression level of mRNA can be determined by various methods such as quantitative PCR, quantitative RT-PCR, and RNAseq by next-generation sequencing (NGS). mRNA is typically determined based on the amount of cDNA, on the premise that the amount of synthesized cDNA reflects the amount of mRNA, after cell lysis or disruption and total RNA extraction, and then cDNA synthesis using mRNA as a template. If the expression level of mRNA and the expression level of a protein correlate, the amount of the protein may be measured as the expression level of the gene. The expression level of a protein can be determined by various methods such as ELISA, Western blot, and dot blot. The expression of mRNA may be determined at the single cell level. The measurement of mRNA expression at the single cell level can be performed after synthesizing cDNA using a primer having a sequence unique to each cell (barcode sequence). The primer may also include a sequence unique to each patient (index sequence). The barcode sequence and index sequence can be designed and used with reference to US 9,260,753B.

The prediction method of the present disclosure may include predicting the effect of treatment with an ICI from the determined expression level using a prediction model constructed to be able to predict the effect of treatment with an ICI. The prediction method of the present disclosure may include outputting the prediction result of the effect of treatment with an ICI from the determined expression level using a prediction model constructed to be able to predict the effect of treatment with an ICI. The prediction model can be constructed based on the expression level of a gene including the one or more genes in peripheral blood mononuclear cells obtained from each of a patient group for whom ICI treatment is effective and a patient group for whom ICI treatment is not effective, and information regarding the effectiveness of ICI treatment. Whether ICI treatment was effective for a specific patient when creating training data and/or validation data can be determined by a medical professional such as a doctor. The output of the prediction result can be performed on a display, on paper, in a recording medium (especially a volatile memory, or preferably a non-volatile memory), or to another device at a communication destination online.

Therefore, according to the present disclosure, a method of predicting the effect of tumor treatment with an immune checkpoint inhibitor (ICI) in a subject is provided, comprising:

determining the expression level of a gene including one or more genes selected from the gene group described in any of Tables 1 to 7 in blood cells, preferably in peripheral blood mononuclear cells, more preferably in monocytes, obtained from the subject; and
using a prediction model constructed to be able to predict the therapeutic effect, predicting the effect of treatment with an ICI from the determined expression level.

Also according to the present disclosure, a method of predicting the effect of tumor treatment with an immune checkpoint inhibitor (ICI) in a subject is provided, comprising:

inputting the expression level of a gene including one or more genes selected from the gene group described in any of Tables 1 to 7 in blood cells, preferably in peripheral blood mononuclear cells, more preferably in monocytes, obtained from the subject to an electronic computer; and
using a prediction model constructed to be able to predict the therapeutic effect, causing the electronic computer to predict the effect of treatment with an ICI from the inputted expression level and output the result.

The prediction model can be constructed based on the expression level of a gene including the one or more genes in peripheral blood mononuclear cells (more preferably monocytes) obtained from each of a patient group for whom ICI treatment is effective and a patient group for whom ICI treatment is not effective, and information regarding the effectiveness of ICI treatment. As described above, various prediction models can be used as the prediction model. The prediction model can be operated on an electronic computer. As the prediction model, a prediction model with an area under the receiver operating characteristic curve (ROC) (AUC) of 0.6 or more, 0.65 or more, 0.7 or more, 0.75 or more, 0.8 or more, 0.9 or more, 0.95 or more, 0.96 or more, 0.97 or more, 0.98 or more, or 0.99 or more for ICI treatment effectiveness can be preferably used. A prediction model with an AUC closer to 1 has higher sensitivity and fewer false positives. As the prediction model, a prediction model with an area under the receiver operating characteristic curve (ROC) (AUC) of 0.6 or more, 0.65 or more, 0.7 or more, 0.75 or more, 0.8 or more, 0.9 or more, 0.95 or more, 0.96 or more, 0.97 or more, 0.98 or more, or 0.99 or more for ICI treatment non-effectiveness can also be preferably used. A prediction model with an AUC closer to 1 has higher sensitivity and fewer false positives.

The gene whose expression level is determined may include one or more, two or more, three or more, four or more, five or more, 10 or more, 15 or more, 20 or more, 25 or more, 30 or more, 35 or more, 40 or more, 45 or more, 50 or more, 55 or more, 60 or more, 65 or more, 70 or more, 75 or more, 80 or more, 85 or more, 90 or more, 95 or more, 100 or more, 110 or more, 120 or more, 130 or more, 140 or more, 150 or more, or 160 genes selected from the gene group described in Table 1. Basically, the more genes selected from the gene group described in Table 1, the more preferable. The genes described in FIGS. 4A to 4NN are all genes for which a prediction model could be constructed using evaluation with a single gene. Therefore, these genes can be used for prediction with a single gene, but a prediction model may also be constructed by combining two or more of these genes. For such a constructed prediction model, it is preferable to input the expression levels of all genes used to create the prediction model. From the gene group described in Table 1, gene sets with high prediction accuracy can be extracted by various methods. Such methods are not particularly limited, but examples include stepwise method, LASSO regression, and elastic net regression.

The gene selected from the gene group described in Table 1 preferably includes CLEC12A.

The gene selected from the gene group described in Table 1 preferably includes TMEM176A, and for example, includes CLEC12A and TMEM176A.

The gene selected from the gene group described in Table 1 preferably includes TMEM176B, and for example, includes CLEC12A and TMEM176B, or CLEC12A and TMEM176A and TMEM176B.

The gene selected from the gene group described in Table 1 preferably includes one or more, two or more, three or more, four or more, five or more, or all genes selected from the group consisting of TMEM176A, CLEC12A, S100B, CD52, SH2D2A, SIDT2, GTF3C1, ZNF266, MS4A7, and LST1. The AUC of the prediction model at this time can be, for example, 0.6 or more, 0.7 or more, 0.8 or more, 0.85 or more, 0.86 or more, 0.87 or more, 0.88 or more, or 0.89 or more.

The gene selected from the gene group described in Table 1 preferably includes one or more, two or more, three or more, four or more, five or more, 10 or more, 15 or more, or all genes selected from the group consisting of TMEM176A, CLEC12A, S100B, CD52, SH2D2A, SIDT2, GTF3C1, ZNF266, MS4A7, LST1, SH2B3, AIF1, LAIR2, RETN, LYPD2, RNF14, UBE2F, CTSS, FGL2, and HAVCR2. The AUC of the prediction model at this time can be, for example, 0.6 or more, 0.7 or more, 0.8 or more, 0.85 or more, 0.9 or more, 0.91 or more, 0.92 or more, 0.93 or more, or 0.94 or more.

The gene selected from the gene group described in Table 1 preferably includes one or more, two or more, three or more, four or more, five or more, 10 or more, 15 or more, 20 or more, 25 or more, or all genes selected from the group consisting of CLEC12A, TMEM176A, CD52, S100B, SH2D2A, SIDT2, GTF3C1, ZNF266, MS4A7, LST1, SH2B3, AIF1, LAIR2, RETN, LYPD2, DIP2A, RNF14, UBE2F, CTSS, FGL2, HAVCR2, LILRB2, TMEM176B, CLU, SELL, PHTF2, PSAP, SLA2, ID2, and HCK. The AUC of the prediction model at this time can be, for example, 0.6 or more, 0.7 or more, 0.8 or more, 0.85 or more, 0.9 or more, 0.91 or more, 0.92 or more, 0.93 or more, 0.94 or more, 0.95 or more, or 0.96 or more.

The gene selected from the gene group described in Table 1 preferably includes one or more, two or more, three or more, four or more, five or more, 10 or more, 15 or more, 20 or more, 30 or more, or all genes selected from the group consisting of CLEC12A, TMEM176A, CD52, S100B, SH2D2A, SIDT2, GTF3C1, ZNF266, MS4A7, LST1, SH2B3, AIF1, LAIR2, RETN, LYPD2, DIP2A, RNF14, UBE2F, CTSS, FGL2, HAVCR2, LILRB2, TMEM176B, CLU, SELL, PHTF2, PSAP, SLA2, ID2, HCK, EGR1, CD247, GNLY, SH2D1B, PRF1, IL1RN, PECAM1, KIR2DL3, UBE2G2, and C1QA. The AUC of the prediction model at this time can be, for example, 0.6 or more, 0.7 or more, 0.8 or more, 0.85 or more, 0.9 or more, 0.91 or more, 0.92 or more, 0.93 or more, 0.94 or more, 0.95 or more, 0.96 or more, or 0.97.

The gene selected from the gene group described in Table 1 preferably includes one or more, two or more, three or more, four or more, five or more, 10 or more, 15 or more, 20 or more, 30 or more, 40 or more, or all genes selected from the group consisting of CLEC12A, TMEM176A, CD52, S100B, SH2D2A, SIDT2, GTF3C1, ZNF266, MS4A7, LST1, SH283, AIF1, LAIR2, RETN, LYPD2, DIP2A, RNF14, UBE2F, CTSS, FGL2, HAVCR2, LILRB2, TMEM176B, CLU, SELL, PHTF2, PSAP, SLA2, ID2, HCK, EGR1, CD247, GNLY, SH2D1B, PRF1, IL1RN, PECAM1, KIR2DL3, UBE2G2, C1QA, NR4A2, HK2, XRCC5, CXCR4, LYST, CSF1R, ANKRD28, WARS, HBEGF, and CAT. The AUC of the prediction model at this time can be, for example, 0.6 or more, 0.7 or more, 0.8 or more, 0.85 or more, 0.9 or more, 0.91 or more, 0.92 or more, 0.93 or more, 0.94 or more, 0.95 or more, 0.96 or more, or 0.97 or more.

The gene selected from the gene group described in Table 1 preferably includes one or more, two or more, three or more, four or more, five or more, 10 or more, 15 or more, 20 or more, 30 or more, 40 or more, 50 or more, or all genes selected from the group consisting of CLEC12A, TMEM176A, CD52, S100B, SH2D2A, SIDT2, GTF3C1, ZNF266, MS4A7, LST1, SH2B3, AIF1, LAIR2, RETN, LYPD2, DIP2A, RNF14, UBE2F, CTSS, FGL2, HAVCR2, LILRB2, TMEM176B, CLU, SELL, PHTF2, PSAP, SLA2, ID2, HCK, EGR1, CD247, GNLY, SH2D1B, PRF1, ILLRN, PECAM1, KIR2DL3, UBE2G2, C1QA, NR4A2, HK2, XRCC5, CXCR4, LYST, CSF1R, ANKRD28, WARS, HBEGF, CAT, CYBB, CTSL, PRMT2, SLC39A8, FCGR3A, GPR183, STAB1, CASP1, EGR2, and FOLR3. The AUC of the prediction model at this time can be, for example, 0.6 or more, 0.7 or more, 0.8 or more, 0.85 or more, 0.9 or more, 0.91 or more, 0.92 or more, 0.93 or more, 0.94 or more, 0.95 or more, 0.96 or more, 0.97 or more, or 0.98 or more.

In this way, by inputting the expression levels of the corresponding genes into the obtained prediction model, the effect of treatment with an ICI can be predicted from the determined expression levels. It is easy for a person skilled in the art to construct a prediction model to be able to predict the effect of treatment with an ICI from the expression levels of the above genes, based on the description in this specification and technical common knowledge.

In a certain embodiment, the ICI can be, for example, an inhibitor of an immune checkpoint selected from the group consisting of PD-1-related immune checkpoints, CTLA-4-related immune checkpoints, TIM-3-related immune checkpoints, LAG-3-related immune checkpoints, and VISTA-related immune checkpoints.

The ICI can, for example, bind to an immune checkpoint molecule or its ligand and inhibit the function of the immune checkpoint. For example, the PD-1 immune checkpoint can be inhibited by inhibiting the binding of PD-1 to PD-L1 or PD-L2. Also, the CTLA-4 immune checkpoint can be inhibited by inhibiting the binding of CTLA-4 to CD80 or CD86. Also, the TIM-3 immune checkpoint can be inhibited by inhibiting the binding of TIM-3 to Galectin-9. Also, the LAG-3 immune checkpoint can be inhibited by inhibiting the binding of LAG-3 to MHC Class II molecules. Also, the VISTA immune checkpoint can be inhibited by inhibiting the binding of VISTA to VSIG-3/IGSF11. In this way, one or more immune checkpoints selected from the group consisting of PD-1 immune checkpoints, CTLA-4 immune checkpoints, TIM-3 immune checkpoints, LAG3 immune checkpoints, and VISTA immune checkpoints can be inhibited. An antibody that inhibits the binding of two proteins can bind to a receptor or a ligand. For example, an antibody that inhibits the PD-1 immune checkpoint can be an antibody selected from the group consisting of anti-PD-1 antibodies, anti-PD-L1 antibodies, and anti-PD-L2 antibodies (e.g., Nivolumab, Pembrolizumab, Avelumab, Atezolizumab, and Durvalumab). Also, an antibody that inhibits the CTLA-4 immune checkpoint can be an antibody selected from the group consisting of anti-CDLA-4 antibodies, anti-CD80 antibodies, and anti-CD86 antibodies (e.g., Ipilimumab and Tremelimumab). Also, an antibody that inhibits the TIM-3 immune checkpoint can be an antibody selected from the group consisting of anti-TIM-3 antibodies and anti-Galectin-9 antibodies (e.g., MGB453). Also, an antibody that inhibits the VISTA immune checkpoint can be an antibody selected from the group consisting of anti-VISTA antibodies and anti-VSIG-3/IGSF11 antibodies (e.g., JNJ-61610588).

In a certain preferred embodiment, the blood cell is a peripheral blood mononuclear cell. In a certain preferred embodiment, the blood cell is a monocyte. In a certain preferred embodiment, the blood cell is a classical monocyte. Classical monocytes are characterized by $CD14^{+}CD16^{low}$. In a certain preferred embodiment, the blood cell is a non-classical monocyte. Non-classical monocytes are characterized by $CD14^{low}CD16^{+}$. In a certain preferred embodiment, the blood cell is an intermediate monocyte. Intermediate monocytes are characterized by $CD14^{+}CD16^{+}$. An example of CD14 and CD16 expression for classical monocytes, non-classical monocytes, and intermediate monocytes is shown in the cytogram in FIG. 2. In a certain preferred embodiment, the blood cell is a non-classical monocyte, and the gene to be measured any one or more genes described in Table 3. In a certain preferred embodiment, the blood cell is a pool of non-classical monocytes and classical monocytes, and the gene to be measured is any one or more genes described in Table 4. In a certain preferred embodiment, the blood cell is an intermediate monocyte, and the gene to be measured is any one or more genes described in Table 5. In a certain preferred embodiment, the blood cell is a classical monocyte, and the gene to be measured is any one or more genes described in Table 6. In a certain preferred embodiment, the monocyte is a monocyte described in the monocyte type of Table 7, and the gene to be measured is the gene in the corresponding row. These blood cells are isolated, concentrated, or purified from blood.

In this way, the effectiveness of treatment with an ICI for a subject can be predicted.

A subject determined to be effective for ICI treatment can be a preferred candidate for ICI treatment. Therefore, according to the present disclosure, a method of treating a tumor in a subject determined to be effective for ICI treatment is provided, comprising administering a therapeutically effective amount of an ICI to the subject. In contrast, a subject determined to be ineffective or not determined to be effective for ICI treatment may not be a preferred candidate for ICI treatment. Therefore, such a subject can be provided with tumor treatment other than ICI.

In the present disclosure, the use of a gene expression testing tool in the manufacture of a kit for use in the above prediction method is provided. Gene expression testing can be performed using mRNA quantification techniques such as microarrays, quantitative PCR, and RNASeq by next-generation sequencing. Examples of gene expression testing tools include reagents that can measure the expression level of mRNA. Examples of reagents that can measure the expression level of mRNA include primers for nucleic acid amplification and probes that bind to mRNA (e.g., nucleic acid probes). The gene expression testing tool can be suitable for the respective application of the mRNA quantification technique. Specifically, the probe may be provided in the form of a microarray, with the probe immobilized on the array, e.g., a microarray, or may be provided in the form of a conjugate with a fluorescent reagent. In addition, gene expression testing can be performed using evaluation methods well known to those skilled in the art, such as ELISA. Examples of gene expression testing tools include antibodies that bind to each protein, labeled secondary antibodies that recognize the antibodies, and reagents that develop the label.

In the present disclosure, the use of an ICI in the manufacture of a medicament for use in the above treatment method is provided.

In the present disclosure, an ICI for use in the above treatment method is provided. In the present disclosure, a pharmaceutical composition including an ICI for use in the above treatment method is provided.

A prediction model created based on information on the effectiveness for a specific ICI can be preferably used for predicting the effectiveness of the specific ICI. Therefore, the specific ICI can be preferably administered to a patient determined to be effective for treatment with the specific ICI. However, a prediction model may also be created based on information on the effectiveness of multiple ICIs, and the created prediction model may be used to predict the effectiveness for treatment with the multiple ICIs.

A prediction model created based on the expression level of a specific gene is preferably used to predict ICI treatment effectiveness based on the expression level of the specific gene. Also, a prediction model created based on the expression levels of multiple specific genes is preferably used to predict ICI treatment effectiveness based on the expression levels of the multiple specific genes.

In a certain embodiment, the peripheral blood mononuclear cell can be an isolated monocyte. In a certain embodiment, the monocyte can be a classical monocyte. In a certain embodiment, the monocyte can be a non-classical monocyte. In a certain embodiment, the monocyte can be a monocyte having intermediate properties (e.g., CD14-positive and CD16-positive) of classical and non-classical monocytes.

In the present disclosure, the method of predicting the effect of treatment with an ICI may include determining the ratio of classical monocytes in peripheral blood mononuclear cells. In the present disclosure, the method of predicting the effect of treatment with an ICI may include determining the ratio of non-classical monocytes in peripheral blood mononuclear cells. This is because the smaller the ratio of classical monocytes, the better the prognosis, and the larger the ratio of non-classical monocytes, the better the prognosis.

According to the present disclosure, an apparatus for predicting the effect of treatment with an ICI in a subject is provided. The apparatus for predicting the effect of treatment with an ICI of the present disclosure comprises:

one or more processors; and
one or more memories operably and electrically connected to the one or more processors, the memory having stored instructions,
wherein when the instructions are executed by at least one of the one or more processors, the apparatus receives information on the expression level of one or more genes described in any of Tables 1 to 7 in peripheral blood mononuclear cells obtained from the subject, processes the received expression level information with a prediction model, and outputs a prediction result of the effect of treatment with an ICI, and the prediction model is one constructed to be able to predict the effectiveness of the treatment based on information regarding the effectiveness of the treatment in each of a patient group for whom ICI treatment was effective and a patient group for whom ICI treatment was not effective, and information on the expression level of the one or more genes in the patients, which is linked to the information. Also according to the present disclosure, the use of the above apparatus for predicting the effect of treatment with an ICI in a subject is provided. Also according to the present disclosure, a method of predicting the effect of treatment with an ICI in a subject using the above apparatus is provided. The prediction model can have an AUC of, for example, 0.6 or more, 0.7 or more, 0.8 or more, 0.9 or more, 0.95 or more, 0.96 or more, 0.97 or more, or 0.98 or more. The prediction model is not particularly limited, but examples include a prediction model based on a simple threshold, a prediction model based on weighted scoring, a prediction model based on logistic regression analysis, and a prediction model based on machine learning (based on a prediction model by a neural network, a prediction model by decision tree analysis, a prediction model by a random forest method, a prediction model by logistic regression analysis, etc.).

## EXAMPLES

### Materials and Methods

### Experimental Model and Subject Details

Peripheral blood samples were obtained from lung cancer patients (n=8) before treatment using a BD Vacutainer® CPT™ tube (BD Biosciences). Cases were pathologically diagnosed with lung cancer and received anti-PD-1 antibody therapy (Nivolumab (240 mg) intravenous every 2 weeks or Pembrolizumab (200 mg) intravenous every 3 weeks) for 4 or more cycles at Showa University Hospital from January 2017 to April 2019.

Progression-free survival (PFS) was defined as the period from anti-PD-1 antibody treatment until disease progression or death from any cause. Responders (n=4) were characterized as patients who showed complete response or partial response by computed tomography (CT) and achieved a PFS of 2 years or more. Non-responders (n=4) refers to patients who showed no objective tumor response and had a PFS of less than 6 months.

### Peripheral Blood Mononuclear Cell (PBMC) Separation

Whole blood was collected in an 8 mL heparin sodium-added CPT vacutainer and inverted several times so that the heparin sodium anticoagulant and blood were well-mixed. After blood collection, the tube was spun to separate the sample into layers, and peripheral blood mononuclear cells (PBMCs) were isolated according to the following protocol. The upper layer was separated into a mixture of plasma and PBMC, the middle layer was polyester resin, and the lower layer was red blood cells and granulocytes. After centrifugation, the CPT was gently inverted several times to resuspend the PBMCs and plasma, and decanted into a sterile 50 mL conical tube. Phosphate buffered saline (PBS, Thermo Scientific) was added to adjust the final volume to 50 mL. The capped 50 mL conical tube was inverted to mix and centrifuged at 250×g for 10 minutes at room temperature. The supernatant was carefully aspirated, and the cell pellet was gently resuspended in 10 mL of fresh PBS, followed by centrifugation at 250×g for 10 minutes at room temperature. After this centrifugation step, the supernatant was carefully aspirated again without disturbing the cell pellet, and the pelleted PBMCs were gently resuspended in 3 mL of fetal bovine serum (FBS, HyClone). 500 μL of the cells were dispensed into 6 pieces of 2 mL cryotubes pre-filled with 500 μL of BAMBANKER (NIPPON GENETICS). The cryotubes were stored at -80°C for 24 hours in a BICELL (Nippon Freezer), and then transferred to liquid nitrogen for long-term storage. The remaining amount of PBMCs in FBS was retained and used for counting and viability evaluation.

Monocyte Sorting

PBMCs were stained with the following antibodies at 4°C for 30 minutes. Staining was performed with CD14-BV650 (#563419, BD Biosciences) and CD16-FITC (#555406, BD Biosciences) for 30 minutes at 4°C. FACS sorting of PBMCs was performed using a CytoFLEX SH800S (Sony). The stained cells were then gated into $CD14^{high}$ CD16- (classical) and $CD14^{low}$ $CD16^{+}$ (non-classical) subsets by conventional flow cytometry, and these gated cells were FACS sorted. The sorted cells were suspended in Bambanker and frozen at -80°C.

Preparation of Single-Cell Suspension

After thawing the frozen cells, cell viability and density were confirmed. The cells were diluted to a density of 4 x $10^4$ cells/ml in a final dispensing mix containing PBS, 100x dilution, recombinant RNase inhibitor, and 1% BSA.

Full-length Single-Cell RNA-seq with ICELL8 System

A Takara ICELL8 5,184 nano-well chip (350V chip) was used with the Full-length SMART-Seq ICELL8 Reagent Kit. The cell suspension was fluorescently labeled with a viability stain, Hoechst 33342, and propidium iodide (NucBlue Cell Stain Reagent, Thermo Fisher Scientific) for 20 minutes at 37°C before being dispensed onto the 350V chip. Using CellSelect Software (Takara Bio), wells containing single and viable cells were visualized and selected. By this operation, 302 cells were obtained, which each contained monocytes from all patients without unevenness. Next, single-cell cDNA and sequencing libraries were prepared using the SMART-seq2 protocol[1]. Full-length cDNA was synthesized via a barcoded primer (UMI) with a poly(dT) end in a one-step RT-PCR reaction. In addition to Terra polymerase and reaction buffer, P5 indexing primers necessary for subsequent library preparation were dispensed into all wells with different indexes. Transposase enzyme and reaction buffer (Tn5 mix) were dispensed into the selected wells. P7 indexing primers were dispensed into the wells. Illumina libraries were amplified, extracted from the chip, and pooled. The pooled libraries were purified and size-selected to an average library size of 500 bp using Agencourt AMPure XP magnetic beads (Beckman Coulter). The libraries were quality-checked with an Agilent 2200 TapeStation. The final libraries were sequenced with 150 paired-end cycles using a Novaseq 6000 system (Illumina), obtaining an average of approximately 20 million reads per cell.

Pre-processing of Data from ICELL8

Raw sequence files (bcl) were converted to a single fastq file for each method using Illumina bc12fastq software (v2.20.0.422). Each fastq file was demultiplexed and analyzed using the Cogent NGS analysis pipeline (CogentAP, version 1.5, Takara Bio inc.). Cogent demux assigns reads to cells based on the cell barcodes listed in the Well-list. Subsequently, by the "cogent analyze" wrapper function, (1) read trimming with cutadapt[2] (version 3.4), (2) genome alignment to the Homo sapiens genome GRCh38 with STAR[3] (version 2.7.8a) were performed. Preliminary analyses were conducted by using featureCounts[4] (version 2.0.0), including (3) read counting for Homo sapiens gene exons, genomes, and mitochondrial regions from the ENSEMBL gene annotation version 103 (https://www.ensembl.org/Homo_sapiens/Info/Index), and (4) aggregating gene reads into a gene matrix with the number of reads expressed for each gene in each cell. The raw gene matrix underwent quality control (QC) filtering for cells and genes using the following parameters: (a) for cells, only those with at least 10,000 reads associated with at least 300 different genes, and (b) for genes, only those with at least 100 reads mapped from at least 3 different cells.

Gene Marker Detection

To determine gene markers expressed in responder and non-responder samples, the QC-filtered read count matrix was used as input, and genes differentially expressed between responder sample cells and non-responder cells were determined using the Wilcoxon rank-sum and signed-rank tests, with p-values adjusted by the Benjamini-Hochberg method. For each individual differential expression result, a rank score for each gene was calculated using the formula: rank = $-\log_{10}(\text{pval}) * \log_2 FC$. Here, pval is the raw p-value and $\log^2 FC$ is the $\log^2$ fold change.

Unsupervised Cell Clustering

Dimensionality reduction and visualization were performed using the Uniform Manifold Approximation and Projection (UMAP) algorithm with optimal principal components (PC) selected from an elbow plot, using Takara Bio's Cogent NGS discovery software v1.5 (CogentDS) R package. Furthermore, unsupervised graph-based clustering was performed using CogentDS to classify cells into specific clusters. For each graph-based clustering, gene markers were detected. The detected gene sets were plotted as a heatmap.

Differential Gene Expression Analysis

A non-parametric Wilcoxon rank-sum test performed with CogentDS was used to test for differential expression (DE) of each gene between the various single-cell groups specified in this text. Unless otherwise specified, each comparison was limited to genes that showed both an estimated $\log_2$ fold change (log_fc) > +0.5 and detectable expression in more than 10% of the cells in one of the two groups being compared. The p-value adjusted for false discovery rate (FDR) (padj) was calculated using the Benjamini-Hochberg method to correct for multiple hypothesis testing. Differentially expressed genes were defined as having an FDR-adjusted p-value (padj) < 0.05 and $|\log_2$(fold change)$| \geq 0.5$. Genes detected with a count < 10 in less than 30 cells were removed. Note that Padj is calculated as P-value * N / i, where N represents the number of genes and i represents the rank from the lowest p-value. A cluster heatmap of gene expression was created with the Subio platform (Subio Inc., Aichi, Japan). The prediction model was constructed using a logistic regression (LR) classifier with JMP (version 16.0, SAS Institute Inc., Cary, NC, USA).

Statistical Analysis

Statistical analysis was performed using JMP (version 16.0, SAS Institute Inc., Cary, NC, USA). The threshold for statistical significance was defined as p-value < 0.05.

Results

Monocytes with $CD14^{high}$ $CD16^-$ (classical monocytes) and $CD14^{low}$ $CD16^+$ (non-classical monocytes) were each separated from isolated PBMCs by sorting, and the ratio of classical monocytes and the ratio of non-classical monocytes in the PBMCs were investigated, respectively. The relationship between each ratio obtained above and the overall survival (OS) of patients who received immune checkpoint inhibitor treatment was plotted on a graph (see FIGS. 1A and B). As shown in FIG. 1, it became clear that the smaller the number of classical monocytes, the better the OS, and the larger the number of non-classical monocytes, the better the OS.

PBMCs were sorted based on CD14 and CD16 expression to obtain a cytogram with the vertical axis as CD16 expression intensity and the horizontal axis as CD14 expression intensity (see FIG. 2). As shown in FIG. 2, monocytes were broadly classified into $CD14^{high}$ CD16- (classical monocytes), $CD14^{low}$ $CD16^+$ (non-classical monocytes), and intermediate monocytes (intermediate) that show intermediate properties. The intermediate monocytes were obtained as a subset of a group of monocytes that were thought to be non-classical monocytes, and were clearly different from classical monocytes.

PBMCs were collected from four patients for whom immune checkpoint inhibitors were effective (responders: patients with progression-free survival (PFS) of 2 years or more; CR) and four patients for whom they were not effective (non-responders: patients with progression-free survival (PFS) of less than 6 months; PD), and classical and non-classical monocytes were obtained from each PBMC and subjected to single-cell RNA-Seq analysis. There were 41 million reads (about 72%) that mapped to a single location. Therefore, it was considered that there was no problem with the quality of the sequencing.

160 genes showing differential expression between responders and non-responders were picked up. The picked-up genes are genes expressed in monocytes and are summarized in Table 1. In Table 1, gene notation, gene name, NCBI registration number, and reference number for each gene in this specification are associated. In other tables, each gene is identified by referring to the reference number in Table 1. Tables 2 to 7 also show genes that are differentially expressed in each cell type between responders (CR) and non-responders (PD). Gene expression was normalized by the Count per million (CPM) method. When the median value of the normalized gene was higher than the background, the median value was indicated as CR, and when it was lower, the median value was indicated as PD.

By logistic regression analysis, it was confirmed whether each of the 160 genes could distinguish responders from non-responders with a single gene. A representative example is as shown in FIGS. 3A and 3B and 4A to 400. As shown in FIGS. 3A and 3B and4A to 400, these genes could distinguish responders from non-responders with a single gene. The logistic regression analysis was performed using JMP pro 16. Specifically, a univariate logistic regression analysis was performed using the response/non-response as the objective variable and the gene expression data for each gene in all monocyte data as the explanatory variable. For each gene, the significance and Akaike's information criterion (AIC) were calculated, and a gene that showed a significant difference and a small Akaike's information criterion was considered to be a gene more strongly related to the therapeutic effect. Multivariate logistic regression analysis for creating a prediction model was performed using response/non-response as the objective variable and, for genes with a small AIC in all monocytes, using gene expression data in ascending order of AIC as explanatory variables. For all prediction models, a Receiver Operating Characteristic (ROC) curve and Area under the curve (AUC) were calculated. Monocytes migrate to tumor tissue and differentiate into macrophages. This is thought to change the tumor environment to inflammatory or immunosuppressive. This is thought to change the effectiveness of ICI against the tumor. Therefore, it is considered that the method of the present invention can be used in principle to predict the effectiveness of any ICI.

A logistic regression analysis was performed using expression data of multiple genes. FIG. 5 shows the results of a logistic regression analysis based on the expression of the 3 genes shown in the upper panel table, FIG. 6 shows the results of a logistic regression analysis based on the expression of the 10 genes shown in the upper panel table, and FIG. 7 shows the results of a logistic regression analysis based on the expression of the 20 genes shown in the upper panel table. In all cases, cells derived from responders and cells derived from non-responders could be favorably determined. FIG. 8 shows the results of a logistic regression analysis based on the expression of all 160 genes shown in Table 1. In FIG. 8, the AUC became 1, and cells derived from responders and cells derived from non-responders could be completely and correctly distinguished. Logistic regression analysis was performed based on the gene expression of 30 genes (FIG. 13), 40 genes (FIG. 14), 45 genes (FIG. 15), 50 genes (FIG. 16), 55 genes (FIG. 17), and 60 genes (FIG. 18), and in all cases, cells derived from responders and cells derived from non-responders could be favorably determined. In this way, cells derived from responders and cells derived from non-responders could be distinguished based on the expression of each of the identified genes, and by combining the expression of multiple genes, cells derived from responders and cells derived from non-responders could be favorably distinguished. It is understood that the number of genes to be combined for evaluation can be determined as appropriate depending on the needs.

Decision tree analysis was performed. The results were as shown in FIG. 9. With decision tree analysis, cells derived from responders and cells derived from non-responders could also be favorably determined.

A machine learning model using a neural network was created. The data was divided into training data and validation data, a learning model was created with the dataset containing the training data, and the dataset containing the validation data (which was not included in the training data) was analyzed using the obtained learning model. As a result, as shown in FIG. 10, the machine learning model also favorably determined cells derived from responders and cells derived from non-responders. Neural network modeling was performed using JMP Pro 16. It was performed using the response/non-response as the objective variable and the 160 gene expression data in all monocytes as the explanatory variables. A 1-layer Tanh function was used as the activation function for modeling, and the number of nodes in the hidden layer was set to 3. The maximum number of training epochs and the number of tours were both set to 50, and the overfitting penalty and convergence criterion were set to 0.001 and 0.00001, respectively, to prevent the constructed model from overfitting to the learning model. The random K-fold method was adopted as the validation method, and the coefficient of determination R2 (R-squared) and the root of average squared error (RASE) were used as indicators to evaluate the prediction accuracy of the constructed model.

A machine learning model using a random forest was created. A learning model was created with the dataset containing the training data, and the training data was analyzed using the obtained learning model. As a result, as shown in FIG. 11, the machine learning model also favorably determined cells derived from responders and cells derived from non-responders. The data was also divided into training data and validation data, a learning model was created with the dataset containing the training data, and the dataset containing the validation data (which was not included in the training data) was analyzed using the obtained learning model. As a result, as shown in FIG. 12, the machine learning model also favorably determined cells derived from responders and cells derived from non-responders. Random forest modeling was performed using JMP Pro 16. It was performed using the response/non-response as the objective variable and the 160 gene expression data in all monocytes as the explanatory variables. The number of trees to be created by bootstrapping was set to 100, the number of features to be extracted per branch was set to 110, the minimum size of a branch was set to 10, and the maximum size was set to 2000.

TABLE 1

| TABLE 1: List of 160 genes and reference numbers for each gene in this specification | | | |
|---|---|---|---|
| genes | Gene description | NCBI gene ID | Ref. No. |
| CLEC12A | C-type lectin domain family 12 member A [Source:HGNC Symbol;Acc:HGNC:31713] | 160364 | 1 |
| TMEM176A | transmembrane protein 176A [Source:HGNC Symbol ; Acc: HGNC: 24930] | 55365 | 2 |

(continued)

| TABLE 1: List of 160 genes and reference numbers for each gene in this specification | | | |
|---|---|---|---|
| genes | Gene description | NCBI gene ID | Ref. No. |
| CD52 | CD52 molecule [Source:HGNC Symbol ;Acc :IIGNC: 1804] | 1043 | 3 |
| S100B | S100 calcium binding protein B [Source:]HGNC Symbol;Acc:HGNC:10500] | 6285 | 4 |
| SH2D2A | SH2 domain containing 2A [Source:HGNC Symbol;Acc:HGNC:10821] | 9047 | 5 |
| SIDT2 | SID1 transmembrane family member 2 [Source:HGNC Symbol;Acc:HGNC:242721 | 51092 | 6 |
| GTF3C1 | general transcription factor IIIC subunit 1 [Source:HGNC Symbol:Acc:HGNC:4664] | 2975 | 7 |
| ZNf266 | zinc finger protein 266 [Source:HGNC Symbol ;Acc:HGNC: 13059] | 10781 | 8 |
| MS4A7 | membrane spanning 4-domains A7 [Source:NCBI gene;Acc:58475] | 58475 | 9 |
| LST1 | leukocyte specific transcript 1 [Source:HGNC Symbol;Acc:HGNC:14189] | 7910 | 10 |
| SH2B3 | SH2B adaptor protein 3 [Source:HGNC Symbol;Acc:HGNC:296051 | 10019 | 11 |
| AIF1 | allograft inflammatory factor 1 [Source:HGNC Symbol;Acc:/HGNC:352] | 199 | 12 |
| LAIR2 | leukocyte associated immunoglobulin like receptor 2 [Source:HGNC Symbol;Acc:HGNC:6478] | 3904 | 13 |
| RETN | resistin [Source:HGNC Symbol;Acc:HGNC:20389] | 56729 | 14 |
| LYPD2 | LY6/PLAUR domain containing 2 [Source:HGNC Symbol;Acc:HGNC:25215] | 137797 | 15 |
| DIP2A | disco interacting protein 2 homolog A [Source:HGNC Symbol;Acc:HGNC:17217] | 23181 | 16 |
| RNF14 | ring finger protein 14 [Source:HGNC Symbol ;Acc:HGNC: 10058] | 9604 | 17 |
| UBE2F | ubiquitin conjugating enzyme E2 F (putative) [Source:IIGNC Symbol;Acc:IIGNC:12480] | 140739 | 18 |
| CTSS | cathepsin S [Source:HGNC Symbol; Acc:HGNC: 2545] | 1520 | 19 |
| FGL2 | fibrinogen like 2 [Source:HGNC Symbol;Acc:HGNC:3696] | 10875 | 20 |
| HAVCR2 | hepatitis A virus cellular receptor 2 [Source:HGNC Symbol;Acc:HGNC:18437] | 84868 | 21 |
| LILRB2 | leukocyte immunoglobulin like receptor B2 [Source:HGNC Symbol ;Acc:HGNC:6606] | 10288 | 22 |
| TMEM176B | transmembrane protein 176B [Source:HGNC Symbol:Acc:HGNC:29596] | 28959 | 23 |
| CLU | clusterin [Source:HGNC Symbol ;Acc:HGNC:2095] | 1191 | 24 |
| SELL | selectin L [Source:HGNC Symbol;Acc:1-IGNC:10720] | 6402 | 25 |
| PHTF2 | putative homeodomain transcription factor 2 [Source:HGNC Symbol;Acc:HGNC:13411] | 57157 | 26 |
| PSAP | prosaposin [Source:IIGNC Symbol ;Acc:HGNC:9498] | 5660 | 27 |
| SLA2 | Src like adaptor 2 [Source:HGNC Symbol;Acc:HGNC:17329] | 84174 | 28 |
| ID2 | inhibitor of DNA binding 2 [Source:HGNC Symbol;Acc:HGNC:5361] | 3398 | 29 |
| HCK | HCK proto-oncogene, Src family tyrosine kinase [Source:HGNC Symbol;Acc:HGNC:4840] | 3055 | 30 |

(continued)

| TABLE 1: List of 160 genes and reference numbers for each gene in this specification | | | |
|---|---|---|---|
| genes | Gene description | NCBI gene ID | Ref. No. |
| EGR1 | early growth response 1 [Source:HGNC Symbol; Acc: HGNC: 3238] | 1958 | 31 |
| CD247 | CD247 molecule [Source:HGNC Symbol;Acc:HGNC:1677] | 919 | 32 |
| GNLY | granulysin [Source:HGNC Symbol;Acc:HGNC:4414] | 10578 | 33 |
| SH2D1B | SH2 domain containing 1B [Source:HGNC Symbol;Acc:HGNC:30416] | 117157 | 34 |
| PRF1 | perforin 1 [Source:HGNC Symbol;Acc:HGNC:9360] | 5551 | 35 |
| IL1RN | interleukin 1 receptor antagonist [Source:HGNC Symbol; Acc: HGNC: 6000] | 3557 | 36 |
| PECAM1 | platelet and endothelial cell adhesion molecule 1 [Source:HGNC Symbol;Acc:HGNC:8823] | 5175 | 37 |
| KIR2DL3 | killer cell immunoglobulin like receptor, two Ig domains and long cytoplasmic tail 3 [Source:HGNC Symbol;Acc:HGNC:6331] | 3804 | 38 |
| UBE2G2 | ubiquitin conjugating enzyme E2 G2 [Source:HGNC Symbol ;Acc: HGNC: 12183] | 7327 | 39 |
| C1QA | complement Clq A chain [Source:HGNC Symbol ;Acc:HGNC: 1241] | 712 | 40 |
| NR4A2 | nuclear receptor subfamily 4 group A member 2 [Source:HGNC Symbol;Acc:HGNC:7981] | 4929 | 41 |
| IIK2 | hexokinase 2 [SourceHGNC Symbol;Acc:HGNC:4923] | 3099 | 42 |
| XRCC5 | X-ray repair cross complementing 5 [Source:HGNC Symbol;Acc:HGNC:12833] | 7520 | 43 |
| CXCR4 | C-X-C motif chemokine receptor 4 [Source:HGNC Symbol;Acc:HGNC:2561] | 7852 | 44 |
| LYST | lysosomal trafficking regulator [Source:HGNC Symbol ;Acc:HGNC: 1968] | 1130 | 45 |
| CSF1R | colony stimulating factor 1 receptor [Source:HGNC Symbol;Acc:HGNC:2433] | 1436 | 46 |
| ANKRD28 | ankyrin repeat domain 28 [Source:HGNC Symbol;Acc:HGNC:29024] | 23243 | 47 |
| WARS1 | tryptophanyl-tRNA synthetase 1 [Source:HGNC Symbol;Acc:HGNC:12729] | 7453 | 48 |
| HBEGF | heparin binding EGF like growth factor [Source:HGNC Symbol;Acc:HGNC:3059] | 1839 | 49 |
| CAT | catalase [Source:HGNC Symbol;Acc:HGNC:1516] | 847 | 50 |
| CYBB | cytochrome b-245 beta chain [Source:HGNC Symbol;Acc:HGNC:2578] | 1536 | 51 |
| CTSL | cathepsin L [Source:HGNC Symbol;Acc:HGNC:2537] | 1514 | 52 |
| PRMT2 | protein arginine methyltransferase 2 [Source:HGNC Symbol;Acc:HGNC:5186] | 3275 | 53 |
| SLC39A8 | solute carrier family 39 member 8 [Source:HGNC Symbol;Acc:HGNC:20862] | 64116 | 54 |
| FCGR3A | Fc fragment of IgG receptor IIIa [Source:HGNC Symbol;Acc:HGNC:3619] | 2214 | 55 |
| GPR183 | G protein-coupled receptor 183 [Source:HGNC Symbol;Acc:HGNC:3128] | 1880 | 56 |
| STAB1 | stabilin 1 [Source:HGNC Symbol;Acc:HGNC:18628] | 23166 | 57 |

(continued)

| TABLE 1: List of 160 genes and reference numbers for each gene in this specification | | | |
|---|---|---|---|
| genes | Gene description | NCBI gene ID | Ref. No. |
| CASP1 | caspase 1 [Source:HGNC Symbol;Acc:14GNC:1499] | 834 | 58 |
| EGR2 | early growth response 2 [Source:HGNC Symbol;Acc:HGNC:3239] | 1959 | 59 |
| FOLR3 | folate receptor gamma [Source:HGNC Symbol;Acc:HGNC:3795] | 2352 | 60 |
| YWIIAQ | tyrosine 3-monooxygenase/tryptophan 5-monooxygenase activation protein theta [Source:HGNC Symbol;Acc:HGNC:12854] | 10971 | 61 |
| 0TUD1 | OTU deubiquitinase 1 [Source:HGNC Symbol;Acc:HGNC:27346] | 220213 | 62 |
| BHLHE40 | basic helix-loop-helix family member e40 [Source:HGNC Symbol;Acc:HGNC:1046] | 8553 | 63 |
| F2R | coagulation factor II thrombin receptor [Source:HGNC Symbol;Acc:HGNC:3537] | 2149 | 64 |
| CLEC4F | C-type lectin domain family 4 member F [Source:HGNC Symbol;Acc:HGNC:25357] | 165530 | 65 |
| MPHOSPH9 | M-phase phosphoprotein 9 [Source:HGNC Symbol ;Acc:IIGNC:7215] | 10198 | 66 |
| ADM | adrenomedullin [Source:HGNC Symbol;Acc:HGNC:259] | 133 | 67 |
| ISCU | iron-sulfur cluster assembly enzyme [Source:HGNC Symbol;Acc:HGNC:29882] | 23479 | 68 |
| RGCC | regulator of cell cycle [Source:HGNC Symbol;Acc:HGNC:20369] | 28984 | 69 |
| UBQLN1 | ubiquilin 1 [Source:IIGNC Symbol ;Acc :HGNC: 12508] | 29979 | 70 |
| TOPORS | TOP1 binding arginine/serine rich protein [Source:HGNC Symbol;Acc:HGNC:21653] | 10210 | 71 |
| MYOM2 | myomesin 2 [Source:HGNC Symbol;Acc:HGNC:7614] | 9172 | 72 |
| SLC2A3 | solute carrier family 2 member 3 [Source:HGNC Symbol;Acc:HGNC:11007] | 6515 | 73 |
| TPM4 | tropomyosin 4 [Source:IIGNC Symbol;Acc:HGNC:12013] | 7171 | 74 |
| SGK1 | serum/glucocorticoid regulated kinase 1 [Source:HGNC Symbol ;Acc:HGNC: 10810] | 6446 | 75 |
| VPS29 | VPS29 retromer complex component [Source:HGNC Symbol;Acc:HGNC:14340) | 51699 | 76 |
| ADRB2 | adrenoceptor beta 2 [Source:HGNC Symbol;Acc:HGNC:286] | 154 | 77 |
| MAPRE2 | microtubule associated protein RP/EB family member 2 [Source:HGNC Symbol;Acc:HGNC:6891] | 10982 | 78 |
| KIR3DL1 | killer cell immunoglobulin like receptor, three Ig domains and long cytoplasmic tail 1 [Source:HGNC Symbol;Acc:1-HGNC:6338] | 3811 | 79 |
| FOSL2 | FOS like 2, AP-1 transcription factor subunit [Source:HGNC Symbol;Acc:HGNC:3798] | 2355 | 80 |
| STIP1 | stress induced phosphoprotein 1 [Source:HGNC Symbol;Acc:HGNC:11387] | 10963 | 81 |
| CUL5 | cull in 5 [Source:HGNC Symbol;Acc:HGNC:2556] | 8065 | 82 |
| RBM14 | RNA binding motif protein 14 [Source:HGNC Symbol;Acc:HGNC:14219] | 10432 | 83 |
| TSEN54 | tRNA splicing endonuclease subunit 54 [Source:HGNC Symbol;Acc:HGNC:27561] | 283989 | 84 |
| RPN2 | ribophorin II [Source:HGNC Symbol;Acc:HGNC:10382] | 6185 | 85 |

(continued)

| TABLE 1: List of 160 genes and reference numbers for each gene in this specification | | | |
|---|---|---|---|
| genes | Gene description | NCBI gene ID | Ref. No. |
| THEMIS2 | thymocyte selection associated family member 2 [Source:HGNC Symbol;Acc:HGNC:16839] | 9473 | 86 |
| MYLIP | myosin regulatory light chain interacting protein [Source:HGNC Symbol;Acc:HGNC:21155] | 29116 | 87 |
| ITGA5 | integrin subunit alpha 5 [Source:HGNC Symbol;Acc:HGNC:6141] | 3678 | 88 |
| CD63 | CD63 molecule [Source:HGNC Symbol;Acc:HGNC:1692] | 967 | 89 |
| BN1P2 | BCL2 interacting protein 2 [Source:HGNC Symbol;Acc:HGNC:1083] | 663 | 90 |
| CSNK2A2 | casein kinase 2 alpha 2 [Source:HGNC Symbol;Acc:HGNC:2459] | 1459 | 91 |
| CCL2 | C-C motif chemokine ligand 2 [Source:HGNC Symbol;Acc:HGNC: 10618] | 6347 | 92 |
| BRIX1 | biogenesis of ribosomes BRX1 [Source:HGNC Symbol;Acc:HGNC:24170] | 55299 | 93 |
| FOX01 | forkhead box 01 [Source:HGNC Symbol;Acc:HGNC:3819] | 2308 | 94 |
| EREG | epiregulin [Source:HGNC Symbol;Acc:HGNC:3443] | 2069 | 95 |
| STAT1 | signal transducer and activator of transcription 1 [Source:HGNC Symbol;Acc:HGNC:11362] | 6772 | 96 |
| CD14 | CD14 molecule [Source:HGNC Symbol;Acc:HGNC:1628] | 929 | 97 |
| PPP2R5C | protein phosphatase 2 regulatory subunit B'gamma [Source:HGNC Symbol;Acc:HGNC:9311] | 5527 | 98 |
| EMP1 | epithelial membrane protein 1 [Source:HGNC Symbol;Acc:HGNC:3333] | 2012 | 99 |
| CRY2 | cryptochrome circadian regulator 2 [Source:HGNC Symbol;Acc:HGNC:2385] | 1408 | 100 |
| PRAME | preferentially expressed antigen in melanoma [Source:HGNC Symbol;Acc:HGNC:9336] | 23532 | 101 |
| RBBP4 | RB binding protein 4, chromatin remodeling factor [Source:HGNC Symbol;Acc:HGNC:9887] | 5928 | 102 |
| MMGT1 | membrane magnesium transporter 1 [Source:HGNC Symbol;Acc:HGNC:28100] | 93380 | 103 |
| RPS6KA1 | ribosomal protein S6 kinase Al [Source:HGNC Symbol;Acc:HGNC:10430] | 6195 | 104 |
| RNF6 | ring finger protein 6 [Source:HGNC Symbol;Acc:HGNC:10069] | 6049 | 105 |
| CXCL8 | C-X-C motif chemokine ligand 8 [Source:HGNC Symbol;Acc:HGNC:6025] | 3576 | 106 |
| CAPN7 | calpain 7 [Source:HGNC Symbol;Acc:HGNC:1484] | 23473 | 107 |
| SOD2 | superoxide dismutase 2 [Source:HGNC Symbol;Acc:HGNC:11180] | 6648 | 108 |
| STX12 | syntaxin 12 [Source:HGNC Symbol;Acc:]HGNC:11430] | 23673 | 109 |
| NCSTN | nicastrin [Source:HGNC Symbol;Acc:HGNC:17091] | 23385 | 110 |
| GTF2B | general transcription factor IIB [Source:HGNC Symbol;Acc:HGNC:4648] | 2959 | 111 |
| PIM1 | Pim-1 proto-oncogene, serine/threonine kinase [Source:HGNC Symbol;Acc:HGNe:8986] | 5292 | 112 |

(continued)

| TABLE 1: List of 160 genes and reference numbers for each gene in this specification | | | |
|---|---|---|---|
| genes | Gene description | NCBI gene ID | Ref. No. |
| FAM91A1 | family with sequence similarity 91 member A1 [Source:HGNC Symbol;Acc:HGNC:263061 | 157769 | 113 |
| TNFAIP3 | TNF alpha induced protein 3 [Source:HGNC Symbol;Acc:HGNC:11896] | 7128 | 114 |
| ANKRD36B | ankyrin repeat domain 36B [Source:HGNC Symbol;Acc:IIGNC:293331 | 57730 | 115 |
| PMAIP1 | phorbol-12-myristate-13-acetate-induced protein 1 [Source:HGNC Symbol;Acc:HGNC:9108] | 5366 | 116 |
| UBA3 | ubiquitin like modifier activating enzyme 3 [Source:HGNC Symbol;Acc:HGNC:12470] | 9039 | 117 |
| SNX17 | sorting nexin 17 [Source:HGNC Symbol;Acc:HGNC:14979] | 9784 | 118 |
| TRAPPC3 | trafficking protein particle complex 3 [Source:HGNC Symbol ;Acc:HGNC:19942] | 27095 | 119 |
| CXCL3 | C-X-C motif chemokine ligand 3 [Source:HGNC Symbol;Acc:HGNC:4604] | 2921 | 120 |
| ARHGAP12 | Rho GTPase activating protein 12 [Source:HGNC Symbol;Acc:HGNC:16348] | 94134 | 121 |
| PLD4 | phospholipase D family member 4 [Source:HGNC Symbol;Acc:HGNC:23792] | 122618 | 122 |
| CD69 | CD69 molecule [Source:HGNC Symbol;Acc:HGNC:1694] | 969 | 123 |
| NAA25 | N(alpha)-acetyltransferase 25, NatB auxiliary subunit [Source:HGNC Symbol;Acc:HGNC:25783] | 80018 | 124 |
| CAPZAI | capping actin protein of muscle Z-line subunit alpha 1 [Source:HGNC Symbol;Acc:HGNC:1488] | 829 | 125 |
| HERC3 | HECT and RLD domain containing E3 ubiquitin protein ligase 3 [Source:HGNC Symbol;Acc:HGNe:4876] | 8916 | 126 |
| ARHGAP27 | Rho GTPase activating protein 27 [Source:HGNC Symbol;Acc:HGNC:31813] | 201176 | 127 |
| CLTC | clathrin heavy chain [Source:HGNC Symbol;Acc:HGNC:2092] | 1213 | 128 |
| DDB1 | damage specific DNA binding protein 1 [Source:HGNC Symbol;Acc:HGNC:2717] | 1642 | 129 |
| DUSP6 | dual specificity phosphatase 6 [Source:HGNC Symbol ;Acc:HGNC:3072] | 1848 | 130 |
| CXCL2 | C-X-C motif chemokine ligand 2 [Source:HGNC Symbol;Acc:HGNC:4603] | 2920 | 131 |
| TAGAP | T cell activation RhoGTPase activating protein [Source:HGNC Symbol;Acc:HGNC:15669] | 117289 | 132 |
| ARHGEF3 | Rho guanine nucleotide exchange factor 3 [Source:HGNC Symbol;Acc:HGNC:683] | 50650 | 133 |
| VIM | vimentin [Source:HGNC Symbol;Acc:HGNC:12692] | 7431 | 134 |
| RIPK2 | receptor interacting serine/threonine kinase 2 [Source:HGNC Symbol;Acc:HGNC:10020] | 8767 | 135 |
| ANXA1 | annexin A1 [Source:HGNC Symbol;Acc:HGNC:533] | 301 | 136 |

(continued)

| TABLE 1: List of 160 genes and reference numbers for each gene in this specification | | | |
|---|---|---|---|
| genes | Gene description | NCBI gene ID | Ref. No. |
| HEATR5B | HEAT repeat containing 5B [Source:HGNC Symbol;Acc:HGNC:29273] | 54497 | 137 |
| Clorf131 | chromosome 1 open reading frame 131 [Source:HGNC Symbol;Acc:HGNC:25332] | 128061 | 138 |
| MGAT1 | alpha-1,3-mannosyl-glycoprotein 2-beta-N-acetylglucosaminyltransferase [Source:HGNC Symbol;Acc:HGNC:7044] | 4245 | 139 |
| CELF1 | CUGBP Elav-like family member 1 [Source:HGNC Symbol;Acc:HGNC:2549] | 10658 | 140 |
| SFMBT2 | Scm like with four mbt domains 2 [Source:HGNC Symbol;Acc:HGNC:20256] | 57713 | 141 |
| EFR3A | EFR3 homolog A [Source:HGNC Symbol;Acc:HGNC:28970] | 23167 | 142 |
| GCH1 | GTP cyclohydrolase 1 [Source:HGNC Symbol;Acc:HGNC:4193] | 2643 | 143 |
| RSRP1 | arginine and serine rich protein 1 [Source:HGNC Symbol;Acc:HGNC:25234] | 57035 | 144 |
| RASGEF1B | RasGEF domain family member 1B [Source:HGNC Symbol;Acc:HGNC:24881] | 153020 | 145 |
| HSBP1 | heat shock factor binding protein 1 [Source:HGNC Symbol;Acc:HGNC:5203] | 3281 | 146 |
| STX11 | syntaxin 11 [Source:HGNC Symbol; Acc: HGNC: 11429] | 8676 | 147 |
| PANK3 | pantothenate kinase 3 [Source:HGNC Symbol;Acc:HGNC:19365] | 79646 | 148 |
| FAM13B | family with sequence similarity 13 member B [Source:HGNC Symbol;Acc:HGNC:1335] | 51306 | 149 |
| SPATA13 | spermatogenesis associated 13 [Source:HGNC Symbol;Acc:HGNC:23222] | 221178 | 150 |
| KLF7 | Kruppel like factor 7 [Source:HGNC Symbol;Acc:HGNC:6350] | 8609 | 151 |
| SLC35A2 | solute carrier family 35 member A2 [Source:HGNC Symbol;Acc:HGNC:11022] | 7355 | 152 |
| STK26 | serine/threonine kinase 26 [Source:HGNC Symbol ; Acc:HGNC: 18174] | 51765 | 153 |
| AZIN1 | antizyme inhibitor 1 [Source:HGNC Symbol; Acc: HGNC: 16432] | 51582 | 154 |
| C5AR1 | complement C5a receptor 1 [Source:HGNC Symbol;Acc:HGNC:1338] | 728 | 155 |
| CCAR1 | cell division cycle and apoptosis regulator 1 [Source:HGNC Symbol;Acc:HGNC:24236] | 55749 | 156 |
| DDX3X | DEAD-box helicase 3 X-linked [Source:HGNC Symbol;Acc:HGNC:2745] | 1654 | 157 |
| MORF4L2 | mortality factor 4 like 2 [Source:HGNC Symbol;Acc:HGNC:16849] | 9643 | 158 |
| ADD3 | adducin 3 [Source:HGNC Symbol;Acc:HGNC:245] | 120 | 159 |
| SECISBP2L | SECIS binding protein 2 like [Source:HGNC Symbol;Acc:HGNC:28997] | 9728 | 160 |

TABLE 2

| TABLE 2: Data from all mononuclear cells | | | | | |
|---|---|---|---|---|---|
| Ref. No. | CR | background | log_fc | pval | padj |
| 1 | 5.11101 | 1.79148 | 1.048353 | 3.34E-11 | 2.10E-09 |
| 3 | 7.15973 | 3.78013 | 0.638712 | 1.17E-10 | 3.69E-09 |
| 4 | 1.00904 | 2.67297 | -0.97119 | 9.77E-05 | 0.000296 |
| 5 | 1.37736 | 2.49784 | -0.59526 | 1.87E-05 | 0.000107 |
| 7 | 1.67539 | 3.39351 | -0.70582 | 0.0009173 | 0.001993 |
| 8 | 1.22197 | 2.15903 | -0.5692 | 0.0024618 | 0.004847 |
| 9 | 6.36228 | 2.97523 | 0.760065 | 0.0002523 | 0.000611 |
| 10 | 9.64039 | 4.83283 | 0.69053 | 9.78E-06 | 6.84E-05 |
| 12 | 13.2192 | 7.23261 | 0.603072 | 5.30E-05 | 0.000231 |
| 13 | 2.08688 | 1.22852 | 0.529857 | 0.0065391 | 0.01177 |
| 15 | 2.72436 | 1.26564 | 0.766651 | 7.58E-07 | 9.55E-06 |
| 16 | 1.60327 | 3.08171 | -0.65344 | 0.0005138 | 0.001156 |
| 19 | 25.1341 | 14.6067 | 0.542742 | 0.0002043 | 0.000536 |
| 20 | 4.55132 | 2.53979 | 0.583335 | 6.20E-05 | 0.000234 |
| 21 | 1.13673 | 2.44811 | -0.53295 | 3.04E-06 | 2.73E-05 |
| 22 | 7.34048 | 3.97894 | 0.612389 | 5.19E-05 | 0.000231 |
| 23 | 1.94498 | 3.33555 | -0.53938 | 0.0151272 | 0.024436 |
| 25 | 3.44471 | 1.74491 | 0.680139 | 0.0254482 | 0.039103 |
| 26 | 2.60452 | 1.40653 | 0.616123 | 3.88E-06 | 3.06E-05 |
| 27 | 57.7704 | 32.2036 | 0.584397 | 9.85E-05 | 0.000296 |
| 28 | 1.83304 | 3.29966 | -0.58784 | 0.0002178 | 0.000549 |
| 30 | 5.37789 | 3.10634 | 0.548852 | 0.0002863 | 0.000668 |
| 32 | 4.92098 | 8.38231 | -0.53262 | 0.0058916 | 0.011248 |
| 33 | 29.0623 | 50.2806 | -0.54818 | 0.0063941 | 0.01177 |
| 34 | 2.67035 | 4.54831 | -0.53255 | 0.0418851 | 0.061367 |
| 35 | 6.38687 | 11.6063 | -0.5973 | 0.0138876 | 0.023024 |
| 37 | 4.65969 | 2.7152 | 0.540082 | 4.52E-05 | 0.000231 |
| 38 | 1.28713 | 3.28612 | -0.93729 | 1.87E-05 | 0.000107 |
| 40 | 1.99557 | 1.15483 | 0.546981 | 1.46E-06 | 1.53E-05 |
| 44 | 6.13027 | 10.6744 | -0.55461 | 0.0001633 | 0.000447 |
| 45 | 5.90939 | 3.15561 | 0.627359 | 1.69E-09 | 3.55E-08 |
| 46 | 6.19499 | 3.24875 | 0.64547 | 8.15E-05 | 0.00027 |
| 48 | 6.97285 | 4.19461 | 0.508223 | 0.0071783 | 0.012562 |
| 51 | 5.24595 | 3.11531 | 0.521129 | 6.44E-05 | 0.000234 |
| 55 | 15.9691 | 8.63285 | 0.615082 | 0.0018411 | 0.003742 |
| 64 | 1.09475 | 2.10537 | -0.65397 | 0.0001587 | 0.000447 |
| 72 | 1.9356 | 3.5517 | -0.60701 | 0.001815 | 0.003742 |
| 73 | 3.9826 | 6.59961 | -0.50508 | 5.50E-05 | 0.000231 |

(continued)

| TABLE 2: Data from all mononuclear cells | | | | | |
|---|---|---|---|---|---|
| Ref. No. | CR | background | log_fc | pval | padj |
| 84 | 1.37153 | 3.14066 | -0.82851 | 0.0417337 | 0.061367 |
| 101 | 1.00198 | 1.71039 | -0.53475 | 0.016756 | 0.026391 |
| 108 | 4.67957 | 2.81403 | 0.508589 | 6.67E-05 | 0.000234 |
| 138 | 1.33395 | 2.6634 | -0.69146 | 0.0124514 | 0.021201 |

TABLE 3

| TABLE 3: Data from non-classical mononuclear cells | | | | | |
|---|---|---|---|---|---|
| Ref. No. | CR | background | log_fc | pval | padj |
| 1 | 7.9741 | 3.206541132 | 0.9110066 | 6.65E-05 | 0.00101 |
| 2 | 1.02089 | 1.960327019 | -0.652436 | 3.01E-07 | 2.74E-05 |
| 15 | 5.83493 | 3.104241638 | 0.6310927 | 0.025762 | 0.04695 |
| 23 | 2.48241 | 5.673745442 | -0.82662 | 4.69E-05 | 0.00101 |
| 26 | 4.56666 | 2.399399373 | 0.6435645 | 0.0228228 | 0.04515 |
| 40 | 3.79647 | 1.928631246 | 0.6772605 | 0.0151912 | 0.03495 |
| 50 | 3.39853 | 2. 004223701 | 0.528087 | 0.0263129 | 0.04695 |
| 52 | 5.33177 | 3.033683268 | 0.563906 | 0.0437831 | 0.0699 |
| 58 | 4.44327 | 2.528941799 | 0.5635891 | 0.015821 | 0.03495 |
| 62 | 1.74071 | 3.593879044 | -0.724941 | 0.0471049 | 0.07144 |
| 63 | 1.24488 | 2.115434657 | -0.530217 | 0.0303088 | 0.05203 |
| 65 | 1.0009 | 1.721719871 | -0.542427 | 0.0046195 | 0.01911 |
| 75 | 3.74453 | 9.528128665 | -0.933954 | 2.11E-05 | 0.00067 |
| 78 | 2.76144 | 4.851519404 | -0.563538 | 0.0103967 | 0.02867 |
| 80 | 1.57761 | 2.644238269 | -0.516473 | 0.0113186 | 0.02957 |
| 87 | 1.63173 | 3.24893924 | -0.688688 | 0.0026622 | 0.01256 |
| 94 | 1.48451 | 2.933514716 | -0.681119 | 0.0165841 | 0.0351 |
| 98 | 2.70538 | 1.635565181 | 0.5032551 | 0.0308742 | 0.05203 |
| 100 | 1.31385 | 3.59279652 | -1.00597 | 8.92E-05 | 0.00116 |
| 104 | 3.45793 | 2.0402062 | 0.5276203 | 0.0259259 | 0. 04695 |
| 109 | 2.58425 | 1.515920734 | 0.5334138 | 0.0016333 | 0.00991 |
| 111 | 1.53162 | 2.880423039 | -0.631614 | 0.0259255 | 0.04695 |
| 112 | 1.05978 | 1.783732557 | -0.520648 | 0.0192472 | 0.03892 |
| 113 | 2.07362 | 1.02067501 | 0.7088314 | 0.0117816 | 0.02978 |
| 114 | 2.03593 | 4.075608878 | -0.694067 | 0.011372 | 0.02957 |
| 116 | 2.75254 | 5.224775577 | -0.640888 | 0.0015606 | 0.00991 |
| 117 | 2.57569 | 1.318701844 | 0.6694686 | 0.0083959 | 0.02547 |
| 118 | 3.29288 | 1.981418262 | 0.5079505 | 0.0070361 | 0.02287 |
| 119 | 2.06027 | 1.143950863 | 0.5883482 | 0.0027293 | 0.01256 |
| 121 | 1.20919 | 2.170934441 | -0.585204 | 0.0468919 | 0.07144 |

(continued)

| TABLE 3: Data from non-classical mononuclear cells | | | | | |
|---|---|---|---|---|---|
| Ref. No. | CR | background | log_fc | pval | padj |
| 122 | 1.13296 | 2.019308245 | -0.57792 | 6.08E-05 | 0.00101 |
| 123 | 1.43737 | 2.39519825 | -0.510648 | 0.0393409 | 0.06393 |
| 124 | 1.35537 | 2.290899774 | -0.524873 | 0.015761 | 0.03495 |
| 125 | 7.80031 | 4.408059722 | 0.5707283 | 0.0051514 | 0.01994 |
| 126 | 2.11258 | 3.750198596 | -0.573899 | 0.0057195 | 0.02002 |
| 127 | 1.97641 | 3.445405242 | -0.555758 | 0.0052596 | 0.01994 |
| 128 | 2.16169 | 1.237680948 | 0.5576521 | 0.0077395 | 0.02429 |
| 129 | 1.68745 | 1.003003156 | 0.5202185 | 0.0128651 | 0.03164 |
| 132 | 2.80363 | 5.054321693 | -0.589327 | 0.0064241 | 0.02165 |
| 133 | 1.7022 | 2.833450826 | -0.509572 | 0.0092772 | 0.02638 |
| 135 | 2.21933 | 5.415190701 | -0.892005 | 2.20E-05 | 0.00067 |
| 136 | 18.8154 | 40.38247398 | -0.76372 | 0.005561 | 0.02002 |
| 139 | 4.12538 | 7.327550311 | -0.574483 | 0.000213 | 0.00242 |
| 141 | 1.43783 | 2.39507384 | -0.510281 | 0.0089451 | 0.02626 |
| 143 | 1.42963 | 2.430180405 | -0.530552 | 0.0038689 | 0.01677 |
| 144 | 4.72279 | 2.729104278 | 0.5484266 | 0.034939 | 0.05781 |
| 145 | 5.86417 | 10.82447372 | -0.612949 | 0.0004166 | 0.00361 |
| 147 | 2.65033 | 4.738169704 | -0.580967 | 0.0007547 | 0.00572 |
| 148 | 1.10221 | 1.993939679 | -0.592796 | 0.018573 | 0.03841 |
| 149 | 2.11508 | 1.256523706 | 0.5207428 | 0.0161325 | 0.03495 |
| 151 | 1.6183 | 3.613204717 | -0.803221 | 0.0026299 | 0.01256 |
| 152 | 1.20672 | 2.09082434 | -0.549651 | 0.0003417 | 0.00346 |
| 153 | 1.83738 | 1.066540603 | 0.5439199 | 0.0492457 | 0.07346 |
| 154 | 1.99682 | 4.519479027 | -0.81684 | 0.0272667 | 0.04772 |
| 155 | 5.17128 | 8.854339552 | -0.537788 | 0.0013422 | 0.0094 |
| 156 | 1.92403 | 1.100228196 | 0.5589058 | 0.0257324 | 0.04695 |
| 157 | 6.24469 | 14.06512232 | -0.811966 | 0.0004366 | 0.00361 |
| 158 | 1.69855 | 3.502152478 | -0.723602 | 0.0024516 | 0.01256 |
| 159 | 2.84829 | 1.51972787 | 0.6281873 | 0.0027607 | 0.01256 |
| 160 | 1.87114 | 1.127713468 | 0.5063565 | 0.0454254 | 0.07127 |

TABLE 4

| TABLE 4: Data from pool of classical and non-classical monocytes | | | | | |
|---|---|---|---|---|---|
| Ref. No. | CR | background | log_fc | pval | padj |
| 1 | 7.5588 | 2.6626776 | 1.04338049 | 1.55E-13 | 3.50E-12 |
| 2 | 1.18518 | 2.8298142 | -0.8703189 | 2.03E-14 | 9.15E-13 |
| 3 | 8.60091 | 4.574068 | 0.63146473 | 1.50E-06 | 3.75E-06 |
| 6 | 2.24997 | 1.3150835 | 0.53701725 | 1.34E-06 | 3.56E-06 |

(continued)

| TABLE 4: Data from pool of classical and non-classical monocytes | | | | | |
|---|---|---|---|---|---|
| Ref. No. | CR | background | log_fc | pval | padj |
| 9 | 9.65415 | 5.3659486 | 0.58731484 | 0.003357 | 0.003873 |
| 14 | 1.21756 | 2.1118731 | -0.5507289 | 1.34E-08 | 8.59E-08 |
| 15 | 3.7259 | 1.5820464 | 0.85659023 | 0.000112 | 0.0001694 |
| 20 | 6.65562 | 3.7933246 | 0.56221891 | 6.20E-06 | 1.27E-05 |
| 23 | 2.5254 | 6.2110889 | -0.8999353 | 3.10E-10 | 4.66E-09 |
| 24 | 1.09689 | 2.0784111 | -0.6391265 | 1.81E-07 | 6.79E-07 |
| 26 | 3.1296 | 1.4914023 | 0.74118873 | 4.21E-07 | 1.46E-06 |
| 29 | 10.7754 | 20.459613 | -0.6411881 | 1.65E-056 | 2.97E-05 |
| 31 | 1.50841 | 2.9133394 | -0.6582461 | 1.74E-07 | 6.7911-07 |
| 36 | 1.32814 | 2.4600158 | -0.6163908 | 2.80E-05 | 4.84E-05 |
| 40 | 2.6069 | 1.3454511 | 0.66143343 | 0.000397 | 0.0005109 |
| 42 | 1.13786 | 1.923062 | -0.5247698 | 0.000389 | 0.0005109 |
| 47 | 1.55984 | 2.7234057 | -0.5573017 | 0.000141 | 0.0001923 |
| 19 | 1.45009 | 2.6653687 | -0.6087159 | 2.66E-09 | 2.39E-08 |
| 50 | 3.06648 | 1.7824028 | 0.54256827 | 4.06E-05 | 6.77E-05 |
| 55 | 20.8066 | 9.9470198 | 0.73799517 | 0.002673 | 0.0031655 |
| 56 | 2.07311 | 4.8372785 | -0.8472896 | 1.43E-07 | 6.44E-07 |
| 57 | 1.88419 | 4.0128252 | -0.7634435 | 9.08E-07 | 2.92E-06 |
| 59 | 1.5942 | 2.958947 | -0.618462 | 4.86E-06 | 1.04E-05 |
| 60 | 1.32376 | 3.0040998 | -0.819504 | 0.022768 | 0.0238266 |
| 62 | 1.95189 | 3.7626401 | -0.6563222 | 1.39E-05 | 2.61E-05 |
| 67 | 1.43865 | 2.6718405 | -0.6190596 | 1.17E-06 | 3.52E-06 |
| 69 | 1.85745 | 3.3545956 | -0.5911275 | 1.26E-06 | 3.54E-06 |
| 73 | 3.4336 | 8.2520934 | -0.8768581 | 7.22E-06 | 1.41E-05 |
| 74 | 1.48452 | 2.577321 | -0.5516576 | 2.27E-06 | 5.37E-06 |
| 75 | 5.82293 | 11.200414 | -0.6541475 | 6.13E-10 | 6.89E-09 |
| 76 | 4.74138 | 2.7433786 | 0.54713888 | 0.000117 | 0.0001694 |
| 88 | 1.29896 | 2.2100276 | -0.5311393 | 8.24E-05 | 0.0001325 |
| 89 | 4.10354 | 8.0935891 | -0.6792213 | 1.26E-08 | 8.59E-08 |
| 92 | 1.08625 | 1.933769 | -0.5767369 | 0.002416 | 0.0029389 |
| 95 | 2.155 | 5.471957 | -0.801509 | 0.000115 | 0.0001694 |
| 96 | 3.5063 | 2.1168822 | 0.50461678 | 0.005343 | 0.005864 |
| 97 | 5.91056 | 10.787257 | -0.6016297 | 0.000123 | 0.0001723 |
| 99 | 1.45969 | 2.4506215 | -0.5181183 | 7.49E-08 | 3.74E-07 |
| 102 | 2.95583 | 1.7693565 | 0.51316431 | 0.002235 | 0.0027934 |
| 106 | 17.5891 | 35.159565 | -0.6926167 | 0.004296 | 0.0048334 |
| 120 | 1.41407 | 2.7228653 | -0.6552113 | 0.020597 | 0.0220687 |
| 131 | 2.01775 | 3.7757837 | -0.6266251 | 0.035744 | 0.0365569 |

TABLE 5

| TABLE 5: Data from intermediate monocytes | | | | | |
|---|---|---|---|---|---|
| Ref. No. | CR | background | log_fc | pval | padj |
| 4 | 1.00232 | 4.317288367 | -1.1603113 | 1.68E-06 | 5.08E-05 |
| 5 | 1.99267 | 3.925364993 | -0.6779833 | 8.75E-05 | 0.000596 |
| 7 | 2.35905 | 5.386910518 | -0.8257129 | 0.000118 | 0.0006698 |
| 8 | 1.23369 | 3.151268142 | -0.937797 | 3.26E-06 | 5.91E-05 |
| 11 | 2.47791 | 1.174579898 | 0.74650626 | 0.02119 | 0.0419193 |
| 13 | 3.85738 | 1.430290571 | 0.99211183 | 1.31E-08 | 1.20E-06 |
| 16 | 2.03718 | 5.025193777 | -0.9028972 | 1.41E-07 | 6.42E-06 |
| 17 | 1.82702 | 1.097318833 | 0.5098167 | 5.68E-06 | 8.61E-05 |
| 18 | 1.24499 | 2.057705656 | -0.502467 | 0.000589 | 0.0025515 |
| 21 | 1.8069 | 3.03898039 | -0.5199079 | 0.009265 | 0.0227869 |
| 25 | 3.21502 | 1.190299422 | 0.99362781 | 0.023735 | 0.0441348 |
| 28 | 3.18961 | 5.562590129 | -0.5561666 | 0.00274 | 0.0089057 |
| 34 | 4.74275 | 7.961297348 | -0.5179745 | 0.000904 | 0.0034294 |
| 38 | 1.75503 | 3.598492938 | -0.7180283 | 0.00056 | 0.002546 |
| 39 | 3.16232 | 1.602911347 | 0.67948439 | 7.77E-05 | 0.000596 |
| 41 | 10.4511 | 18.05452746 | -0.546694 | 0.016108 | 0.0333141 |
| 43 | 3.91576 | 2.34812787 | 0.51139036 | 0.012561 | 0.0278801 |
| 45 | 4.55003 | 2.311740794 | 0.67713349 | 0.011915 | 0.0271074 |
| 53 | 3.34487 | 5.518928137 | -0.5007564 | 0.000386 | 0.0019497 |
| 54 | 1.83135 | 1.014454369 | 0.59070133 | 0.002374 | 0.0080005 |
| 61 | 5.43967 | 2.490819388 | 0.78110746 | 9.26E-05 | 0.000596 |
| 64 | 1.24649 | 3.194566228 | -0.9411199 | 0.000697 | 0.0027576 |
| 66 | 1.34253 | 2.676832879 | -0.6900798 | 0.017261 | 0.034905 |
| 68 | 2.6901 | 1.373029671 | 0.67255749 | 0.000158 | 0.0008472 |
| 70 | 3.93951 | 2.301517522 | 0.53748662 | 7.34E-05 | 0.000596 |
| 71 | 2.02346 | 3.122981409 | -0.5257042 | 0.014947 | 0.0316317 |
| 72 | 3.42788 | 6.07197252 | -0.5717414 | 1.28E-05 | 0.0001658 |
| 77 | 1.58864 | 2.801468586 | -0.5672634 | 0.023765 | 0.0441348 |
| 79 | 3.10461 | 1.424527738 | 0.77904734 | 9.82E-05 | 0.000596 |
| 81 | 1.12671 | 2.066795425 | -0.6066943 | 0.010508 | 0.0245195 |
| 82 | 2.90372 | 1.747560369 | 0.50777317 | 0.003604 | 0.0113078 |
| 83 | 1.95926 | 1.143846236 | 0.53817019 | 0.042306 | 0.0726379 |
| 85 | 5.77864 | 3.47369625 | 0.50894951 | 0.004803 | 0.0136592 |
| 86 | 2.93286 | 4.903335295 | -0.5139378 | 0.000641 | 0.0026498 |
| 90 | 4.57475 | 2.745880276 | 0.51045072 | 0.03377 | 0.0602563 |
| 91 | 2.12833 | 1.130364156 | 0.63279595 | 0.000475 | 0.0022729 |
| 93 | 2.74382 | 1.648450614 | 0.50951562 | 0.004747 | 0.0136592 |
| 103 | 1.79946 | 1.065204256 | 0.52431869 | 0.001361 | 0.0049555 |

(continued)

| TABLE 5: Data from intermediate monocytes | | | | | |
|---|---|---|---|---|---|
| Ref. No. | CR | background | log_fc | pval | padj |
| 105 | 3.87093 | 2.109680187 | 0.60695779 | 0.004957 | 0.0136703 |
| 107 | 2.22537 | 1.226486639 | 0.59576731 | 0.009235 | 0.0227869 |
| 110 | 1.44085 | 2.723895911 | -0.6368308 | 5.14E-05 | 0.0005844 |
| 115 | 2.65202 | 1.580794325 | 0.51739495 | 0.004322 | 0.0131091 |
| 134 | 2.5676 | 1.540640577 | 0.51077295 | 0.001618 | 0.0056643 |
| 137 | 2.14202 | 1.298121271 | 0.50082924 | 0.029599 | 0.0538696 |
| 138 | 1.55736 | 4.223607749 | -0.9976996 | 0.006309 | 0.016885 |
| 140 | 2.478 | 1.495020532 | 0.50531058 | 8.51E-05 | 0.000596 |
| 142 | 2.44716 | 1.422327255 | 0.54263259 | 0.037474 | 0.0655801 |
| 146 | 1.28347 | 2.131752752 | -0.5073766 | 0.023642 | 0.0441348 |
| 150 | 3.30613 | 1.893465927 | 0.55737061 | 0.013768 | 0.0298317 |

TABLE 6

| TABLE 6: Data from classical monocytes | | | | | |
|---|---|---|---|---|---|
| Ref. No. | CR | background | log_fc | pval | padj |
| 106 | 46.0357 | 25.88433219 | 0.57577825 | 0.022382 | 0.0223815 |
| 130 | 7.39273 | 4.361418589 | 0.5276993 | 0.00087 | 0.0017403 |

TABLE 7

| TABLE 7: genes other than the above | | | | | | |
|---|---|---|---|---|---|---|
| Gene name | Type of monocyte | PD | background | log_fc | pval | padj |
| ANKRD20A11P | Intermediate | 4.02 | 1.60 | 0.92 | 2. 25. E-06 | 5. 12. E-05 |
| ATP2B1-AS1 | CD14+CD16 | 4.29 | 7.46 | -0.55 | 6. 87.E-08 | 3. 74.E-07 |
| CYP4F29P | Intermediate | 2.76 | 1.12 | 0.90 | 8. 67.E-05 | 5. 96. E-04 |
| GPX1 | CD14+CD16 | 5.54 | 9.69 | -0.56 | 3. 97. E-06 | 8. 93. E-06 |
| LINCO1578 | Intermediate | 3.41 | 1.78 | 0.65 | 6. 79. E-03 | 1. 76. E-02 |
| NAPSB | CD16 | 3.27 | 5.91 | -0.59 | 1. 33. E-02 | 3. 18. E-02 |
| SMIM25 | Total monocytes | 3.68 | 2.01 | 0.61 | 3. 16. E-07 | 4. 97. E-06 |
| TTTY 15 | Intermediate | 1.39 | 2.19 | -0.58 | 1. 02. E-02 | 2. 45. E-02 |

We further attempted to narrow down genes considered useful for predicting treatment efficacy from the 160 genes. Specifically, genes were narrowed down by three different methods. Based on the narrowed-down gene groups, a predictive model was constructed by logistic regression analysis. The logistic regression analysis was performed using JMP pro 16.

First, genes related to treatment effectiveness were narrowed down by a stepwise method from the relationship between the expression level of the 160 genes in each monocyte and treatment efficacy. As a result, as shown in FIG. 19, 10 genes were selected. When predicting whether a case from which each monocyte was derived was PD or non-PD based on the expression of the 10 genes, the prediction shown by the ROC curve and AUC in FIG. 19 was possible.

Similarly, genes related to treatment effectiveness were narrowed down by LASSO regression from the relationship between the expression level of each of the 160 genes and treatment outcome. As a result, as shown in FIG. 20, 47 genes were selected. When predicting whether a case from which each monocyte was derived was PD or non-PD based on the expression of the 47 genes, the prediction shown by the ROC curve and AUC in FIG. 20 was possible.

Furthermore, the 27 genes showing p < 0.0001, and the prediction by the expression of the 27 genes were as shown in FIG. 21.

Similarly, genes related to treatment effectiveness were narrowed down by elastic net regression from the relationship between the expression level of each of the 160 genes and treatment outcome. As a result, as shown in FIG. 22, 45 genes were selected. When predicting whether a case from which each monocyte was derived was PD or non-PD based on the expression of the 45 genes, the prediction shown by the ROC curve and AUC in FIG. 22 was possible. Furthermore, the 17 genes showing p < 0.0001, and the prediction by the expression of the 17 genes were as shown in FIG. 23.

In the above example, the treatment outcome (CR or PD) was classified by the length of PFS, but in the following examples and Tables 8 to 10 and FIGS. 19 to 23, the treatment efficacy was classified by the following criteria.
CR (complete response): A state where the tumor has completely disappeared.
PR (partial response): A state where the sum of the tumor sizes has decreased by 30% or more.
SD (stable disease): A state where the size of the tumor has not changed.
PD (progressive disease): A state where the sum of the tumor sizes has increased by 20% or more and also by 5 mm or more in absolute value, or a state where new lesions have appeared. Hereinafter, non-PD indicates something other than PD, that is, CR, PR, or SD.

TABLE 8

| TABLE 8: 8 lung cancer cases | | | | | | | | |
|---|---|---|---|---|---|---|---|---|
| sample | Treatment start age | Sex | Clinical stage | Treatment | Best effect | Survival | PFS | os |
| 1 | 53 | 男 | pT1cN2M0 Stage IIIA | Pembrolizumab | non-PD | 生存 | 334 | 2000 |
| 2 | 79 | 男 | pT4N3M1c Stage IVB | Pembrolizumab | PD | 死亡 | 77 | 226 |
| 3 | 81 | 男 | pT2bN1MO Stage IIB | Pembrolizumab | non-PD | 死亡 | 525 | 850 |
| 4 | 80 | 男 | pT2aN0M0 Stage IB | Pembrolizumab | PD | 死亡 | 29 | 29 |
| 5 | 73 | 男 | pT3N0M0 Stage IIB | Nivolumab | PD | 生存 | 66 | 1079 |
| 6 | 72 | 男 | pT1bN2M0 Stage IIIA | Pembrolizumab | PD | 死亡 | 168 | 196 |
| 7 | 51 | 女 | cT3N2M0 Stage IIIB | CDDP+MTA+Pembrolizumab | non-PD | 生存 | 1372 | 1372 |
| 8 | 68 | 女 | cT4N0M0 Stage IIIA | CDDP+MTA+Pembrolizumab | non-PD | 死亡 | 154 | 607 |

A new batch of pre-treatment PBMCs was obtained from 8 lung cancer patients at Fukushima Medical University (see TABLE 8). As shown in TABLE 8, the best treatment effect for cases 1, 3, 7, and 8 was non-PD (i.e., CR, PR, or SD), while the best treatment effect for cases 2 and 4-6 was PD. In the example above, gene expression analysis was conducted on monocytes, but in this study, the PBMCs themselves were used for the analysis. Specifically, PBMCs were obtained from blood samples from each case and used as the subject of analysis. RNA was extracted from each obtained PBMC, and RNA-Seq analysis was performed according to a conventional method to obtain gene expression data for each gene.

In the example above, a prediction model was constructed based on the gene expression of the 10 genes narrowed down by the stepwise method as shown in FIG. 19. When the therapeutic effectiveness of the patients in TABLE 8 was predicted using this prediction model based on the mRNA expression in each patient's pre-treatment PBMCs, cases 1, 5, 7, and 8 were predicted to be non-PD, as shown in TABLE 9, and the others were predicted to be PD. In other words, it was possible to correctly predict whether 6 out of 8 cases were non-PD or PD.

TABLE 9

| TABLE 9: prediction result from 10 genes | | | |
|---|---|---|---|
| Patient | Provability[PD]_1_2 | Provability[non-PD]_1_2 | Prediction result |
| 1 | 0.07 | 0.93 | non-PD |
| 2 | 0.48 | 0.52 | PD |
| 3 | 0.74 | 0.26 | PD |
| 4 | 0.79 | 0.21 | PD |
| 5 | 0.26 | 0.74 | non-PD |
| 6 | 0.35 | 0. 65 | PD |
| 7 | 0.22 | 0.78 | non-PD |
| 8 | 0.23 | 0.77 | non-PD |

In addition to the 10 genes mentioned above, a prediction model was constructed by logistic regression with 12 genes, adding RBBP4 and HSBP1, and re-evaluated. As a result, cases 1, 7, and 8 were predicted to be non-PD, and the others were predicted to be PD. In other words, it was possible to correctly predict whether 7 out of 8 cases were non-PD or PD. In this way, it was possible to construct a prediction model for treatment efficacy using patient's PBMCs by appropriately utilizing the narrowed-down gene groups.

TABLE 10

| TABLE 10: prediction result from 12 genes | | | |
|---|---|---|---|
| Patient | Provability[PD]_1_2 | Provability[non-PD]_1_2 | Prediction result |
| 1 | 0.93 | 0.07 | non-PD |
| 2 | 0.48 | 0. 52 | PD |
| 3 | 0.30 | 0.70 | PD |
| 4 | 0.21 | 0.79 | PD |
| 5 | 0.70 | 0. 30 | PD |
| 6 | 0.63 | 0. 37 | PD |
| 7 | 0.77 | 0.23 | non-PD |
| 8 | 0.72 | 0.28 | non-PD |

As described above, when using lung cancer patients from Fukushima Medical University as a validation dataset, it was also possible to predict the responsiveness of each patient to PD-1-related immune checkpoint inhibitors. Furthermore, it was possible to predict responsiveness not only using isolated monocytes but also using a sample containing monocytes, such as PBMCs. In addition, the prediction could be made based on a pre-treatment sample. As described above, it was understood that it is possible to predict the effectiveness of PD-1-related immune checkpoint inhibitors from the RNA expression in the PBMCs of pre-treatment cancer patients. When the therapeutic effect is increased or decreased in a patient who has been co-administered an immune checkpoint inhibitor and another anti-cancer drug, it may be possible to predict the effect that the other anti-cancer drug has on the patient's therapeutic responsiveness to immune checkpoint inhibitor treatment by checking the mRNA in the patient's sample.

Immune checkpoint inhibitors are agents that release the immune suppression switch of immune cells. The present disclosure shows that the therapeutic effectiveness of an ICI can be predicted by the gene expression on the immune cell side, not the cancer cell side. That is, the present disclosure shows a method of predicting immune cells whose immune checkpoint switch is likely to be inhibited by an ICI by using their intracellular gene expression as an indicator before treatment. Since the present disclosure provides a method of specifying the ease of immune checkpoint release on the immune cell side by gene expression on the immune cell side before treatment, it is considered that this therapeutic effectiveness prediction method is useful for all cancer types for which ICIs are considered effective, regardless of the cancer type.

## Claims

1. A method of predicting the effect of tumor treatment with an immune checkpoint inhibitor (ICI) in a subject, comprising:

    determining the expression level of a gene including one or more genes selected from the gene group described in any of Tables 1 to 7 in a sample obtained from the subject; and
    using a prediction model constructed to be able to predict the therapeutic effect, predicting the effect of treatment with an ICI from the determined expression level,
    wherein the prediction model is constructed based on the expression level of the gene including the one or more genes in a sample obtained from each of a patient group for whom ICI treatment is effective and a patient group for whom ICI treatment is not effective, and information regarding the effectiveness of ICI treatment.

2. The method according to CLAIM 1, wherein the one or more genes selected from the gene group described in Table 1 include any one or more genes from the gene group described in FIGS. 4A to 4NN.

3. The method according to CLAIM 1 or 2, wherein the one or more genes selected from the gene group described in Table 1 include one or more or all genes selected from the group consisting of CLEC12A, TMEM176A, and TMEM176B.

4. The method according to any one of CLAIMS 1 to 3, wherein the one or more genes selected from the gene group described in Table 1 include five or more genes.

5. The method according to any one of CLAIMS 1 to 4, wherein the one or more genes selected from the gene group described in Table 1 include 10 or more genes.

6. The method according to any one of CLAIMS 1 to 5, wherein the one or more genes selected from the gene group described in Table 1 include 20 or more genes.

7. The method according to any one of CLAIMS 1 to 6, wherein the prediction model is constructed by machine learning.

8. The method according to any one of CLAIMS 1 to 6, wherein the prediction model is constructed by logistic regression.

9. A method of treating a tumor in a subject, comprising:
administering a therapeutically effective amount of an immune checkpoint inhibitor (ICI) to the subject, wherein the subject is a subject for whom a therapeutic effect of an immune checkpoint inhibitor (ICI) is predicted to be effective by the method according to any one of CLAIMS 1 to 8.

10. The method according to any one of CLAIMS 1 to 9, wherein the immune checkpoint inhibitor (ICI) is an antibody or a PD-1 binding fragment of an antibody that can bind to PD-1 and neutralize the binding between PD-1 and PD-L1.

11. The method according to any one of CLAIMS 1 to 9, wherein the immune checkpoint inhibitor (ICI) is an antibody or a PD-1 binding fragment of an antibody that can bind to PD-L1 and neutralize the binding between PD-1 and PD-L1.

12. An immune checkpoint inhibitor (ICI) or a pharmaceutical composition including an immune checkpoint inhibitor, for use in the method according to any one of CLAIMS 9 to 11.

13. A method for predicting the action of a candidate substance on a patient's therapeutic responsiveness to immune checkpoint inhibitor treatment (ICI treatment), comprising:

    determining the expression level of a gene including one or more genes selected from the gene groups described in any of Tables 1 to 7 in each of a first sample obtained from a patient before administration of the candidate substance and a second sample obtained from the patient after administration of the candidate substance; and
    predicting the effect of treatment with an ICI from the determined expression levels using a prediction model constructed to be able to predict the effect of immune checkpoint inhibitor treatment,
    wherein the prediction model is constructed based on the expression levels of the one or more genes in a sample obtained from a patient group in which ICI treatment is effective and a patient group in which ICI treatment is not effective, and information regarding the effectiveness of ICI treatment, and
    wherein an increase in the prediction result of the effectiveness of treatment with an ICI in the first sample and the second sample indicates a possibility that the candidate substance increases the effectiveness of ICI treatment, and a decrease in the prediction result of the effectiveness of treatment with an ICI in the first sample and the second sample indicates a possibility that the candidate substance decreases the effectiveness of ICI treatment.

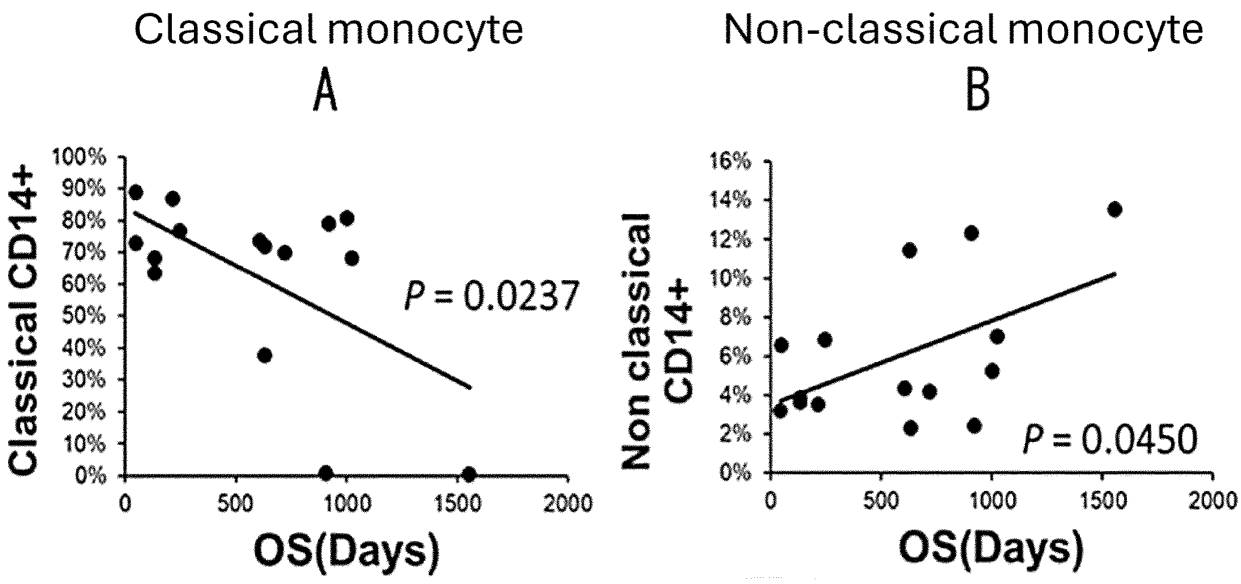
Classical monocyte
A
Classical CD14+
100%
90%
80%
70%
60%
50%
40%
30%
20%
10%
0%
P = 0.0237
0
500
1000
1500
2000
OS(Days)
Non-classical monocyte
B
Non classical CD14+
16%
14%
12%
10%
8%
6%
4%
2%
0%
P = 0.0450
0
500
1000
1500
2000
OS(Days)

FIG. 1

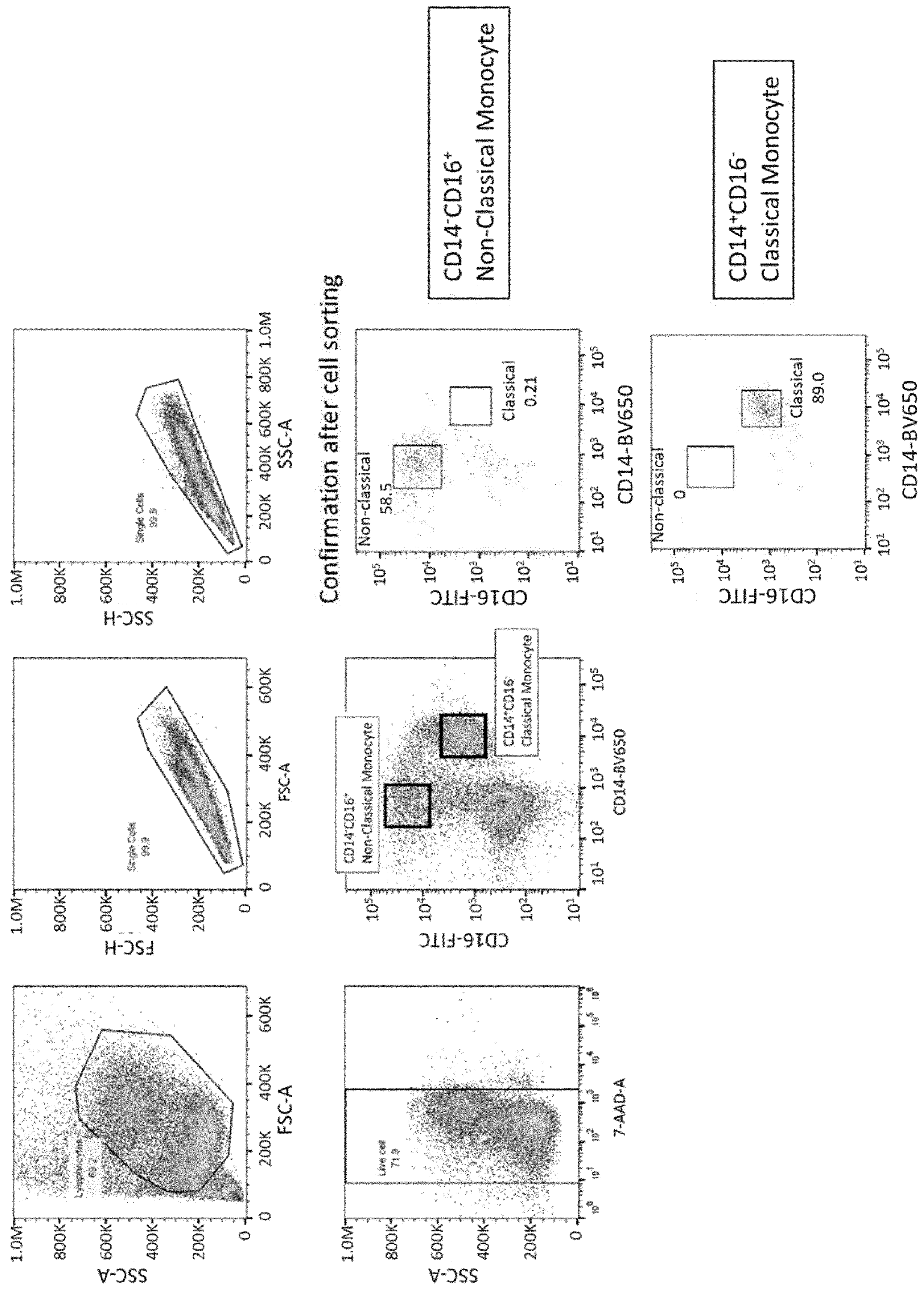
SSC-A
FSC-A
Lymphocytes 69.2
FSC-H
Single Cells 99.9
SSC-H
SSC-A
Single Cells 99.9
Live cell 71.9
7-AAD-A
CD16-FITC
CD14-BV650
CD14⁻CD16⁺ Non-Classical Monocyte
CD14⁺CD16⁻ Classical Monocyte
Confirmation after cell sorting
Non-classical 58.5
Classical 0.21
Non-classical 0
Classical 89.0
CD14⁻CD16⁺
Non-Classical Monocyte
CD14⁺CD16⁻
Classical Monocyte

FIG. 2

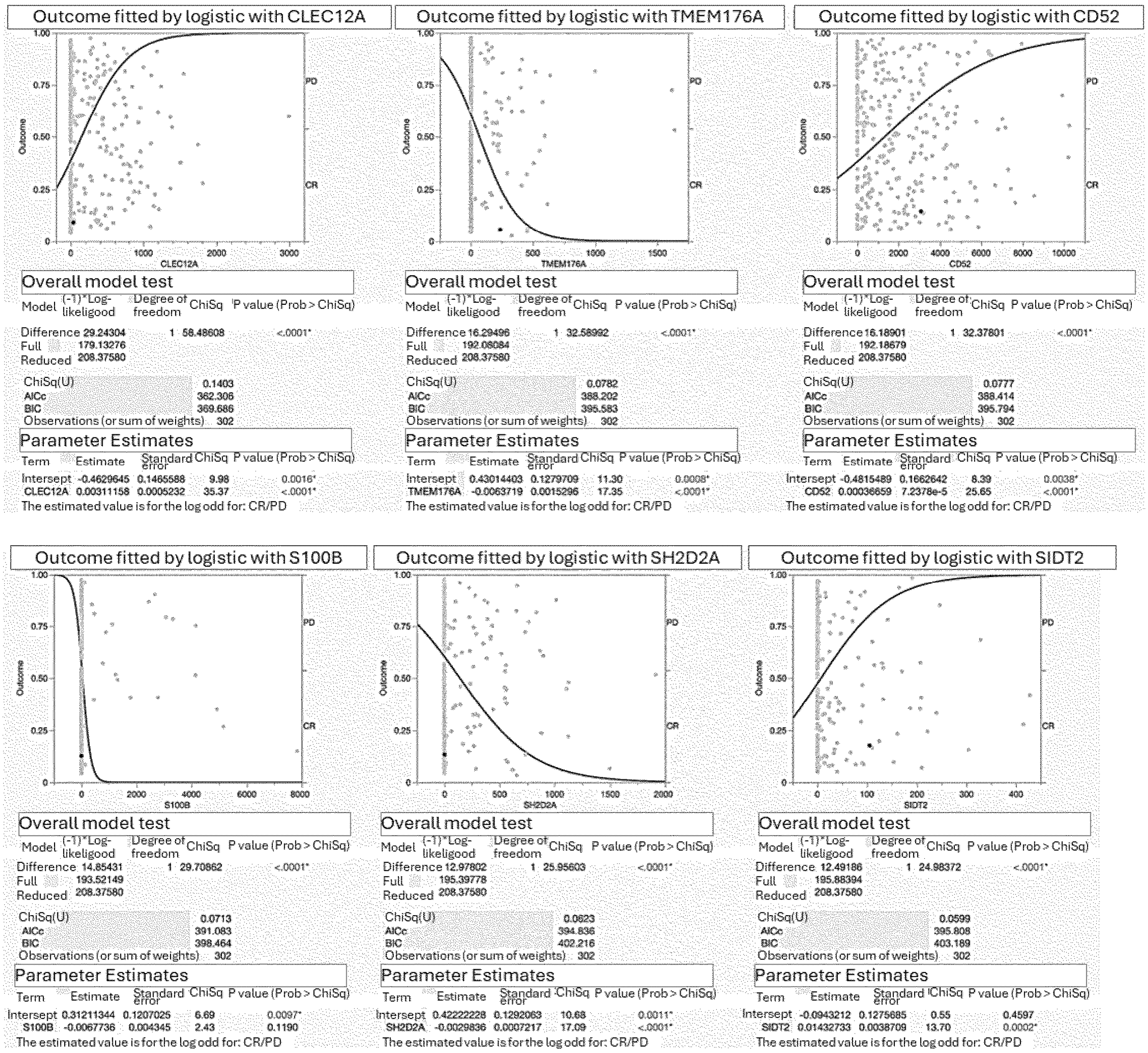

Outcome fitted by logistic with CLEC12A
Outcome
PD
CR
CLEC12A
Overall model test
Model (-1)*Log-likeligood Degree of freedom ChiSq P value (Prob > ChiSq)
Difference 29.24304 1 58.48608 <.0001*
Full 179.13276
Reduced 208.37580
ChiSq(U) 0.1403
AICc 362.306
BIC 369.686
Observations (or sum of weights) 302
Parameter Estimates
Term Estimate Standard error ChiSq P value (Prob > ChiSq)
Intersept -0.4629645 0.1465588 9.98 0.0016*
CLEC12A 0.00311158 0.0005232 35.37 <.0001*
The estimated value is for the log odd for: CR/PD
Outcome fitted by logistic with TMEM176A
TMEM176A
Overall model test
Model (-1)*Log-likeligood Degree of freedom ChiSq P value (Prob > ChiSq)
Difference 16.29496 1 32.58992 <.0001*
Full 192.08084
Reduced 208.37580
ChiSq(U) 0.0782
AICc 388.202
BIC 395.583
Observations (or sum of weights) 302
Parameter Estimates
Term Estimate Standard error ChiSq P value (Prob > ChiSq)
Intersept 0.43014403 0.1279709 11.30 0.0008*
TMEM176A -0.0063719 0.0015296 17.35 <.0001*
The estimated value is for the log odd for: CR/PD
Outcome fitted by logistic with CD52
CD52
Overall model test
Model (-1)*Log-likeligood Degree of freedom ChiSq P value (Prob > ChiSq)
Difference 16.18901 1 32.37801 <.0001*
Full 192.18679
Reduced 208.37580
ChiSq(U) 0.0777
AICc 388.414
BIC 395.794
Observations (or sum of weights) 302
Parameter Estimates
Term Estimate Standard error ChiSq P value (Prob > ChiSq)
Intersept -0.4815489 0.1662642 8.39 0.0038*
CD52 0.00036659 7.2378e-5 25.65 <.0001*
The estimated value is for the log odd for: CR/PD
Outcome fitted by logistic with S100B
S100B
Overall model test
Model (-1)*Log-likeligood Degree of freedom ChiSq P value (Prob > ChiSq)
Difference 14.85431 1 29.70862 <.0001*
Full 193.52149
Reduced 208.37580
ChiSq(U) 0.0713
AICc 391.083
BIC 398.464
Observations (or sum of weights) 302
Parameter Estimates
Term Estimate Standard error ChiSq P value (Prob > ChiSq)
Intersept 0.31211344 0.1207025 6.69 0.0097*
S100B -0.0067736 0.004345 2.43 0.1190
The estimated value is for the log odd for: CR/PD
Outcome fitted by logistic with SH2D2A
SH2D2A
Overall model test
Model (-1)*Log-likeligood Degree of freedom ChiSq P value (Prob > ChiSq)
Difference 12.97802 1 25.95603 <.0001*
Full 195.39778
Reduced 208.37580
ChiSq(U) 0.0623
AICc 394.836
BIC 402.216
Observations (or sum of weights) 302
Parameter Estimates
Term Estimate Standard error ChiSq P value (Prob > ChiSq)
Intersept 0.42222228 0.1292063 10.68 0.0011*
SH2D2A -0.0029836 0.0007217 17.09 <.0001*
The estimated value is for the log odd for: CR/PD
Outcome fitted by logistic with SIDT2
SIDT2
Overall model test
Model (-1)*Log-likeligood Degree of freedom ChiSq P value (Prob > ChiSq)
Difference 12.49186 1 24.98372 <.0001*
Full 195.88394
Reduced 208.37580
ChiSq(U) 0.0599
AICc 395.808
BIC 403.189
Observations (or sum of weights) 302
Parameter Estimates
Term Estimate Standard error ChiSq P value (Prob > ChiSq)
Intersept -0.0943212 0.1275685 0.55 0.4597
SIDT2 0.01432733 0.0038709 13.70 0.0002*
The estimated value is for the log odd for: CR/PD


FIG. 3A

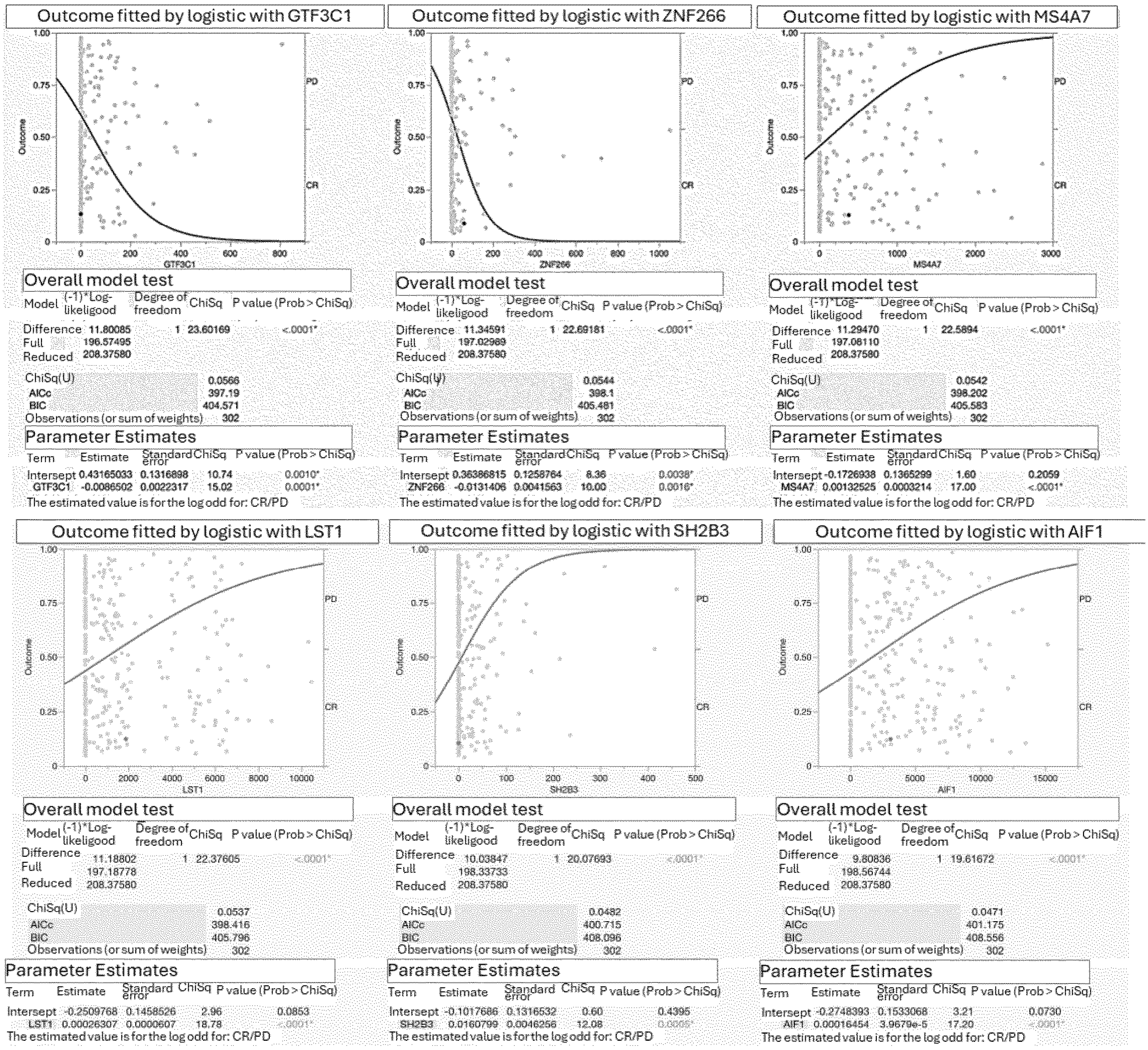
Outcome fitted by logistic with GTF3C1
Overall model test
Difference 11.80085 1 23.60169 <.0001*
Full 196.57495
Reduced 208.37580
ChiSq(U) 0.0566
AICc 397.19
BIC 404.571
Observations (or sum of weights) 302
Parameter Estimates
Intersept 0.43165033 0.1316898 10.74 0.0010*
GTF3C1 -0.0086502 0.0022317 15.02 0.0001*
The estimated value is for the log odd for: CR/PD
Outcome fitted by logistic with ZNF266
Overall model test
Difference 11.34591 1 22.69181 <.0001*
Full 197.02989
Reduced 208.37580
ChiSq(U) 0.0544
AICc 398.1
BIC 405.481
Observations (or sum of weights) 302
Parameter Estimates
Intersept 0.35386815 0.1258764 8.36 0.0038*
ZNF266 -0.0131406 0.0041563 10.00 0.0016*
The estimated value is for the log odd for: CR/PD
Outcome fitted by logistic with MS4A7
Overall model test
Difference 11.29470 1 22.5894 <.0001*
Full 197.08110
Reduced 208.37580
ChiSq(U) 0.0542
AICc 398.202
BIC 405.583
Observations (or sum of weights) 302
Parameter Estimates
Intersept -0.1726938 0.1365299 1.60 0.2059
MS4A7 0.00132525 0.0003214 17.00 <.0001*
The estimated value is for the log odd for: CR/PD
Outcome fitted by logistic with LST1
Overall model test
Difference 11.18802 1 22.37605 <.0001*
Full 197.18778
Reduced 208.37580
ChiSq(U) 0.0537
AICc 398.416
BIC 405.796
Observations (or sum of weights) 302
Parameter Estimates
Intersept -0.2509768 0.1458526 2.96 0.0853
LST1 0.00026307 0.0000607 18.78 <.0001*
The estimated value is for the log odd for: CR/PD
Outcome fitted by logistic with SH2B3
Overall model test
Difference 10.03847 1 20.07693 <.0001*
Full 198.33733
Reduced 208.37580
ChiSq(U) 0.0482
AICc 400.715
BIC 408.096
Observations (or sum of weights) 302
Parameter Estimates
Intersept -0.1017686 0.1316532 0.60 0.4395
SH2B3 0.0160799 0.0046256 12.08 0.0005*
The estimated value is for the log odd for: CR/PD
Outcome fitted by logistic with AIF1
Overall model test
Difference 9.80836 1 19.61672 <.0001*
Full 198.56744
Reduced 208.37580
ChiSq(U) 0.0471
AICc 401.175
BIC 408.556
Observations (or sum of weights) 302
Parameter Estimates
Intersept -0.2748393 0.1533068 3.21 0.0730
AIF1 0.00016454 3.9679e-5 17.20 <.0001*
The estimated value is for the log odd for: CR/PD

FIG. 3B

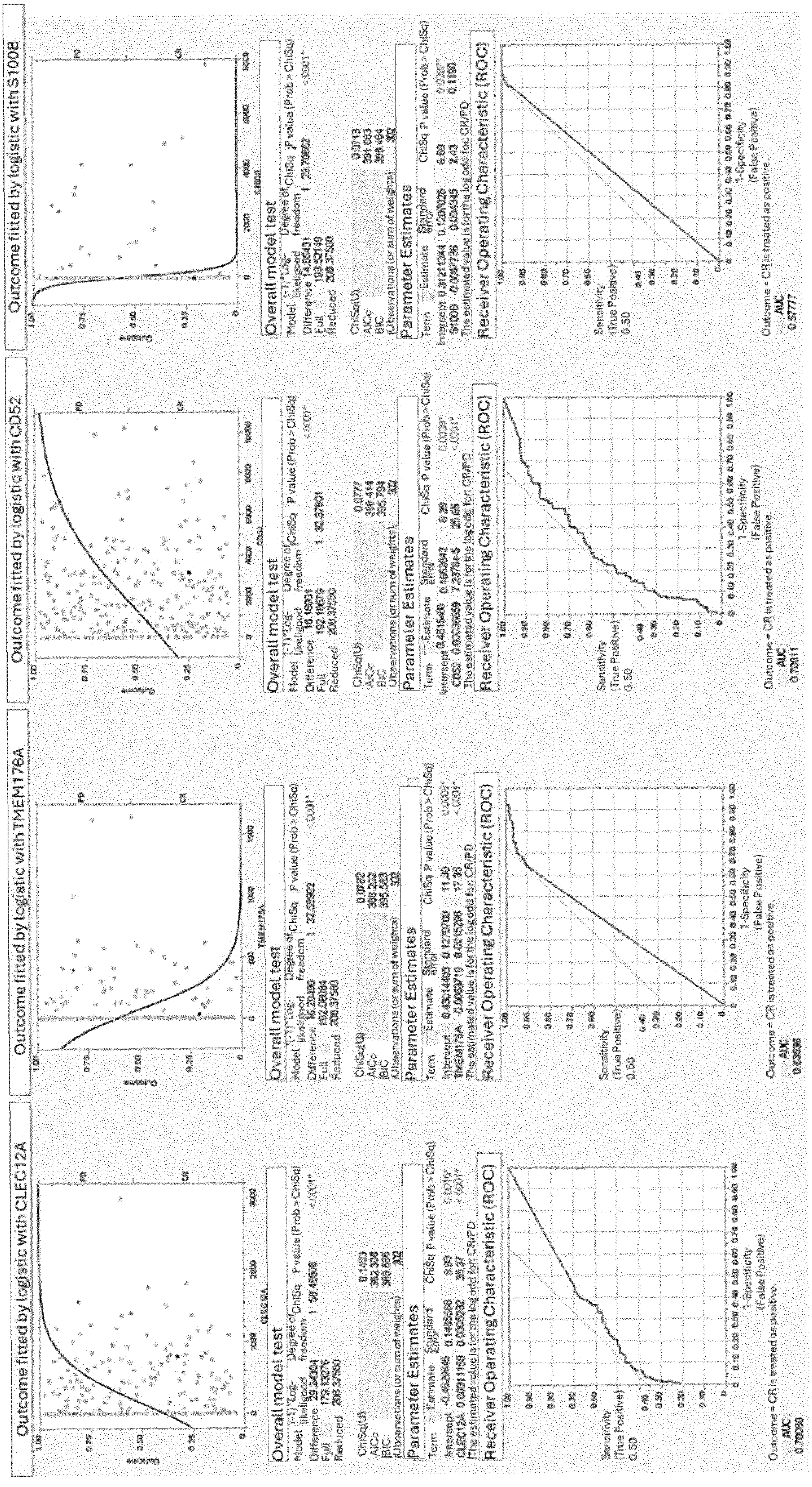
Outcome fitted by logistic with CLEC12A
Outcome fitted by logistic with TMEM176A
Outcome fitted by logistic with CD52
Outcome fitted by logistic with S100B
Overall model test
Parameter Estimates
Receiver Operating Characteristic (ROC)

FIG. 4A

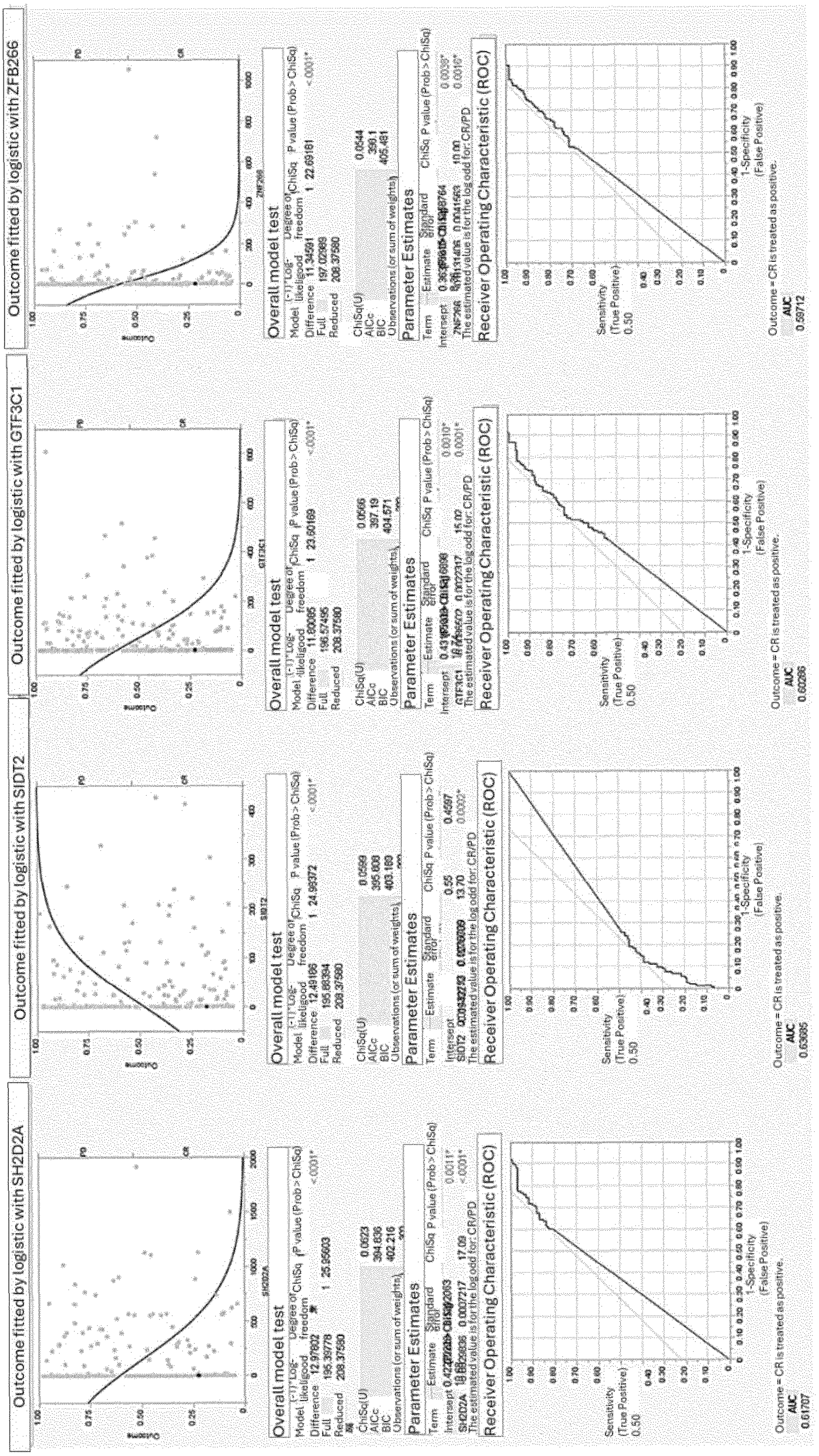
Outcome fitted by logistic with SH2D2A
Outcome fitted by logistic with SIDT2
Outcome fitted by logistic with GTF3C1
Outcome fitted by logistic with ZFB266
Overall model test
Parameter Estimates
Receiver Operating Characteristic (ROC)
Outcome = CR is treated as positive.
AUC
0.61707
0.63685
0.60286
0.59712

FIG. 4B

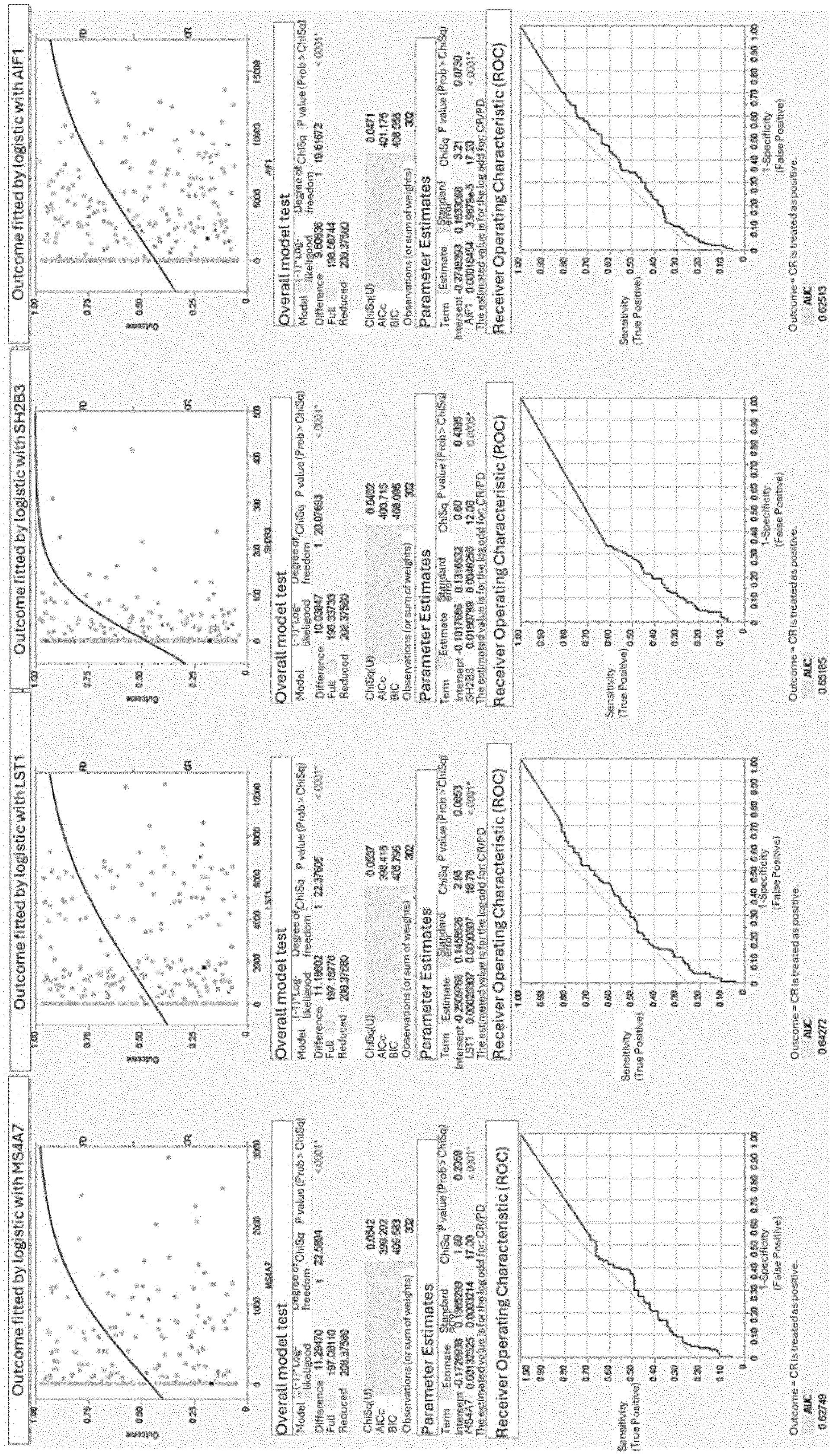
Outcome fitted by logistic with MS4A7
Outcome fitted by logistic with LST1
Outcome fitted by logistic with SH2B3
Outcome fitted by logistic with AIF1
Overall model test
Parameter Estimates
Receiver Operating Characteristic (ROC)

FIG. 4C

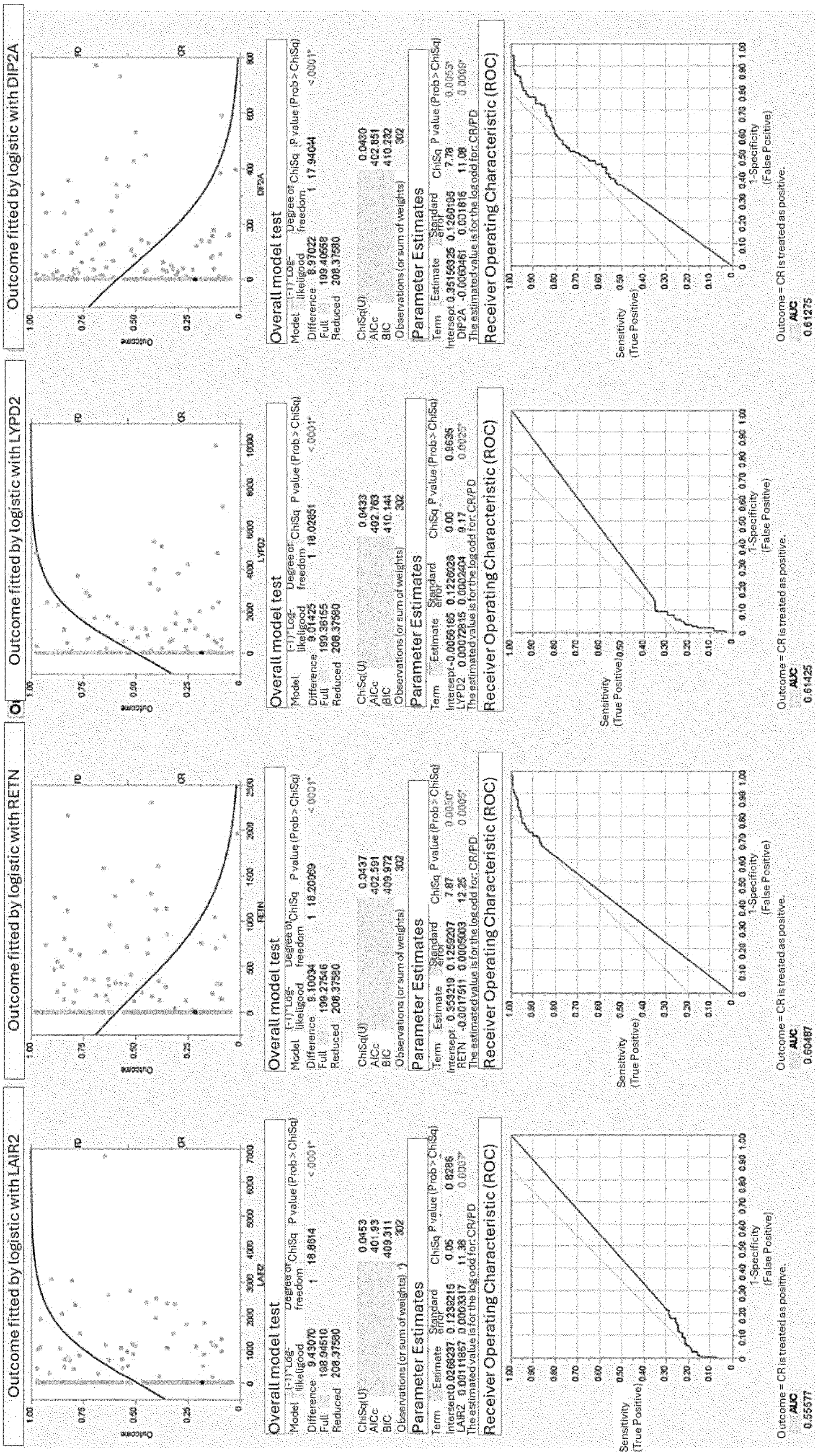
Outcome fitted by logistic with LAIR2
Outcome fitted by logistic with RETN
Outcome fitted by logistic with LYPD2
Outcome fitted by logistic with DIP2A
Overall model test
Parameter Estimates
Receiver Operating Characteristic (ROC)
Outcome = CR is treated as positive.
AUC 0.55577
AUC 0.60487
AUC 0.61425
AUC 0.61275

FIG. 4D

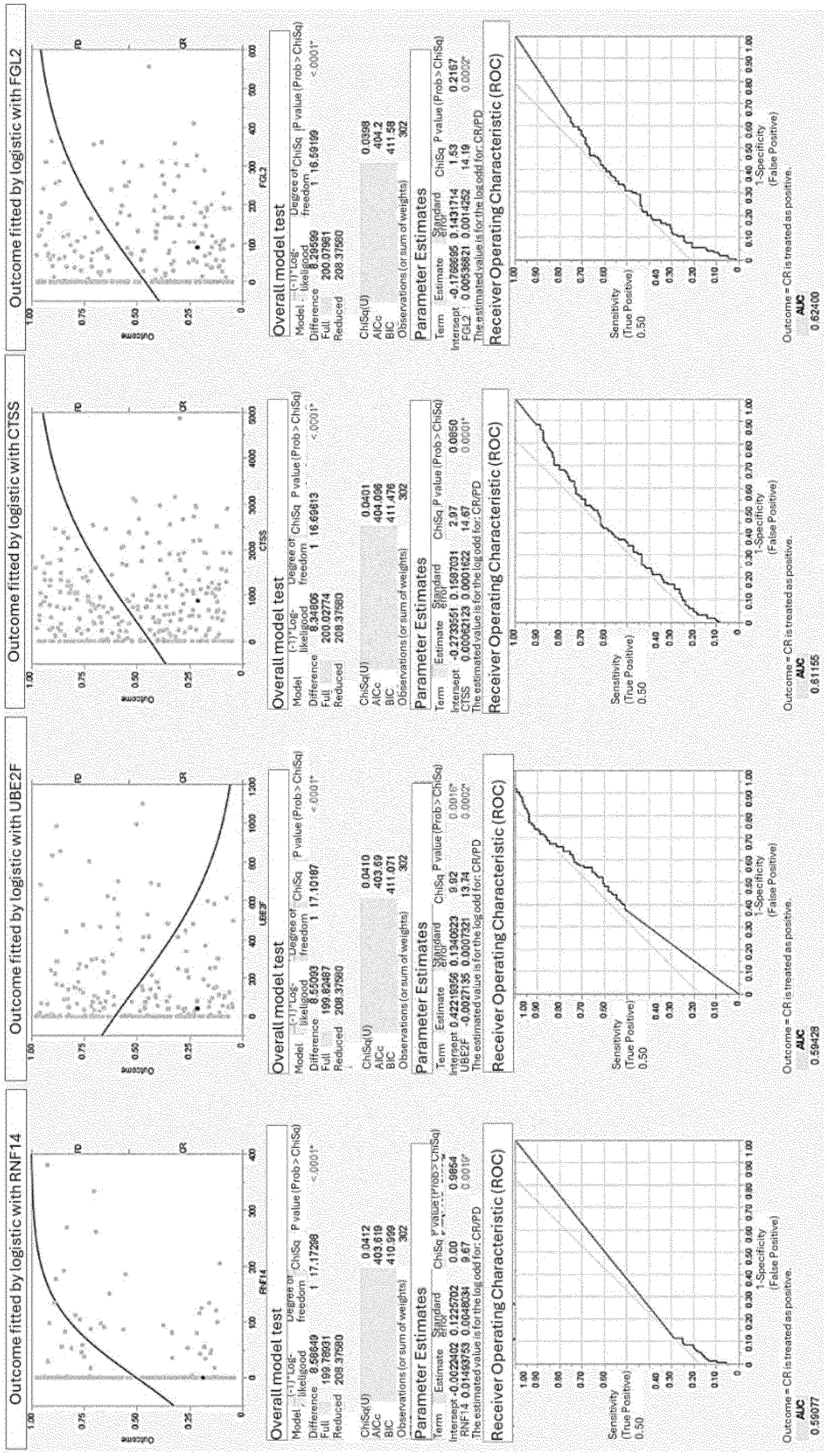
Outcome fitted by logistic with RNF14
Outcome fitted by logistic with UBE2F
Outcome fitted by logistic with CTSS
Outcome fitted by logistic with FGL2
Overall model test
Parameter Estimates
Receiver Operating Characteristic (ROC)

FIG. 4E

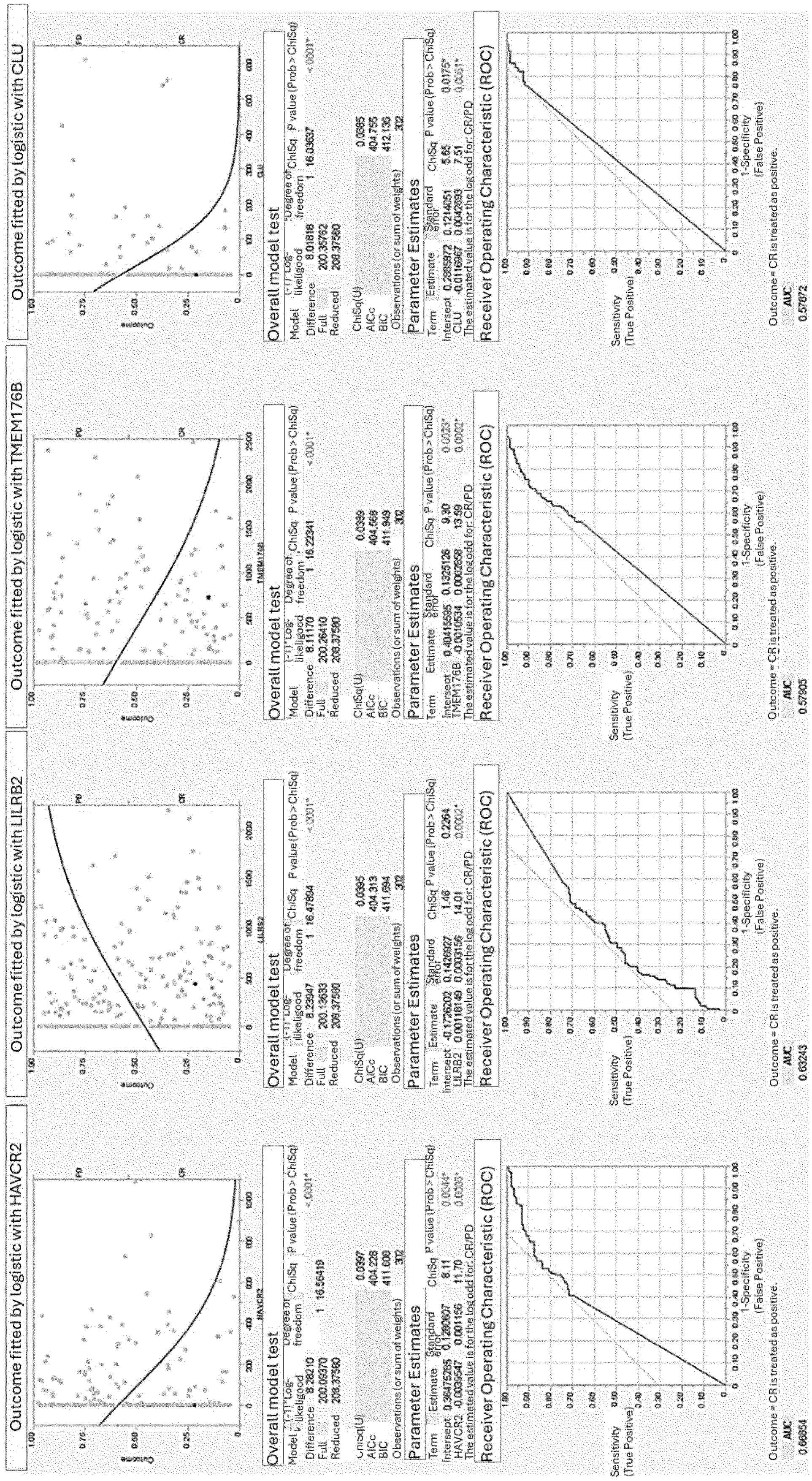
Outcome fitted by logistic with HAVCR2
Overall model test
Parameter Estimates
Receiver Operating Characteristic (ROC)
AUC
0.66854
Outcome fitted by logistic with LILRB2
Overall model test
Parameter Estimates
Receiver Operating Characteristic (ROC)
AUC
0.63243
Outcome fitted by logistic with TMEM176B
Overall model test
Parameter Estimates
Receiver Operating Characteristic (ROC)
AUC
0.57905
Outcome fitted by logistic with CLU
Overall model test
Parameter Estimates
Receiver Operating Characteristic (ROC)
AUC
0.57872

FIG. 4F

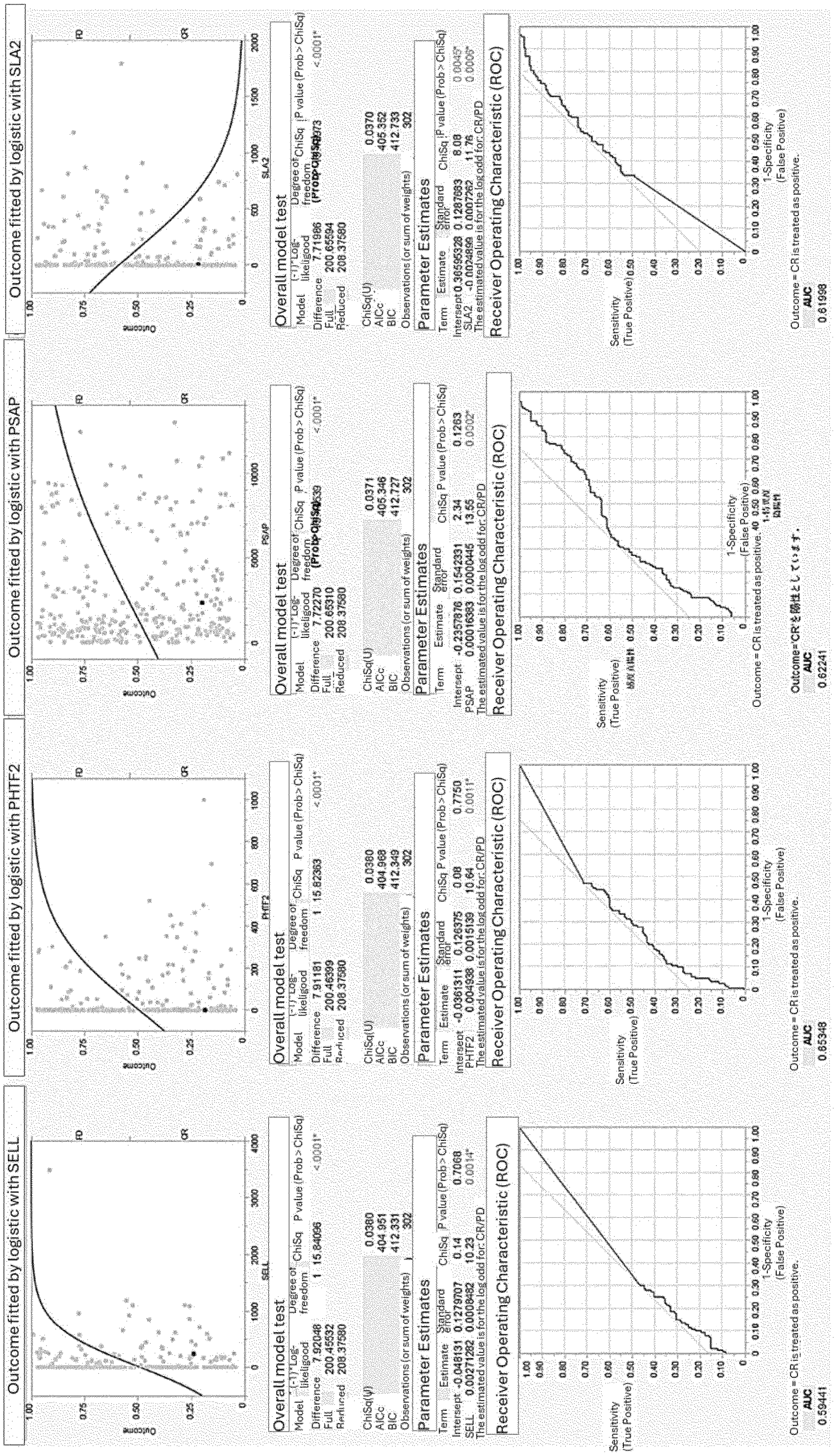
Outcome fitted by logistic with SELL
Overall model test
Parameter Estimates
Receiver Operating Characteristic (ROC)
AUC
0.59441
Outcome fitted by logistic with PHTF2
Overall model test
Parameter Estimates
Receiver Operating Characteristic (ROC)
AUC
0.65348
Outcome fitted by logistic with PSAP
Overall model test
Parameter Estimates
Receiver Operating Characteristic (ROC)
AUC
0.62241
Outcome fitted by logistic with SLA2
Overall model test
Parameter Estimates
Receiver Operating Characteristic (ROC)
AUC
0.61998

FIG. 4G

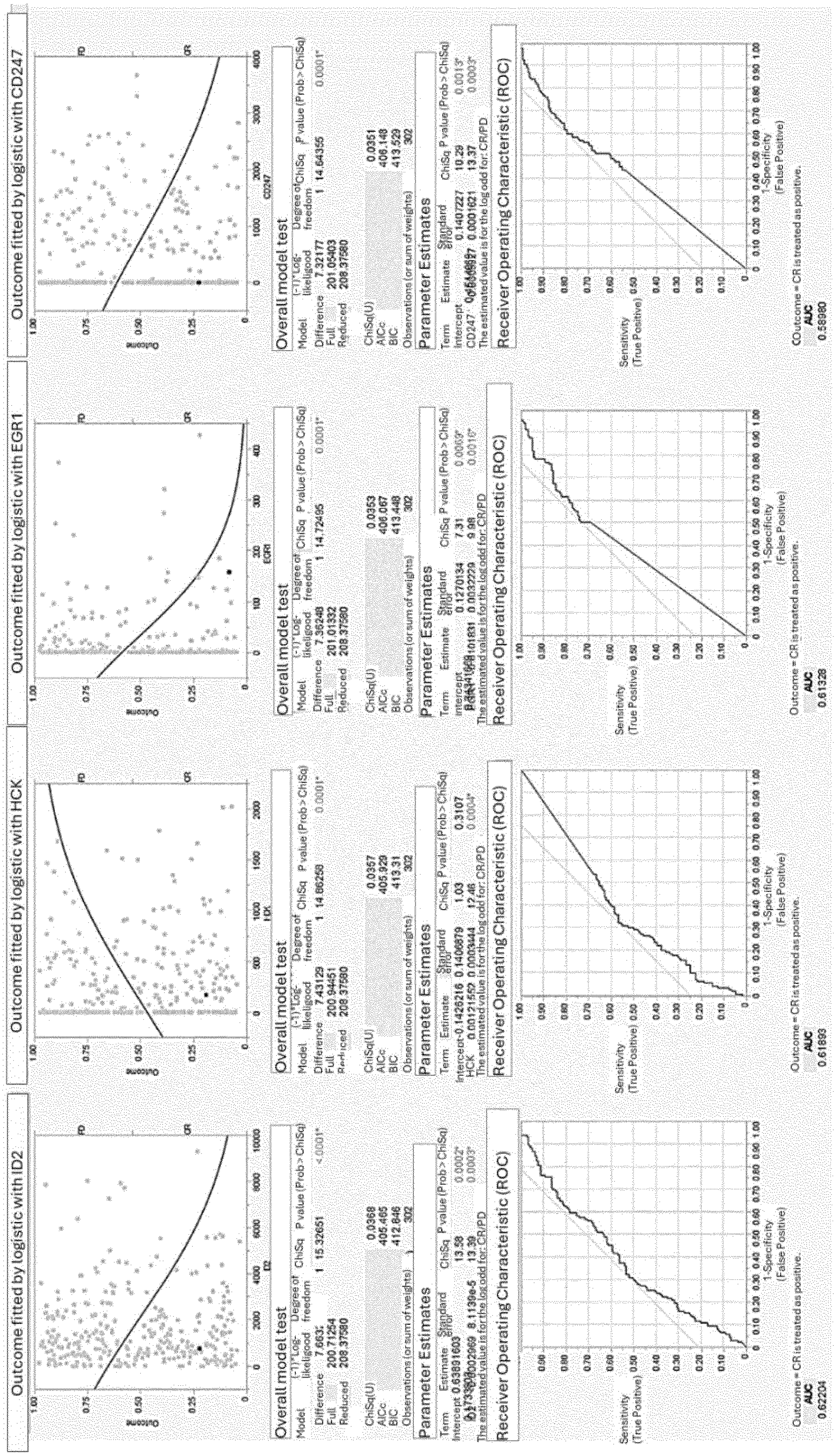
Outcome fitted by logistic with ID2
Overall model test
Parameter Estimates
Receiver Operating Characteristic (ROC)
AUC
0.62204
Outcome fitted by logistic with HCK
Overall model test
Parameter Estimates
Receiver Operating Characteristic (ROC)
AUC
0.61893
Outcome fitted by logistic with EGR1
Overall model test
Parameter Estimates
Receiver Operating Characteristic (ROC)
AUC
0.61328
Outcome fitted by logistic with CD247
Overall model test
Parameter Estimates
Receiver Operating Characteristic (ROC)
AUC
0.58980

FIG. 4H

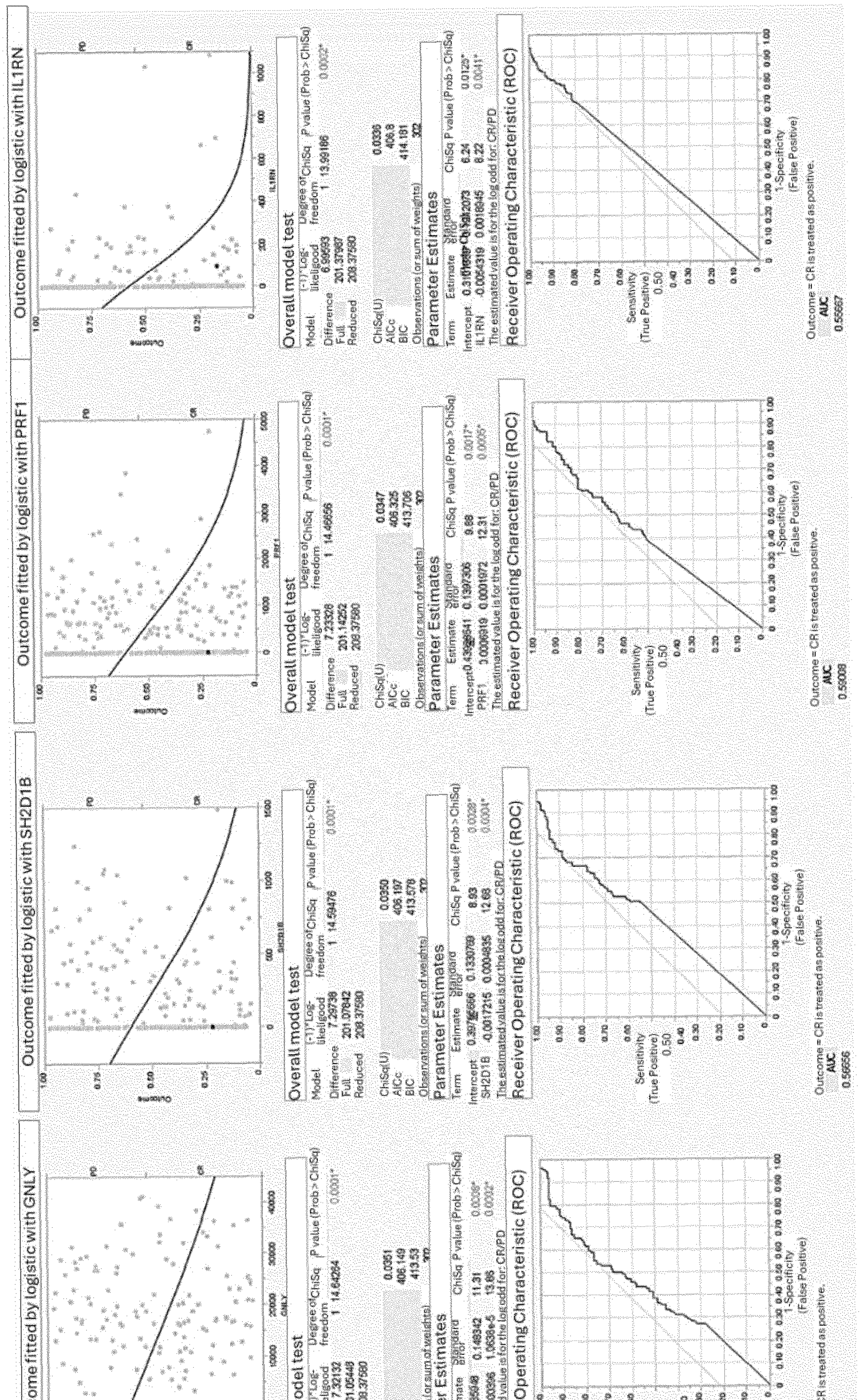
Outcome fitted by logistic with GNLY
Overall model test
Parameter Estimates
Receiver Operating Characteristic (ROC)
Outcome = CR is treated as positive.
AUC
0.59110
Outcome fitted by logistic with SH2D1B
Overall model test
Parameter Estimates
Receiver Operating Characteristic (ROC)
Outcome = CR is treated as positive.
AUC
0.56656
Outcome fitted by logistic with PRF1
Overall model test
Parameter Estimates
Receiver Operating Characteristic (ROC)
Outcome = CR is treated as positive.
AUC
0.59009
Outcome fitted by logistic with IL1RN
Overall model test
Parameter Estimates
Receiver Operating Characteristic (ROC)
Outcome = CR is treated as positive.
AUC
0.55667

FIG. 4I

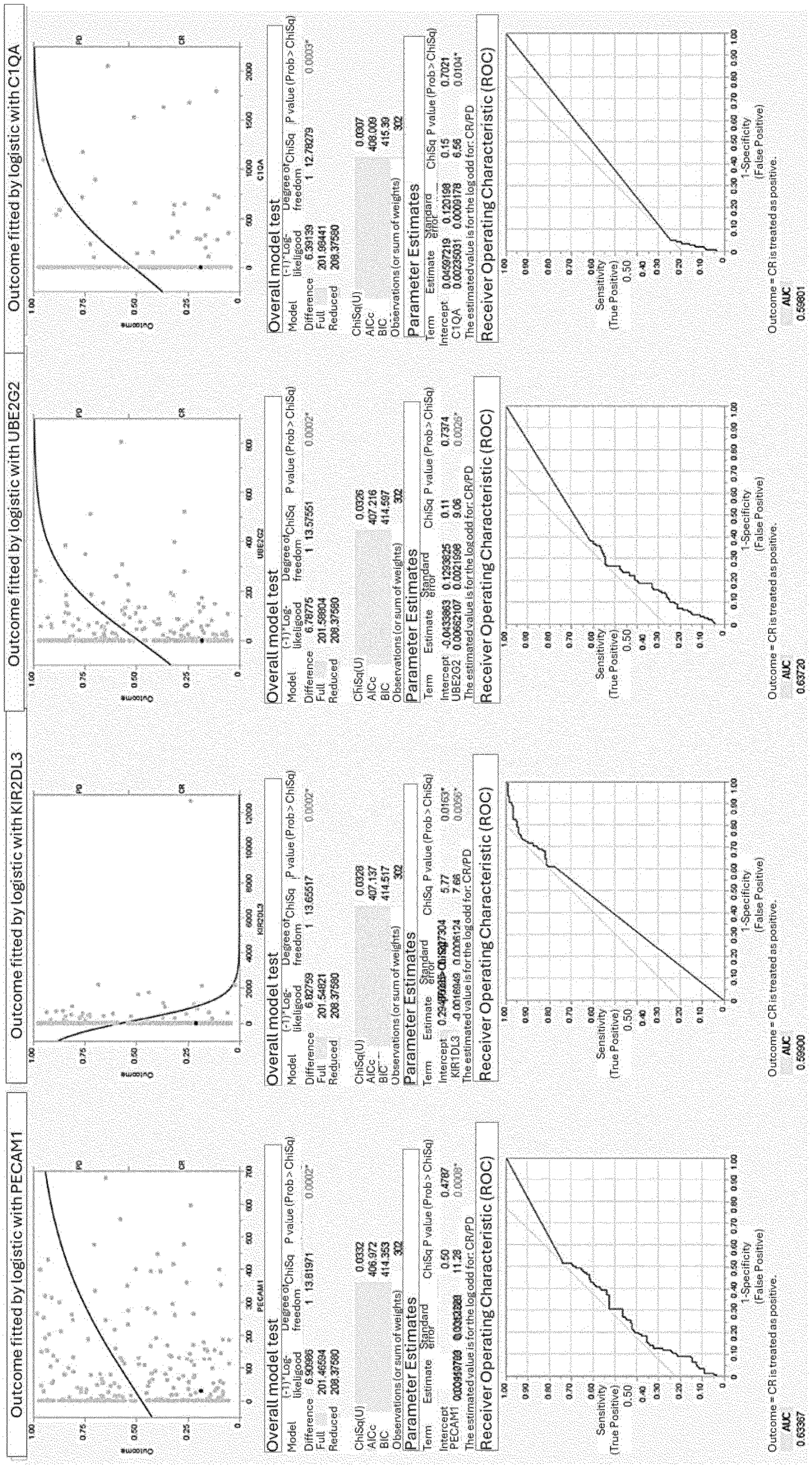
Outcome fitted by logistic with PECAM1
Outcome fitted by logistic with KIR2DL3
Outcome fitted by logistic with UBE2G2
Outcome fitted by logistic with C1QA

FIG. 4J

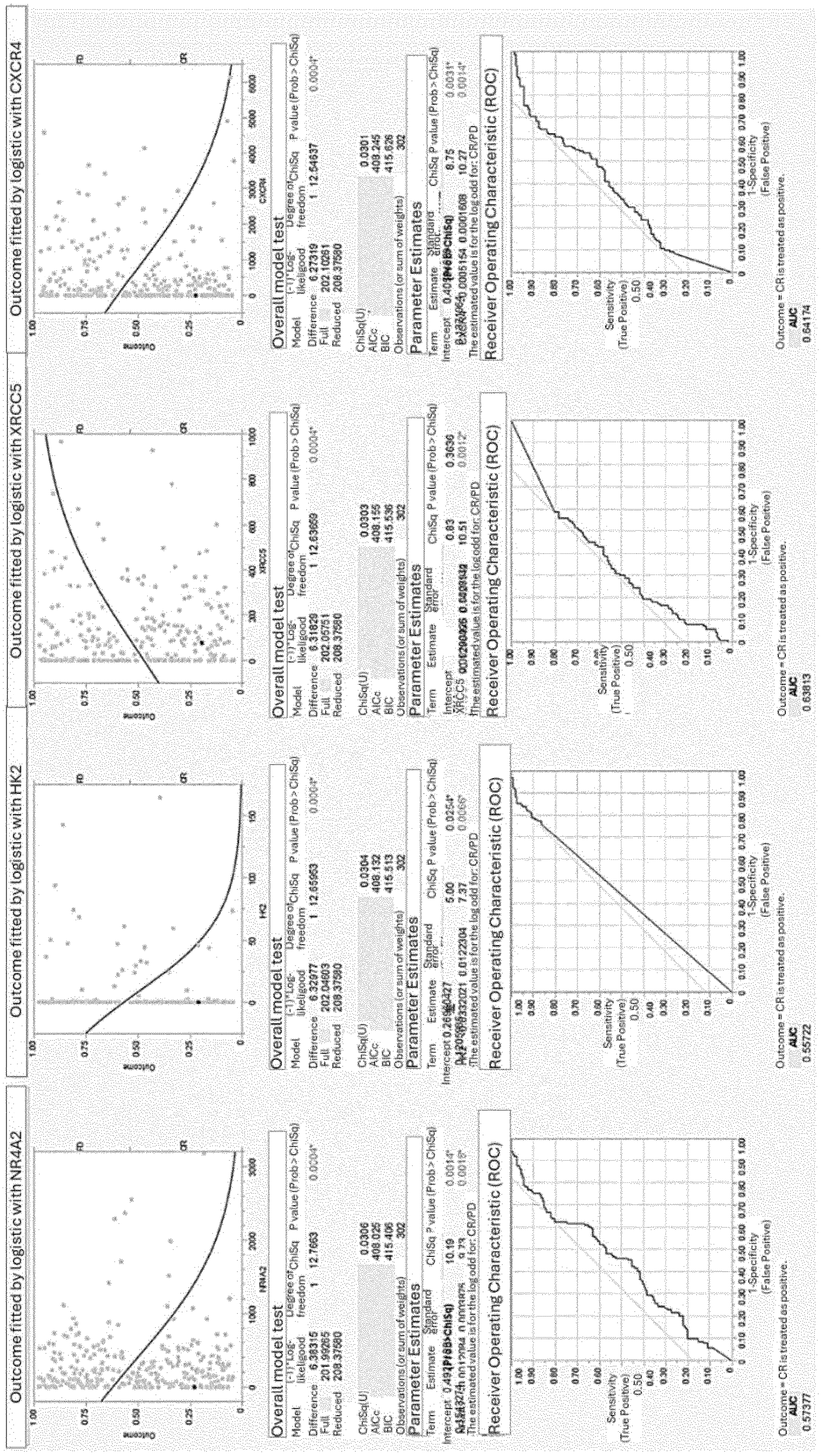
Outcome fitted by logistic with NR4A2
Outcome fitted by logistic with HK2
Outcome fitted by logistic with XRCC5
Outcome fitted by logistic with CXCR4
Overall model test
Parameter Estimates
Receiver Operating Characteristic (ROC)

FIG. 4K

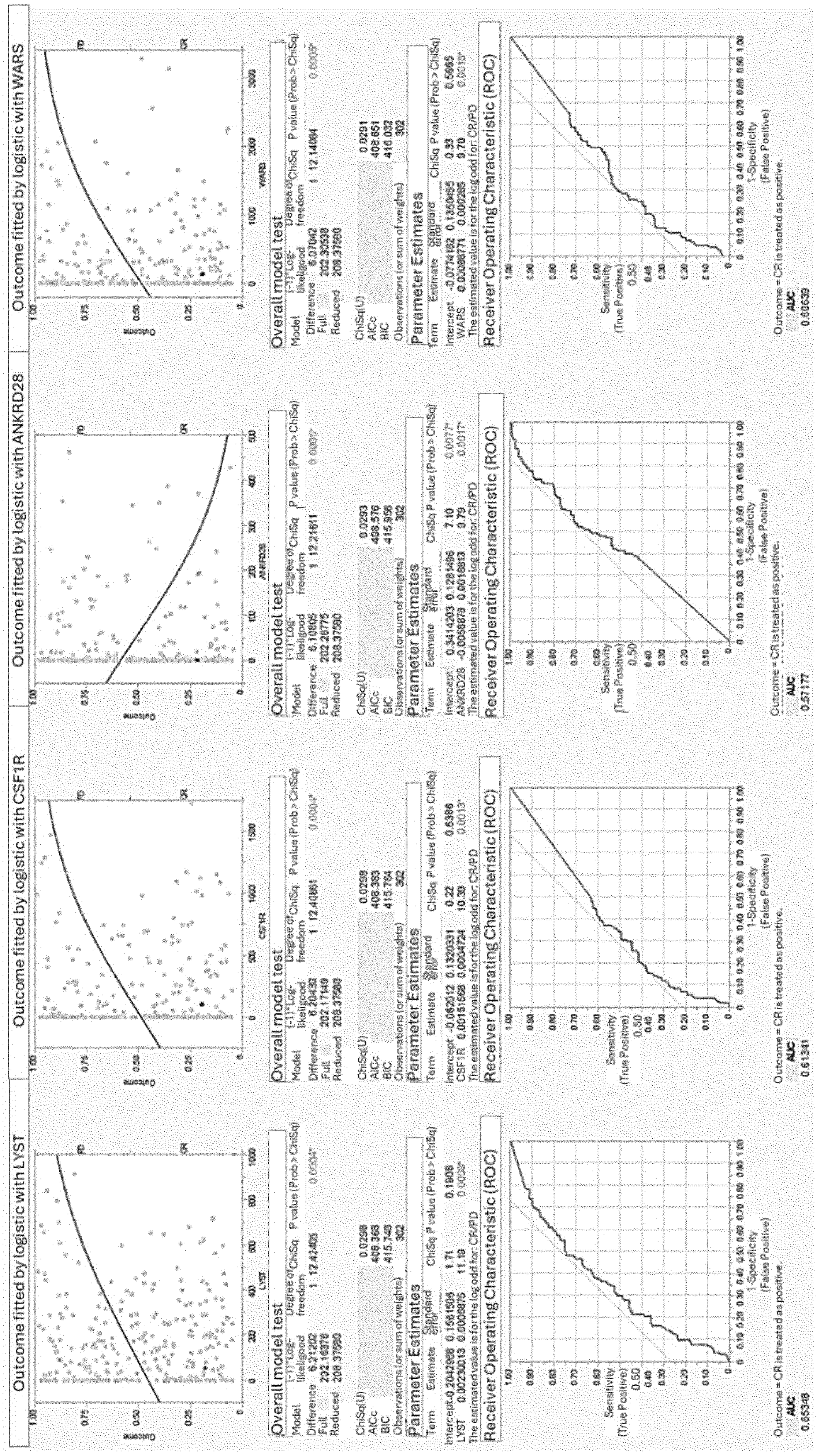

Outcome fitted by logistic with LYST
Outcome fitted by logistic with CSF1R
Outcome fitted by logistic with ANKRD28
Outcome fitted by logistic with WARS
Overall model test
Parameter Estimates
Receiver Operating Characteristic (ROC)


FIG. 4L

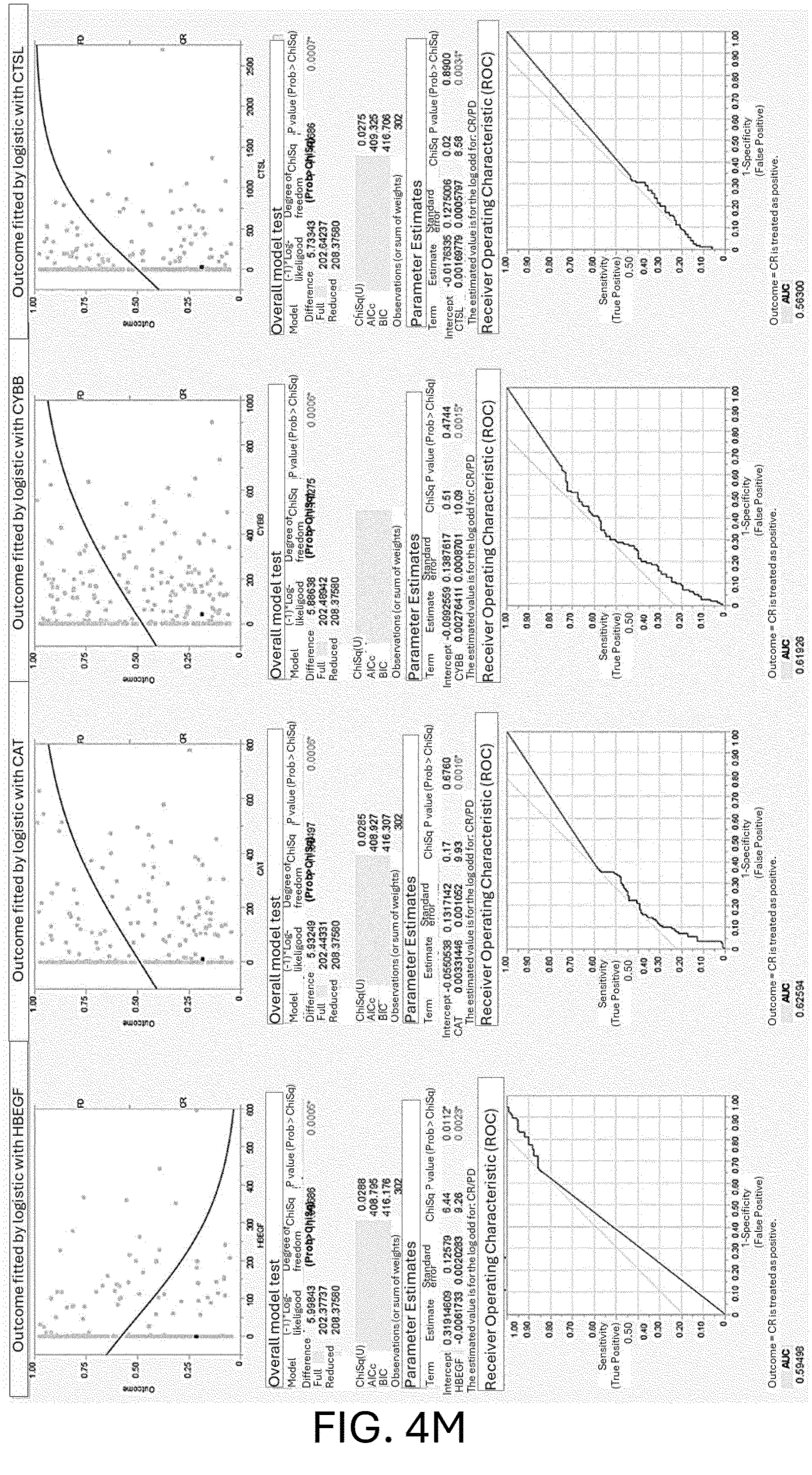
Outcome fitted by logistic with HBEGF
Outcome fitted by logistic with CAT
Outcome fitted by logistic with CYBB
Outcome fitted by logistic with CTSL

FIG. 4M

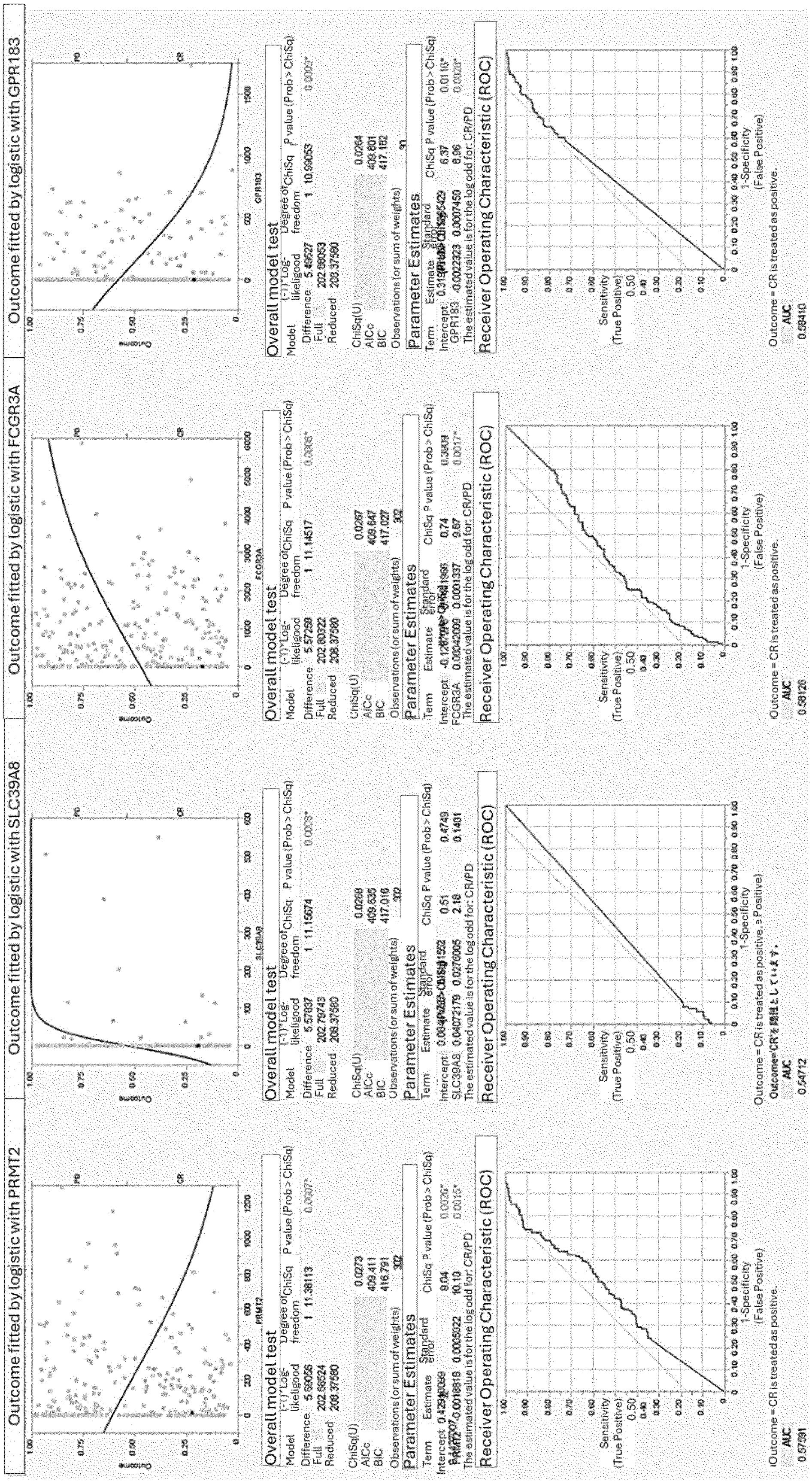
Outcome fitted by logistic with PRMT2
Outcome fitted by logistic with SLC39A8
Outcome fitted by logistic with FCGR3A
Outcome fitted by logistic with GPR183
Overall model test
Parameter Estimates
Receiver Operating Characteristic (ROC)
Outcome = CR is treated as positive.

FIG. 4N

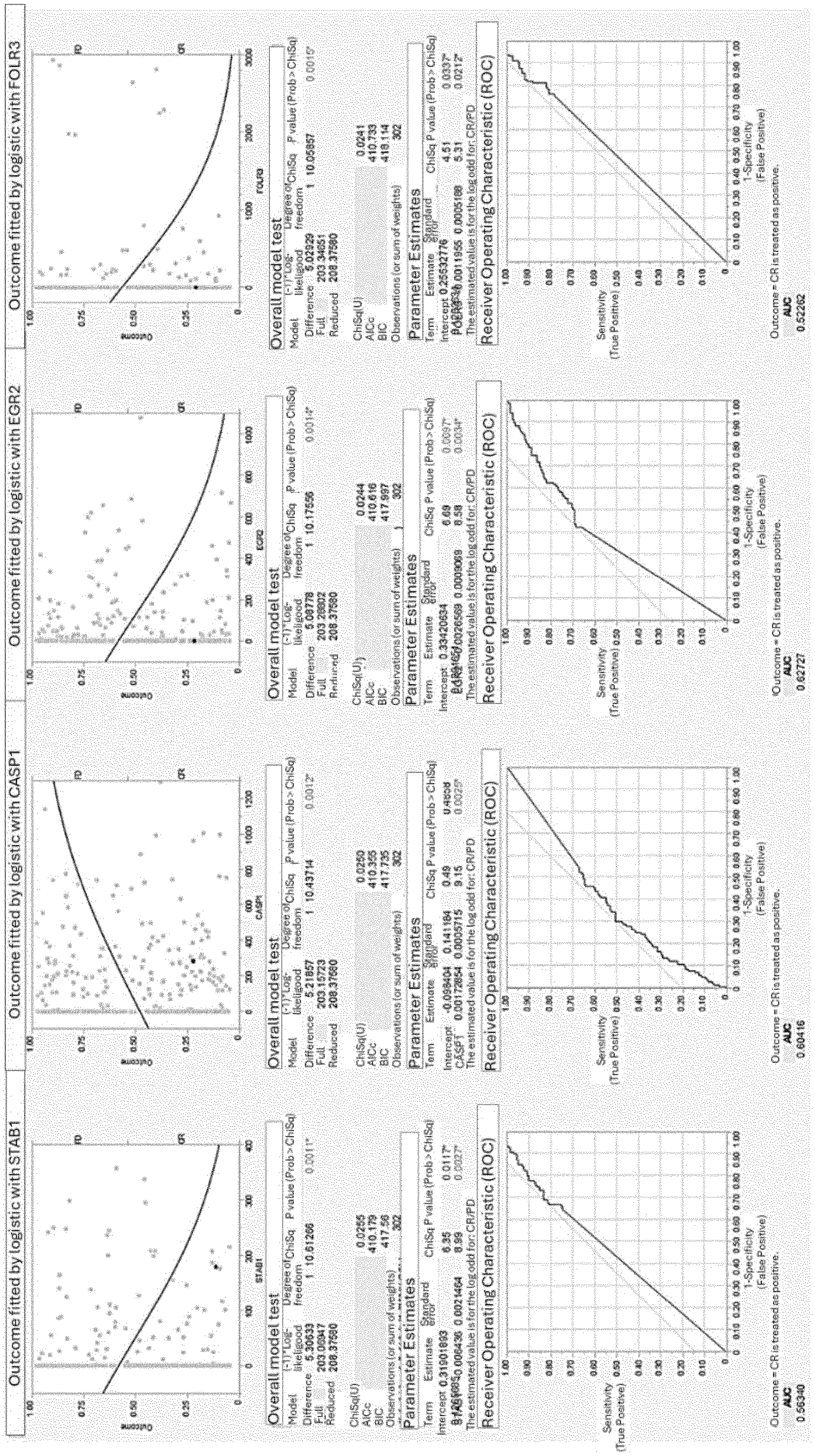
Outcome fitted by logistic with STAB1
Outcome fitted by logistic with CASP1
Outcome fitted by logistic with EGR2
Outcome fitted by logistic with FOLR3
Overall model test
Parameter Estimates
Receiver Operating Characteristic (ROC)

FIG. 4O

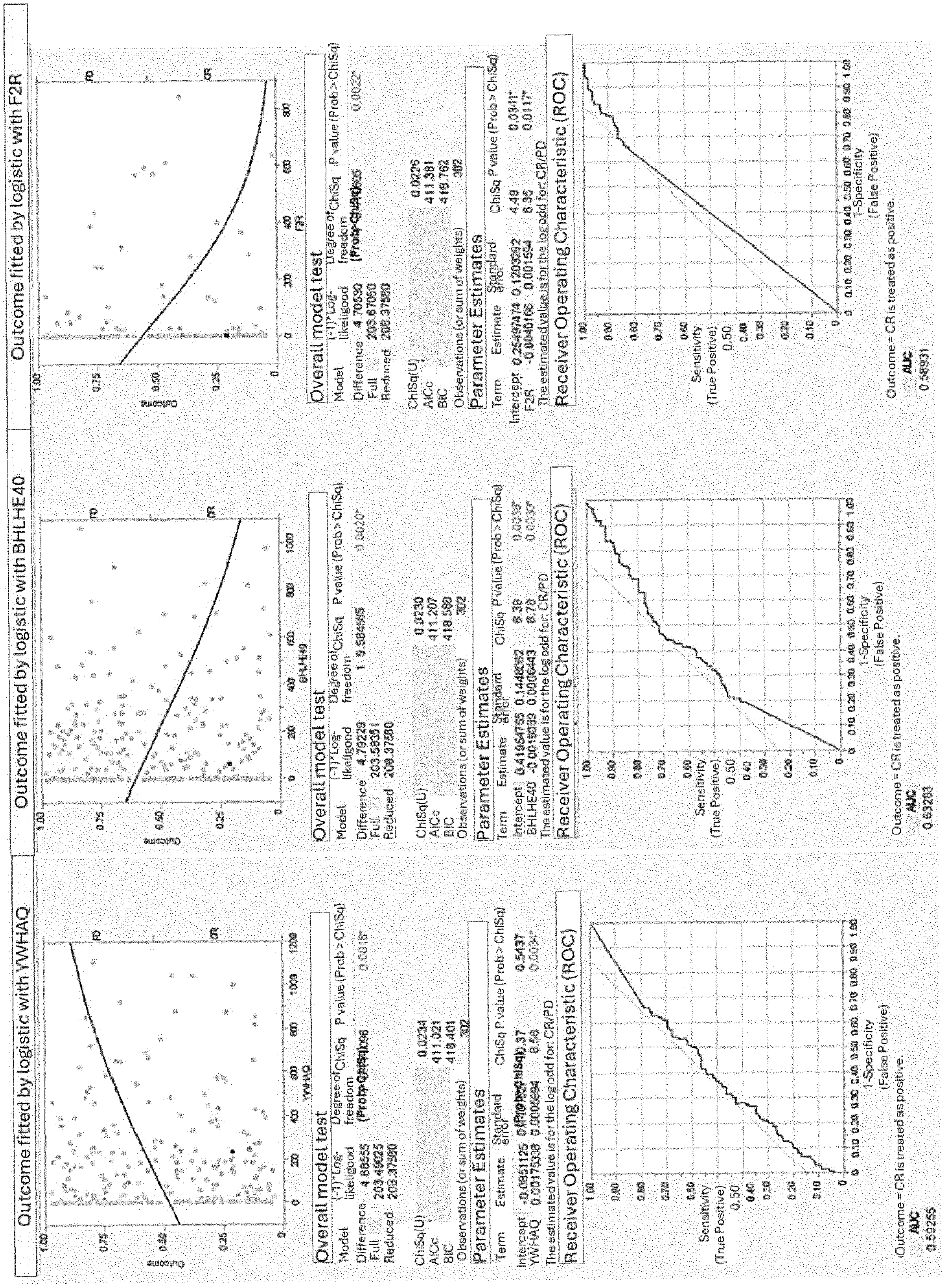
Outcome fitted by logistic with YWHAQ
Outcome
YWHAQ
PD
CR
Overall model test
Model (-1)*Log-likeligood Degree of freedom ChiSq (Prob>ChiSq) P value (Prob > ChiSq)
Difference 4.88555
Full 203.49025
Reduced 208.37580
0.0018*
ChiSq(U) 0.0234
AICc 411.021
BIC 418.401
Observations (or sum of weights) 302
Parameter Estimates
Term Estimate Standard error ChiSq P value (Prob > ChiSq)
Intercept -0.0851125
YWHAQ 0.00175338 0.0005994 8.56 0.0034*
0.5437
The estimated value is for the log odd for: CR/PD
Receiver Operating Characteristic (ROC)
Sensitivity (True Positive)
1-Specificity (False Positive)
Outcome = CR is treated as positive.
AUC
0.59255
Outcome fitted by logistic with BHLHE40
Outcome
BHLHE40
PD
CR
Overall model test
Model (-1)*Log-likeligood Degree of freedom ChiSq P value (Prob > ChiSq)
Difference 4.79229 1 9.584585 0.0020*
Full 203.58351
Reduced 208.37580
ChiSq(U) 0.0230
AICc 411.207
BIC 418.588
Observations (or sum of weights) 302
Parameter Estimates
Term Estimate Standard error ChiSq P value (Prob > ChiSq)
Intercept 0.41954765 0.1448062 8.39 0.0038*
BHLHE40 -0.0019089 0.0006443 8.78 0.0030*
The estimated value is for the log odd for: CR/PD
Receiver Operating Characteristic (ROC)
Sensitivity (True Positive)
1-Specificity (False Positive)
Outcome = CR is treated as positive.
AUC
0.63283
Outcome fitted by logistic with F2R
Outcome
F2R
PD
CR
Overall model test
Model (-1)*Log-likeligood Degree of freedom ChiSq (Prob>ChiSq) P value (Prob > ChiSq)
Difference 4.70530
Full 203.67050
Reduced 208.37580
0.0022*
ChiSq(U) 0.0226
AICc 411.381
BIC 418.762
Observations (or sum of weights) 302
Parameter Estimates
Term Estimate Standard error ChiSq P value (Prob > ChiSq)
Intercept 0.25497474 0.1203292 4.49 0.0341*
F2R -0.0040166 0.001594 6.35 0.0117*
The estimated value is for the log odd for: CR/PD
Receiver Operating Characteristic (ROC)
Sensitivity (True Positive)
1-Specificity (False Positive)
Outcome = CR is treated as positive.
AUC
0.58931

FIG. 4P

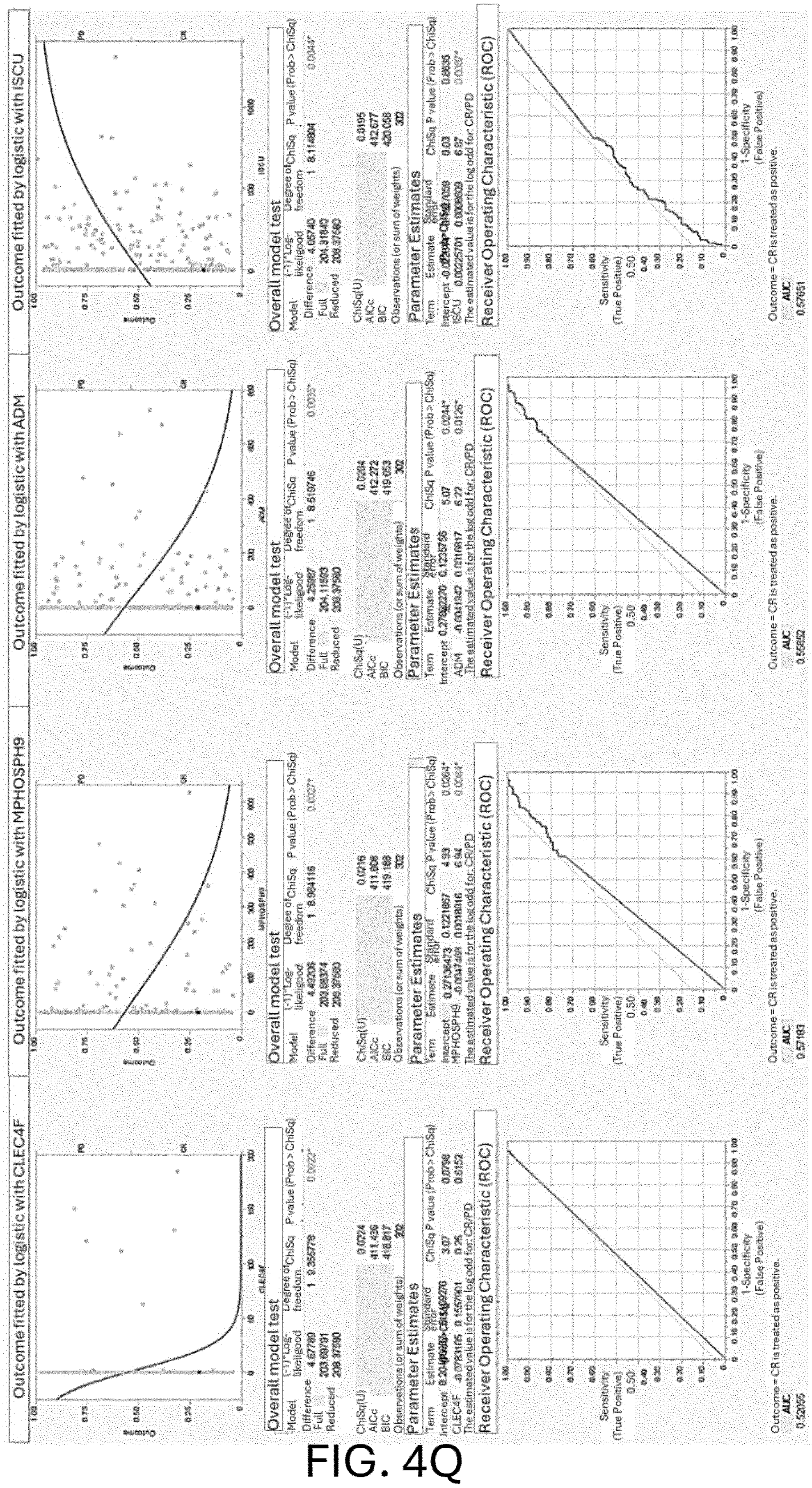
Outcome fitted by logistic with CLEC4F
Outcome fitted by logistic with MPHOSPH9
Outcome fitted by logistic with ADM
Outcome fitted by logistic with ISCU
Overall model test
Parameter Estimates
Receiver Operating Characteristic (ROC)
Outcome = CR is treated as positive.

FIG. 4Q

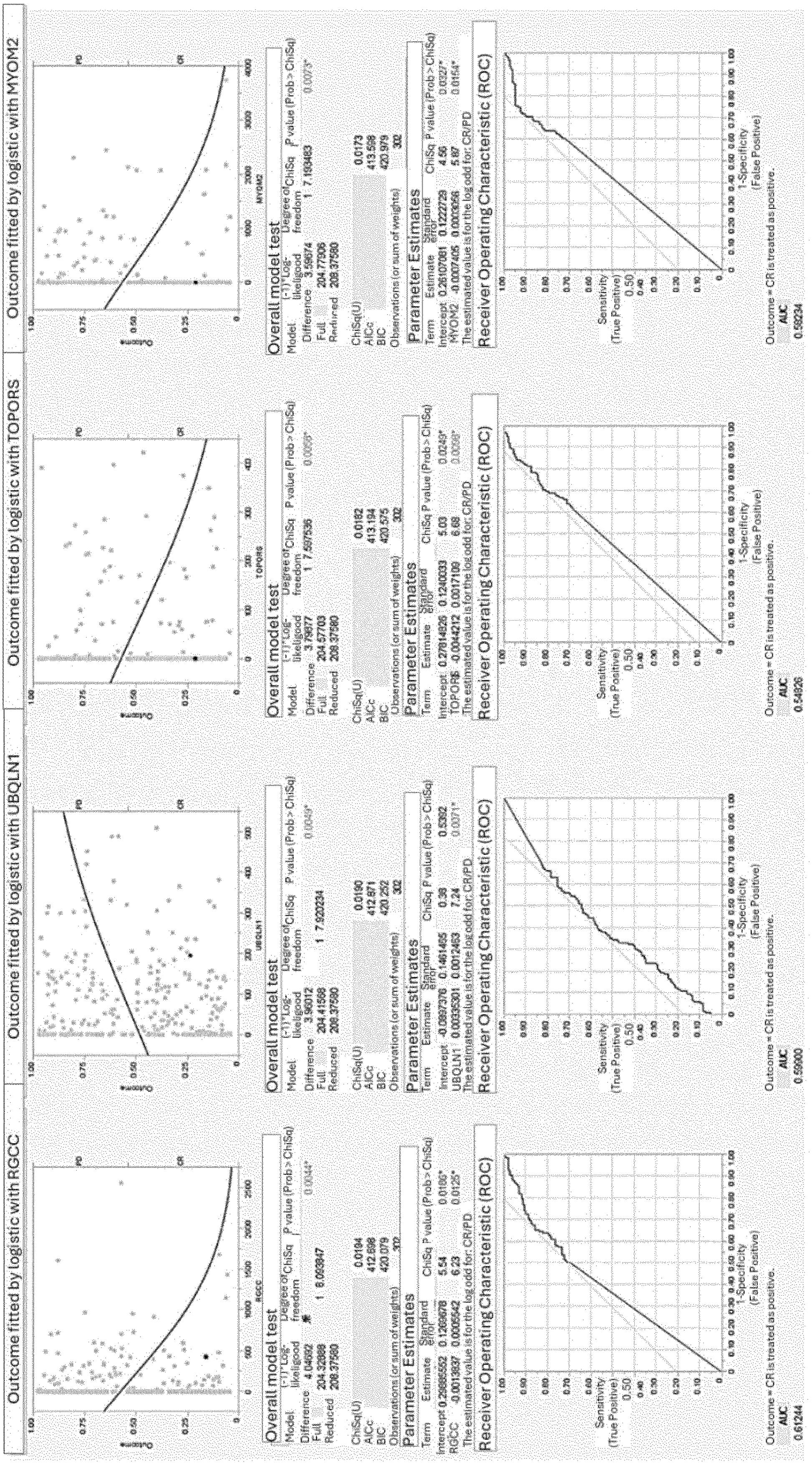
Outcome fitted by logistic with RGCC
Outcome fitted by logistic with UBQLN1
Outcome fitted by logistic with TOPORS
Outcome fitted by logistic with MYOM2
Overall model test
Parameter Estimates
Receiver Operating Characteristic (ROC)
Outcome = CR is treated as positive.
AUC
0.61244
0.59000
0.54926
0.59234

FIG. 4R

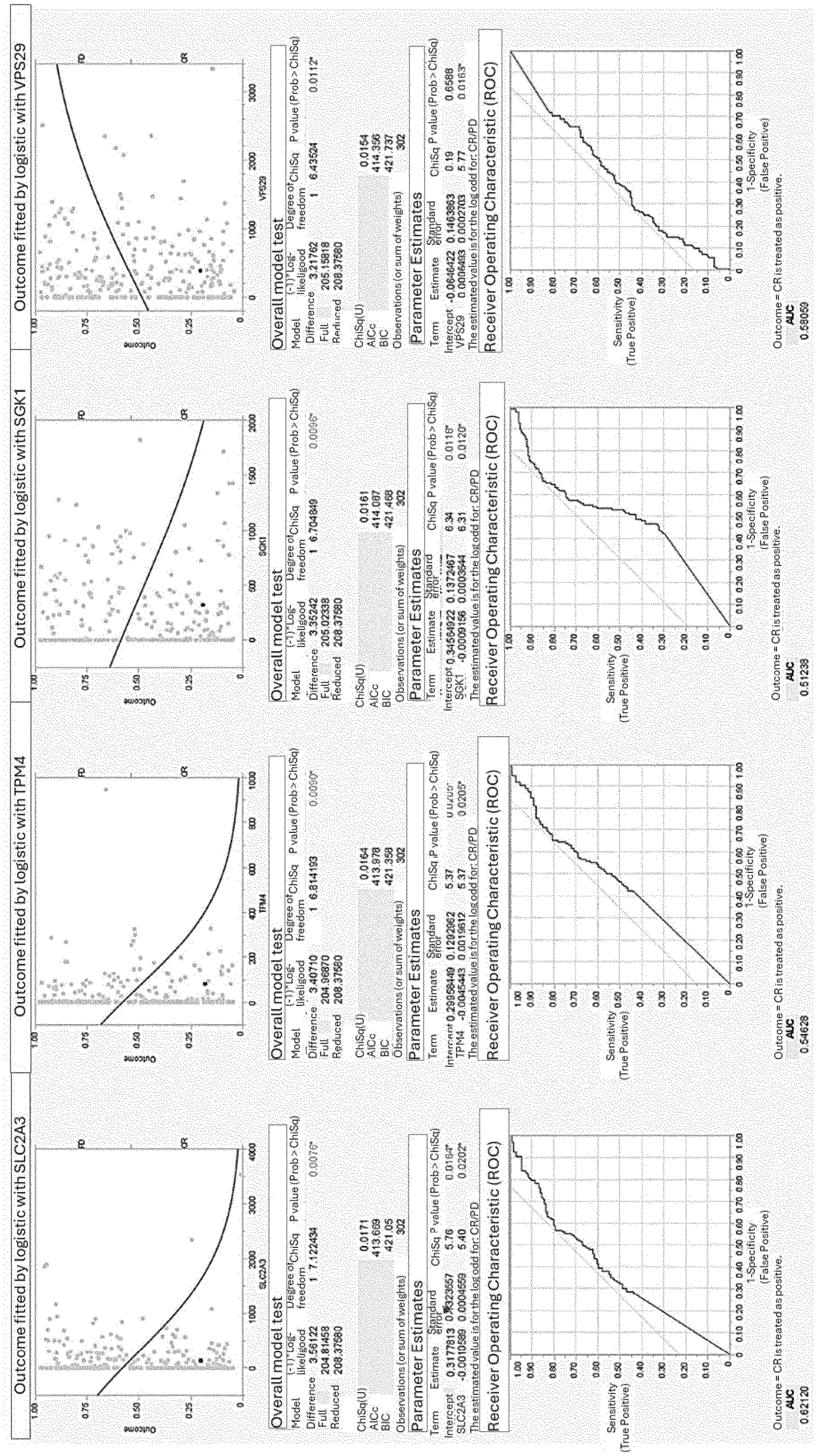
Outcome fitted by logistic with SLC2A3
Overall model test
Model (-1)*Log-likelihood Degree of freedom ChiSq P value (Prob > ChiSq)
Difference 3.56122 1 7.122434 0.0076*
Full 204.81458
Reduced 208.37580
ChiSq(U) 0.0171
AICc 413.669
BIC 421.05
Observations (or sum of weights) 302
Parameter Estimates
Term Estimate Standard error ChiSq P value (Prob > ChiSq)
Intercept 0.3177813 0.1323557 5.76 0.0164*
SLC2A3 -0.0010589 0.0004559 5.40 0.0202*
The estimated value is for the log odd for: CR/PD
Receiver Operating Characteristic (ROC)
Sensitivity (True Positive)
1-Specificity (False Positive)
Outcome = CR is treated as positive.
AUC 0.62120
Outcome fitted by logistic with TPM4
Overall model test
Difference 3.40710 1 6.814193 0.0090*
Full 204.96870
Reduced 208.37580
ChiSq(U) 0.0164
AICc 413.978
BIC 421.358
Observations (or sum of weights) 302
Parameter Estimates
Intercept 0.29959449 0.1292962 5.37 0.0205*
TPM4 -0.0045443 0.0019612 5.37 0.0205*
The estimated value is for the log odd for: CR/PD
Receiver Operating Characteristic (ROC)
Outcome = CR is treated as positive.
AUC 0.54629
Outcome fitted by logistic with SGK1
Overall model test
Difference 3.35242 1 6.704849 0.0096*
Full 205.02338
Reduced 208.37580
ChiSq(U) 0.0161
AICc 414.087
BIC 421.468
Observations (or sum of weights) 302
Parameter Estimates
Intercept 0.34554922 0.13724667 6.34 0.0118*
SGK1 -0.0009156 0.0003644 6.31 0.0120*
The estimated value is for the log odd for: CR/PD
Receiver Operating Characteristic (ROC)
Outcome = CR is treated as positive.
AUC 0.51233
Outcome fitted by logistic with VPS29
Overall model test
Difference 3.21762 1 6.43524 0.0112*
Full 205.15818
Reduced 208.37580
ChiSq(U) 0.0154
AICc 414.356
BIC 421.737
Observations (or sum of weights) 302
Parameter Estimates
Intercept -0.0646422 0.1463863 0.19 0.6588
VPS29 0.0006493 0.0002703 5.77 0.0163*
The estimated value is for the log odd for: CR/PD
Receiver Operating Characteristic (ROC)
Outcome = CR is treated as positive.
AUC 0.58059

FIG. 4S

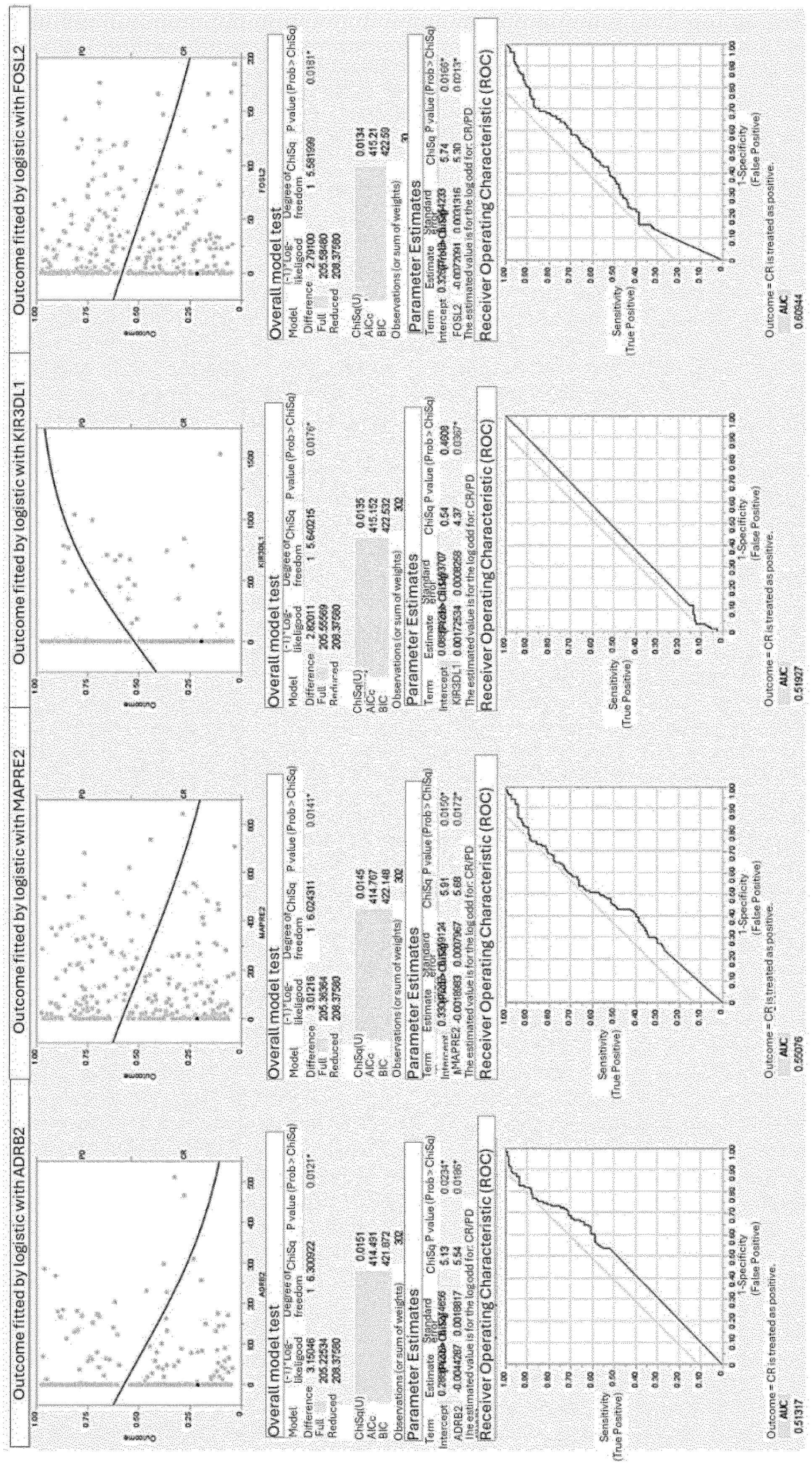
Outcome fitted by logistic with ADRB2
Outcome fitted by logistic with MAPRE2
Outcome fitted by logistic with KIR3DL1
Outcome fitted by logistic with FOSL2

FIG. 4T

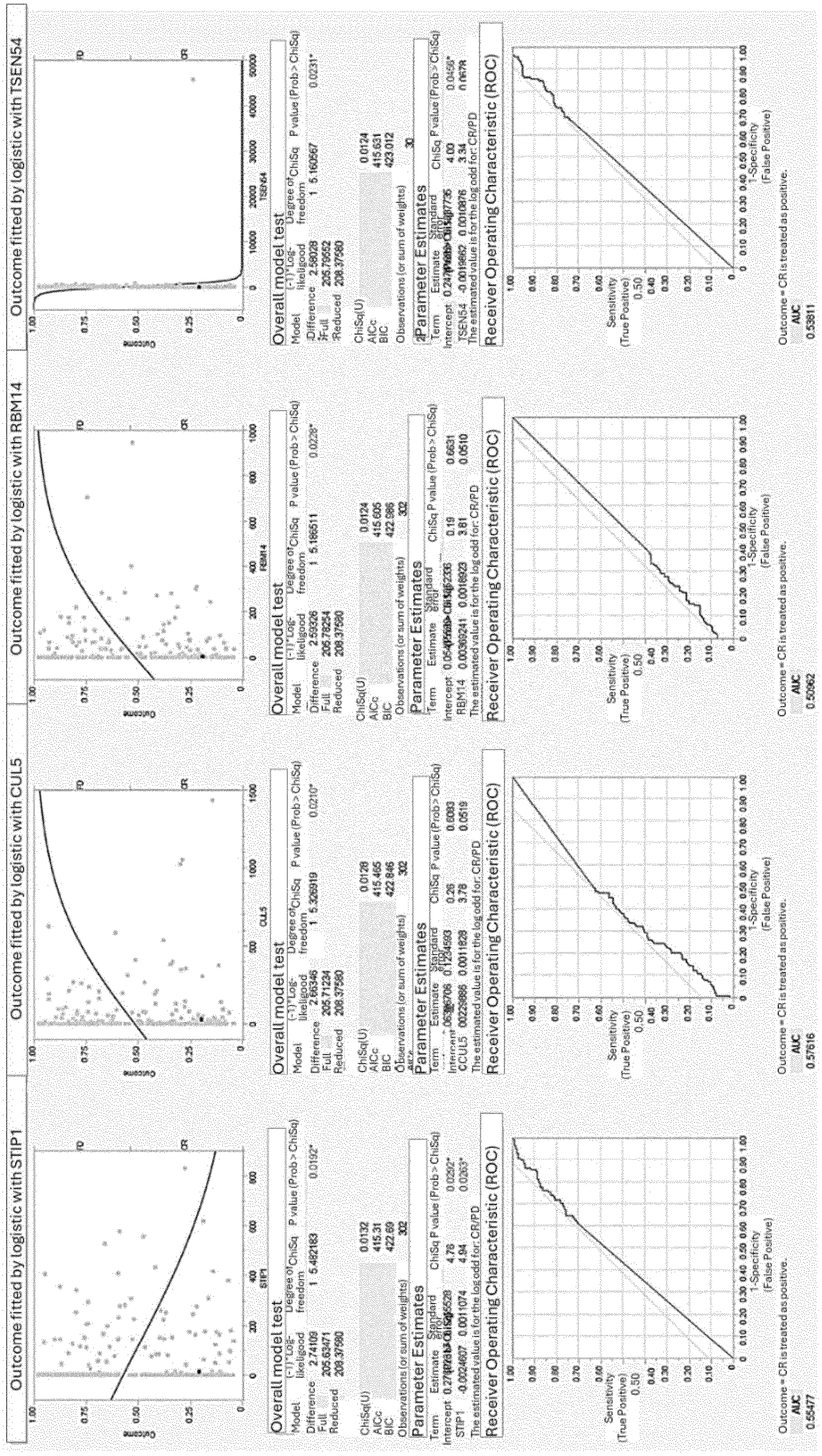

Outcome fitted by logistic with STIP1
Outcome fitted by logistic with CUL5
Outcome fitted by logistic with RBM14
Outcome fitted by logistic with TSEN54
Overall model test
Parameter Estimates
Receiver Operating Characteristic (ROC)
Outcome = CR is treated as positive.
AUC
0.55477
0.57616
0.50962
0.53811


FIG. 4U

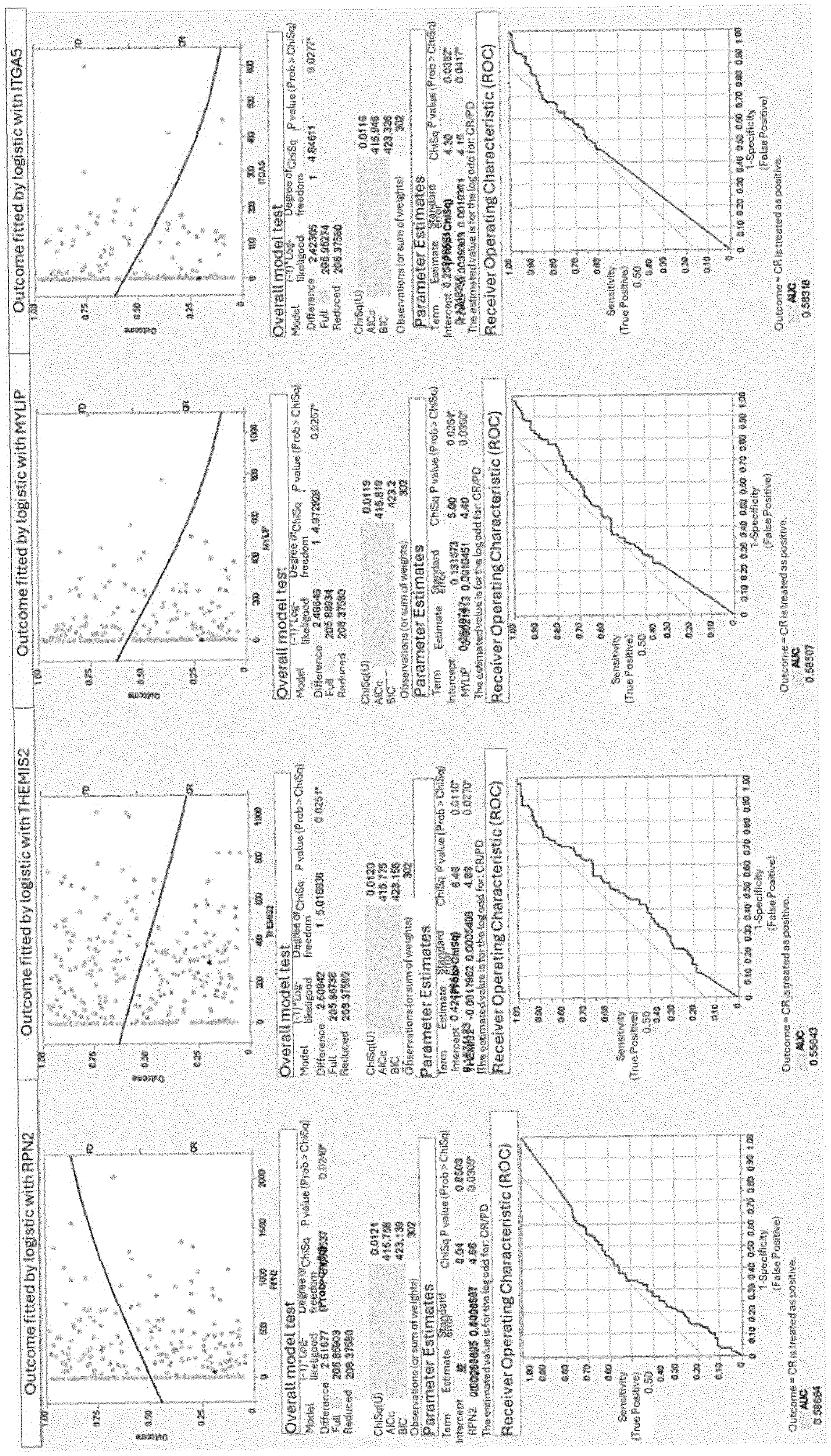
Outcome fitted by logistic with RPN2
Outcome fitted by logistic with THEMIS2
Outcome fitted by logistic with MYLIP
Outcome fitted by logistic with ITGA5
Overall model test
Parameter Estimates
Receiver Operating Characteristic (ROC)
Outcome = CR is treated as positive.
AUC
0.58684
0.55643
0.58507
0.58318

FIG. 4V

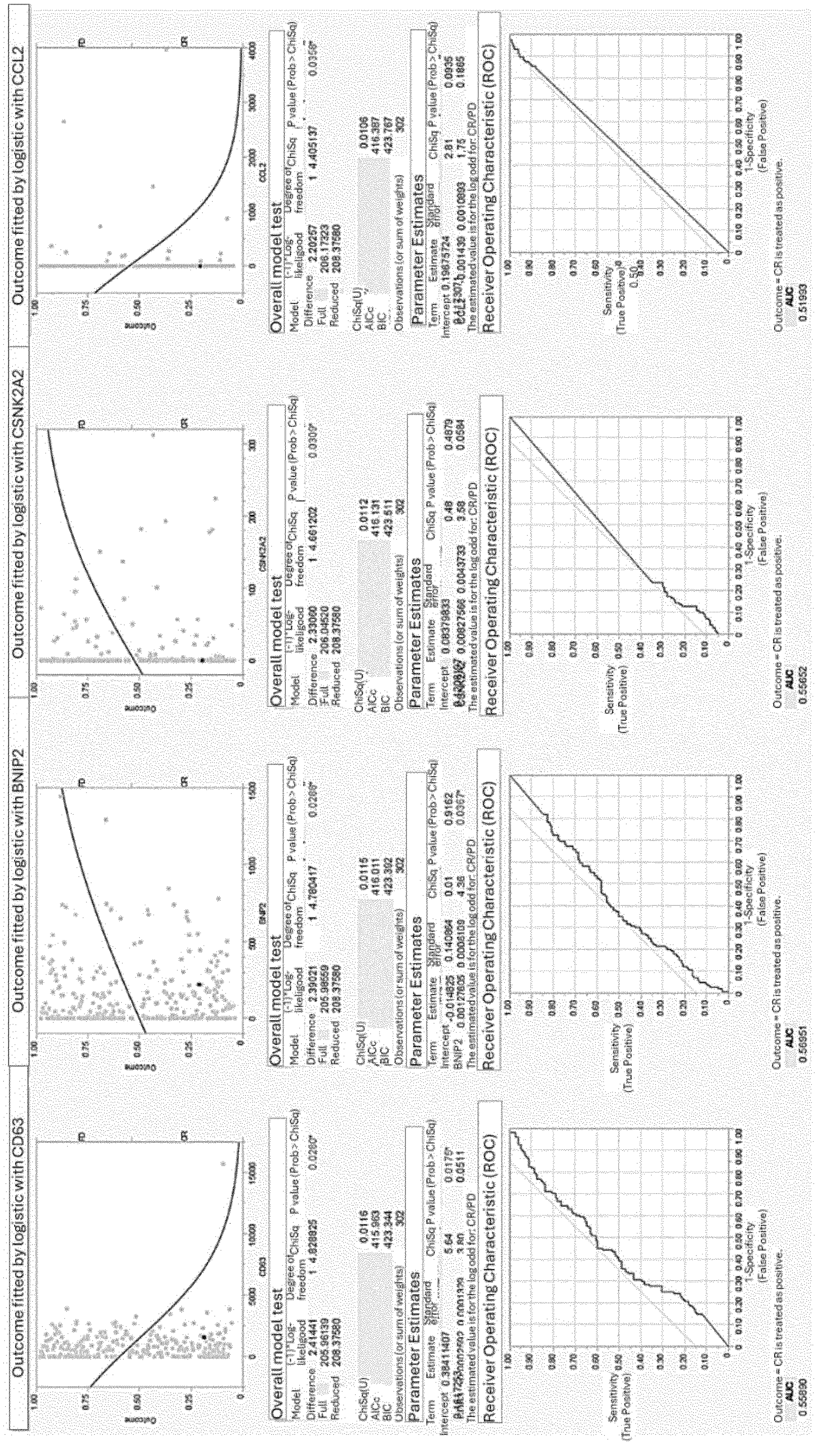
Outcome fitted by logistic with CD63
Outcome fitted by logistic with BNIP2
Outcome fitted by logistic with CSNK2A2
Outcome fitted by logistic with CCL2

FIG. 4W

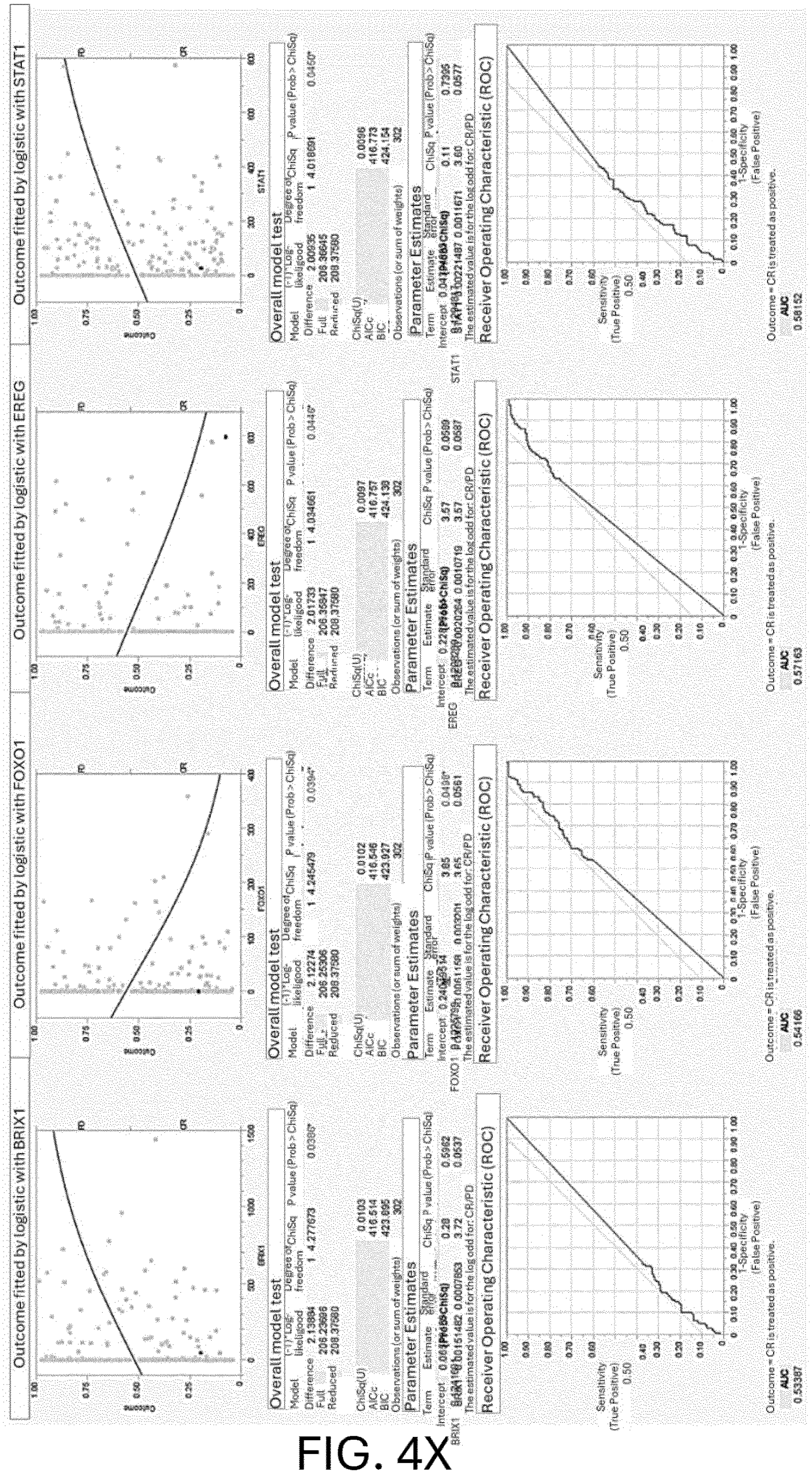
Outcome fitted by logistic with BRIX1
Outcome fitted by logistic with FOXO1
Outcome fitted by logistic with EREG
Outcome fitted by logistic with STAT1
Overall model test
Parameter Estimates
Receiver Operating Characteristic (ROC)
Sensitivity (True Positive)
1-Specificity (False Positive)
Outcome = CR is treated as positive.
AUC
0.53387
0.54166
0.57163
0.58152

FIG. 4X

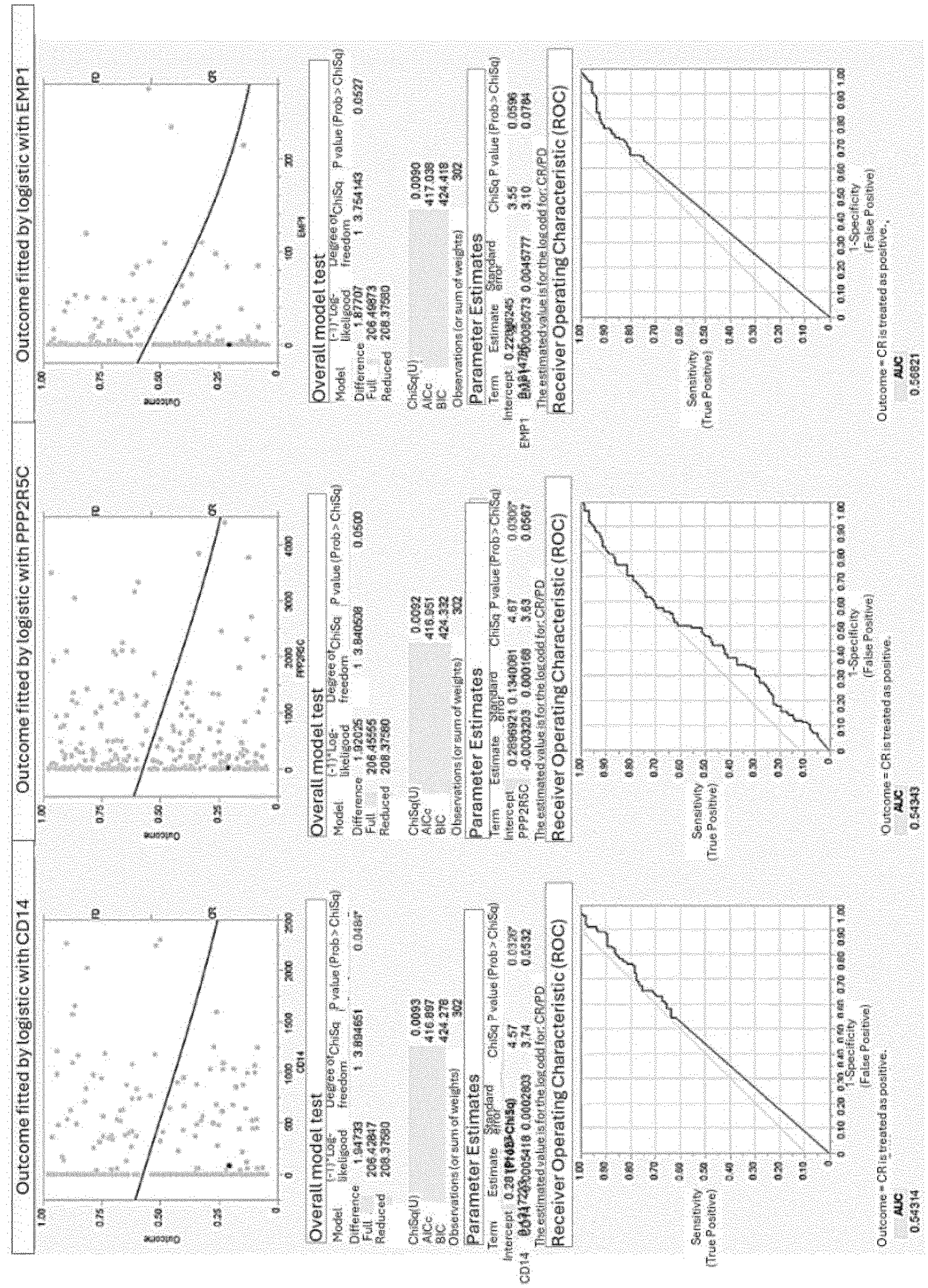
Outcome fitted by logistic with CD14
Outcome fitted by logistic with PPP2R5C
Outcome fitted by logistic with EMP1
Overall model test
Parameter Estimates
Receiver Operating Characteristic (ROC)

FIG. 4Y

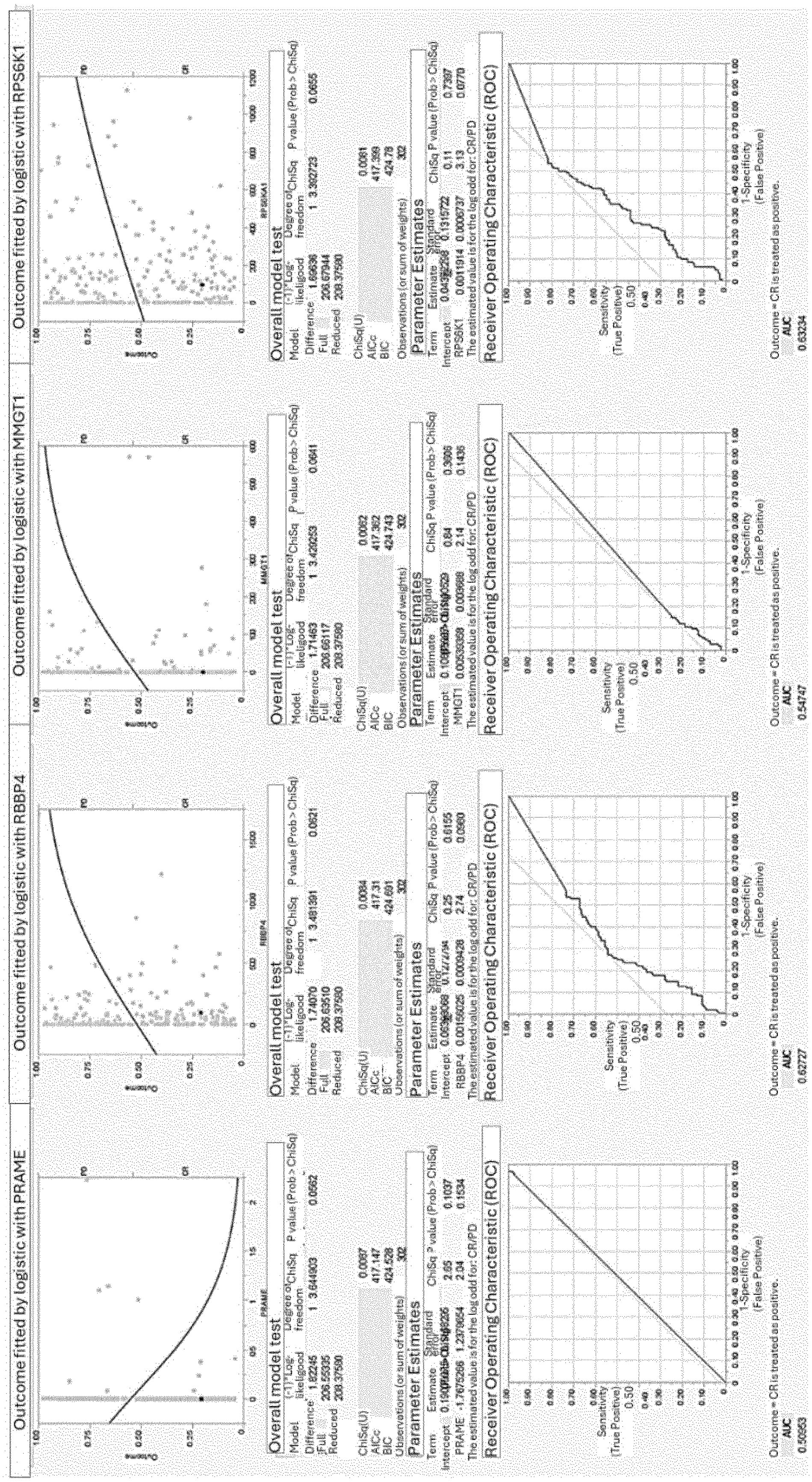
Outcome fitted by logistic with PRAME
Outcome fitted by logistic with RBBP4
Outcome fitted by logistic with MMGT1
Outcome fitted by logistic with RPS6K1
Overall model test
Parameter Estimates
Receiver Operating Characteristic (ROC)
Sensitivity (True Positive)
1-Specificity (False Positive)
Outcome = CR is treated as positive.

FIG. 4Z

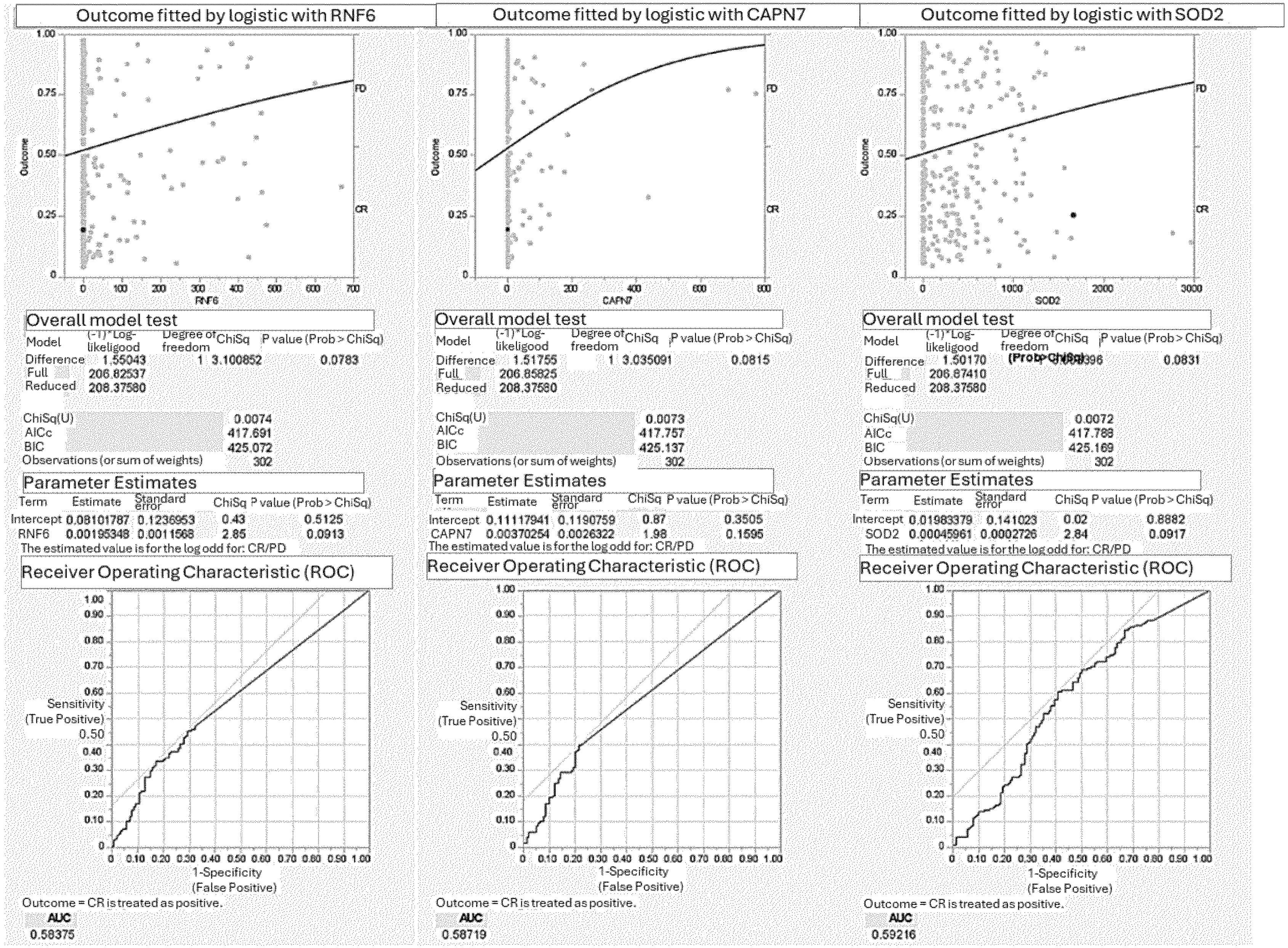
Outcome fitted by logistic with RNF6
Outcome
1.00
0.75
0.50
0.25
0
PD
CR
0 100 200 300 400 500 600 700
RNF6
Overall model test
Model (-1)*Log-likeligood Degree of freedom ChiSq P value (Prob > ChiSq)
Difference 1.55043 1 3.100852 0.0783
Full 206.82537
Reduced 208.37580
ChiSq(U) 0.0074
AICc 417.691
BIC 425.072
Observations (or sum of weights) 302
Parameter Estimates
Term Estimate Standard error ChiSq P value (Prob > ChiSq)
Intercept 0.08101787 0.1236953 0.43 0.5125
RNF6 0.00195348 0.0011568 2.85 0.0913
The estimated value is for the log odd for: CR/PD
Receiver Operating Characteristic (ROC)
Sensitivity (True Positive)
1-Specificity (False Positive)
Outcome = CR is treated as positive.
AUC
0.58375
Outcome fitted by logistic with CAPN7
0 200 400 600 800
CAPN7
Overall model test
Model (-1)*Log-likeligood Degree of freedom ChiSq P value (Prob > ChiSq)
Difference 1.51755 1 3.035091 0.0815
Full 206.85825
Reduced 208.37580
ChiSq(U) 0.0073
AICc 417.757
BIC 425.137
Observations (or sum of weights) 302
Parameter Estimates
Term Estimate Standard error ChiSq P value (Prob > ChiSq)
Intercept 0.11117941 0.1190759 0.87 0.3505
CAPN7 0.00370254 0.0026322 1.98 0.1595
The estimated value is for the log odd for: CR/PD
Receiver Operating Characteristic (ROC)
Outcome = CR is treated as positive.
AUC
0.58719
Outcome fitted by logistic with SOD2
0 1000 2000 3000
SOD2
Overall model test
Model (-1)*Log-likeligood Degree of freedom ChiSq P value (Prob > ChiSq)
Difference 1.50170 0.0831
Full 206.87410
Reduced 208.37580
ChiSq(U) 0.0072
AICc 417.788
BIC 425.169
Observations (or sum of weights) 302
Parameter Estimates
Term Estimate Standard error ChiSq P value (Prob > ChiSq)
Intercept 0.01983379 0.141023 0.02 0.8882
SOD2 0.00045961 0.0002726 2.84 0.0917
The estimated value is for the log odd for: CR/PD
Receiver Operating Characteristic (ROC)
Outcome = CR is treated as positive.
AUC
0.59216

FIG. 4AA

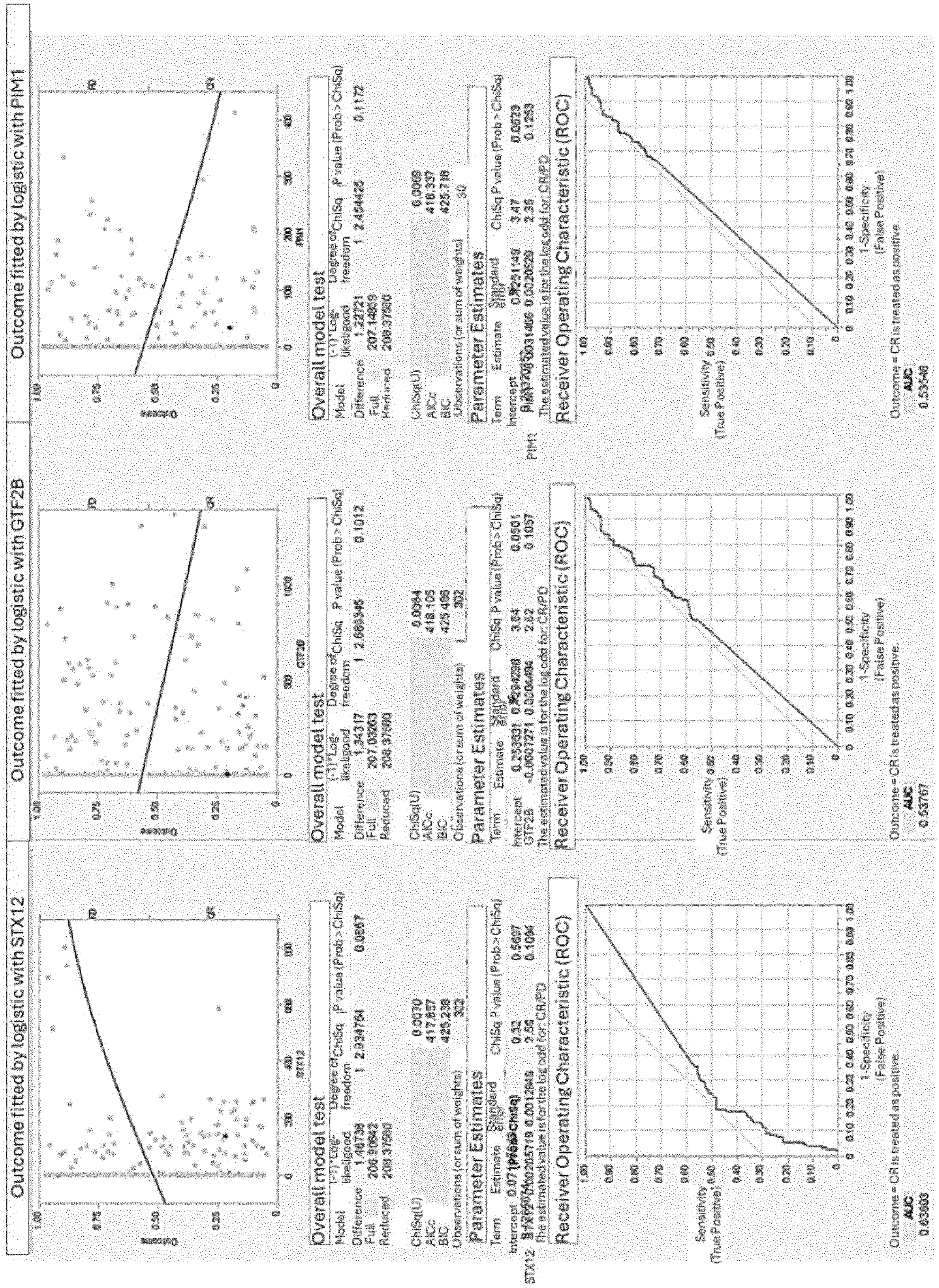
Outcome fitted by logistic with STX12
Outcome fitted by logistic with GTF2B
Outcome fitted by logistic with PIM1
Overall model test
Parameter Estimates
Receiver Operating Characteristic (ROC)
Sensitivity (True Positive)
1-Specificity (False Positive)
Outcome = CR is treated as positive.
AUC
0.63603
0.53767
0.53546

FIG. 4BB

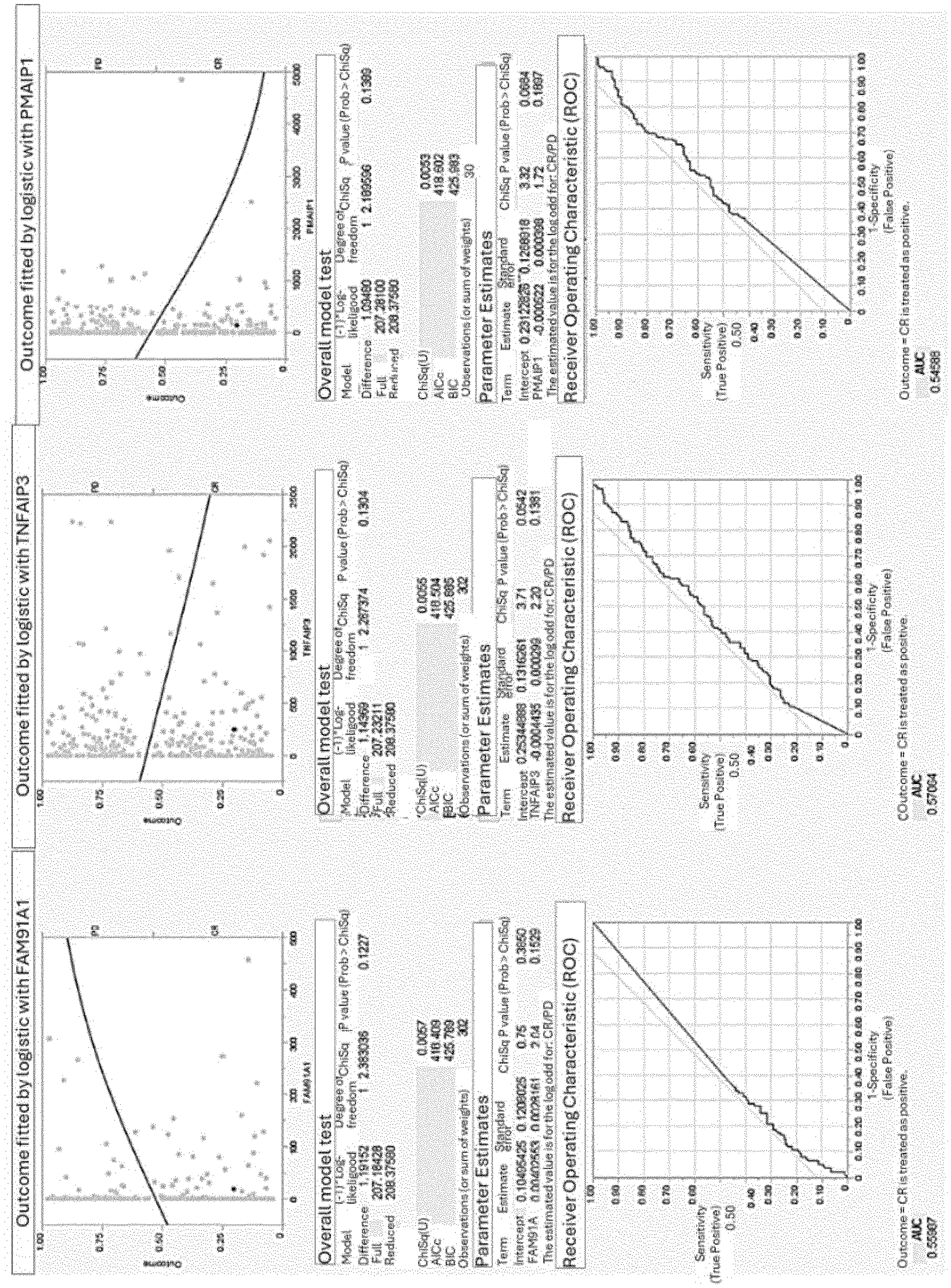
Outcome fitted by logistic with FAM91A1
Outcome fitted by logistic with TNFAIP3
Outcome fitted by logistic with PMAIP1
Overall model test
Parameter Estimates
Receiver Operating Characteristic (ROC)
Outcome = CR is treated as positive.
AUC
0.55597
0.57064
0.54588

FIG. 4CC

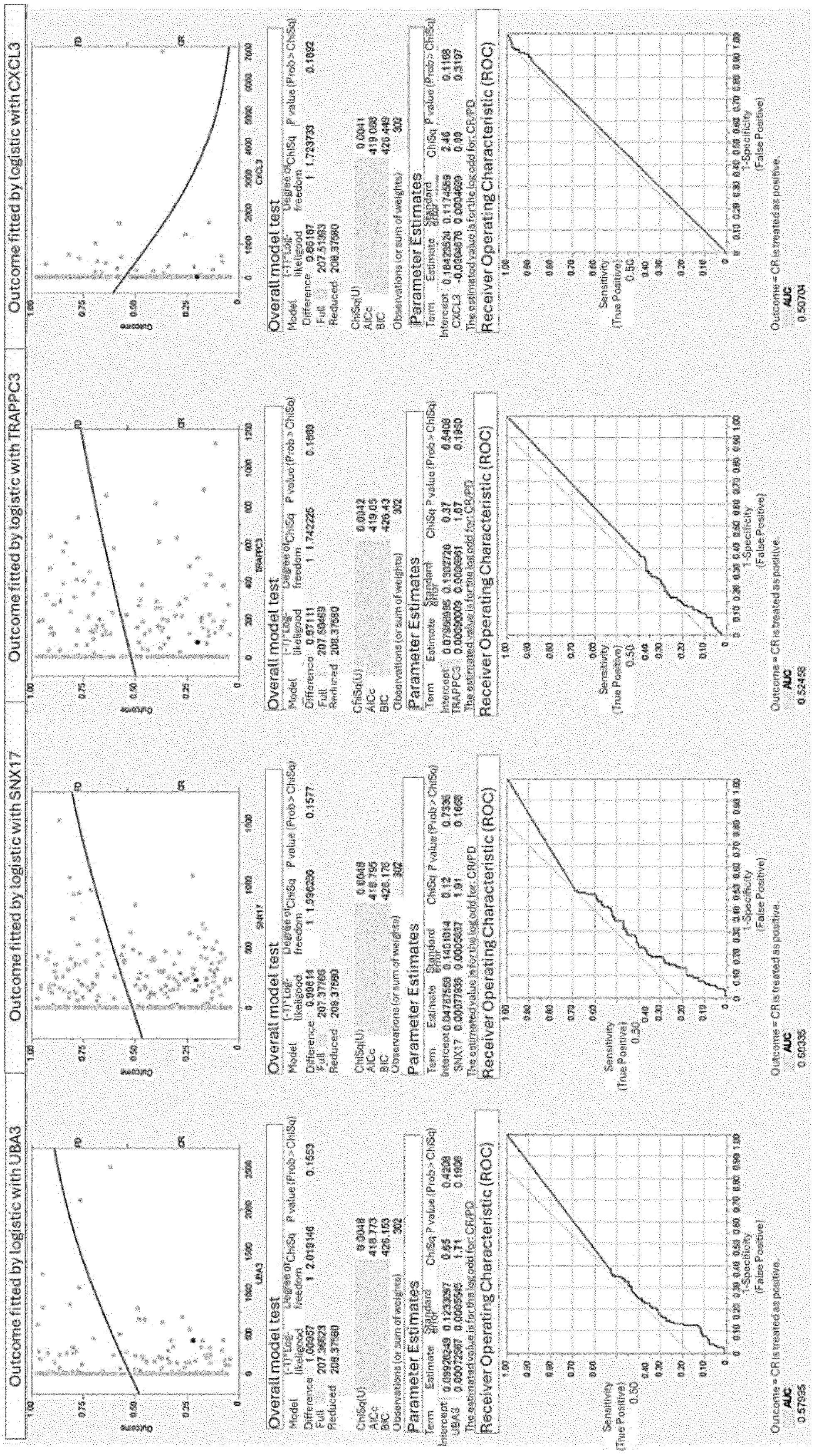
Outcome fitted by logistic with UBA3
Outcome fitted by logistic with SNX17
Outcome fitted by logistic with TRAPPC3
Outcome fitted by logistic with CXCL3
Overall model test
Parameter Estimates
Receiver Operating Characteristic (ROC)
The estimated value is for the log odd for: CR/PD
Outcome = CR is treated as positive.
AUC
0.57995
0.60335
0.52458
0.50704

FIG. 4DD

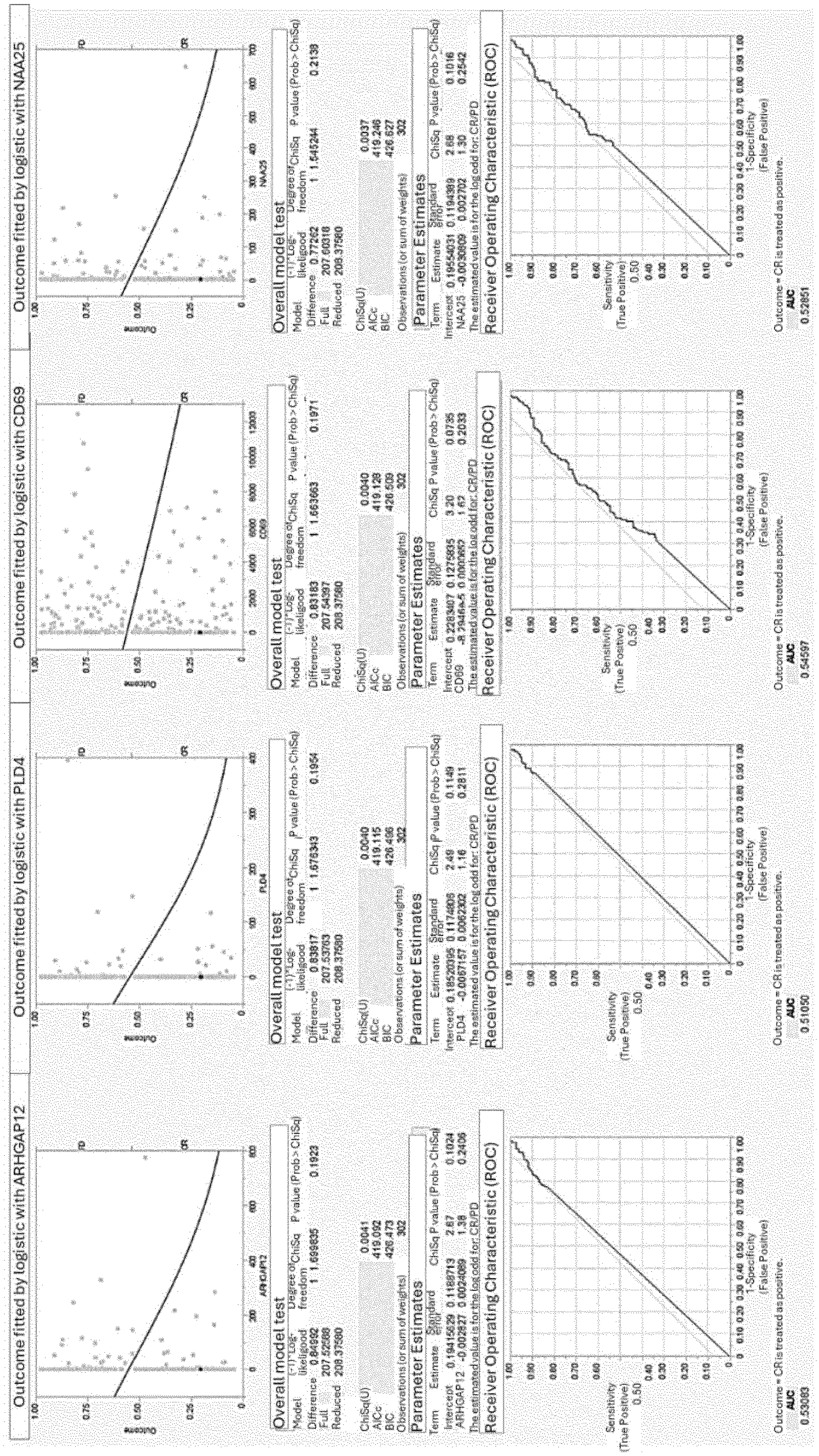

Outcome fitted by logistic with ARHGAP12
Overall model test
Difference 0.84992 1 1.699835 0.1923
Full 207.52588
Reduced 208.37580
ChiSq(U) 0.0041
AICc 419.092
BIC 426.473
Observations (or sum of weights) 302
Parameter Estimates
Intercept 0.19415529 0.1188713 2.67 0.1024
ARHGAP12 -0.002827 0.0024089 1.38 0.2406
The estimated value is for the log odd for: CR/PD
Receiver Operating Characteristic (ROC)
AUC 0.53083
Outcome fitted by logistic with PLD4
Overall model test
Difference 0.83817 1 1.676343 0.1954
Full 207.53763
Reduced 208.37580
ChiSq(U) 0.0040
AICc 419.115
BIC 426.496
Observations (or sum of weights) 302
Parameter Estimates
Intercept 0.18520395 0.1174806 2.49 0.1149
PLD4 -0.0067157 0.0062302 1.16 0.2811
The estimated value is for the log odd for: CR/PD
Receiver Operating Characteristic (ROC)
AUC 0.51050
Outcome fitted by logistic with CD69
Overall model test
Difference 0.83183 1 1.663663 0.1971
Full 207.54397
Reduced 208.37580
ChiSq(U) 0.0040
AICc 419.128
BIC 426.509
Observations (or sum of weights) 302
Parameter Estimates
Intercept 0.2283407 0.1275835 3.20 0.0735
CD69 -8.2946e-5 0.0000652 1.62 0.2033
The estimated value is for the log odd for: CR/PD
Receiver Operating Characteristic (ROC)
AUC 0.54597
Outcome fitted by logistic with NAA25
Overall model test
Difference 0.77262 1 1.545244 0.2139
Full 207.60318
Reduced 208.37580
ChiSq(U) 0.0037
AICc 419.246
BIC 426.627
Observations (or sum of weights) 302
Parameter Estimates
Intercept 0.19554031 0.1194389 2.68 0.1016
NAA25 -0.0030609 0.002702 1.30 0.2542
The estimated value is for the log odd for: CR/PD
Receiver Operating Characteristic (ROC)
AUC 0.52851
Outcome = CR is treated as positive.


FIG. 4EE

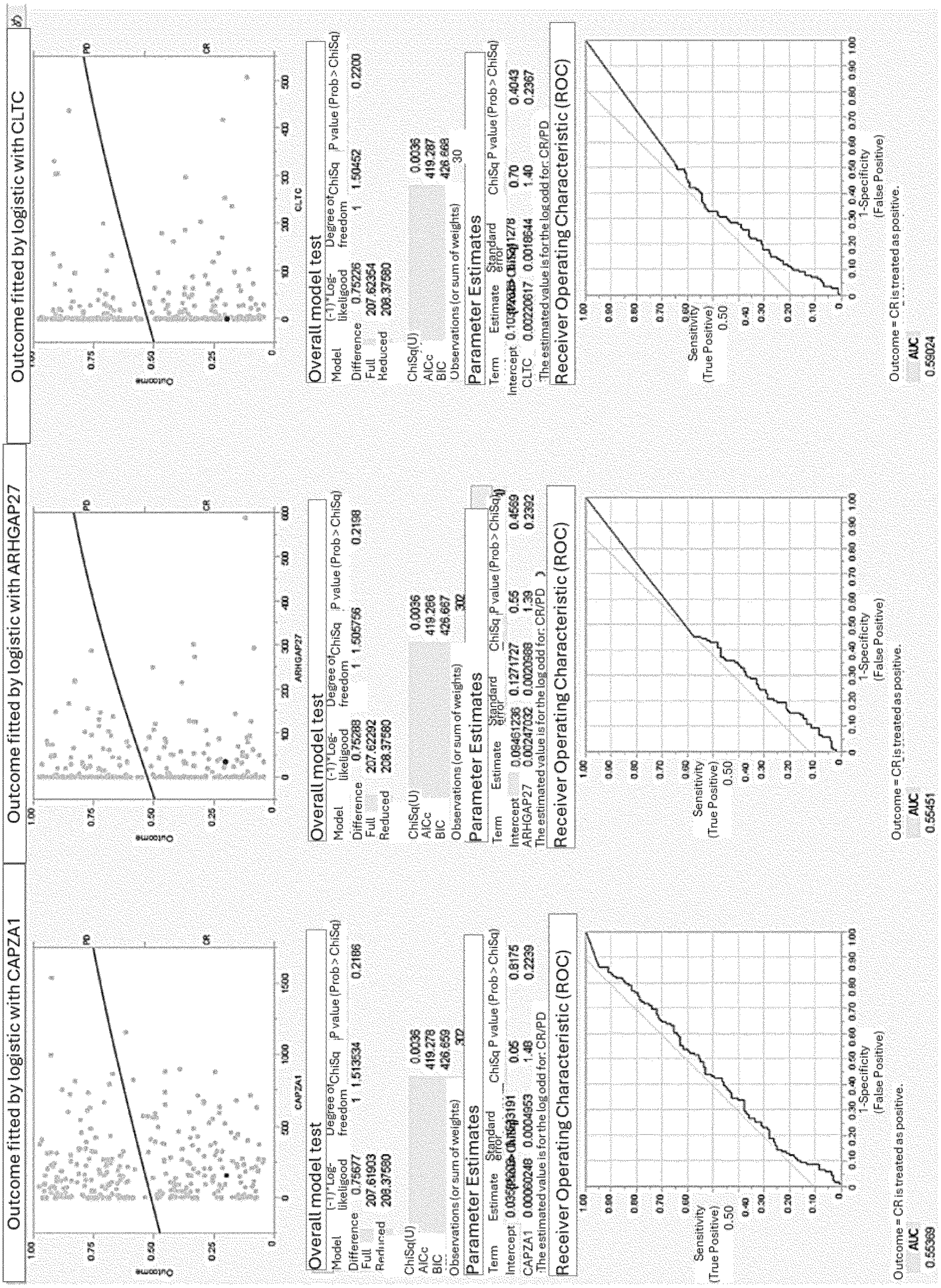
Outcome fitted by logistic with CAPZA1
Outcome fitted by logistic with ARHGAP27
Outcome fitted by logistic with CLTC
Overall model test
Parameter Estimates
Receiver Operating Characteristic (ROC)

FIG. 4FF

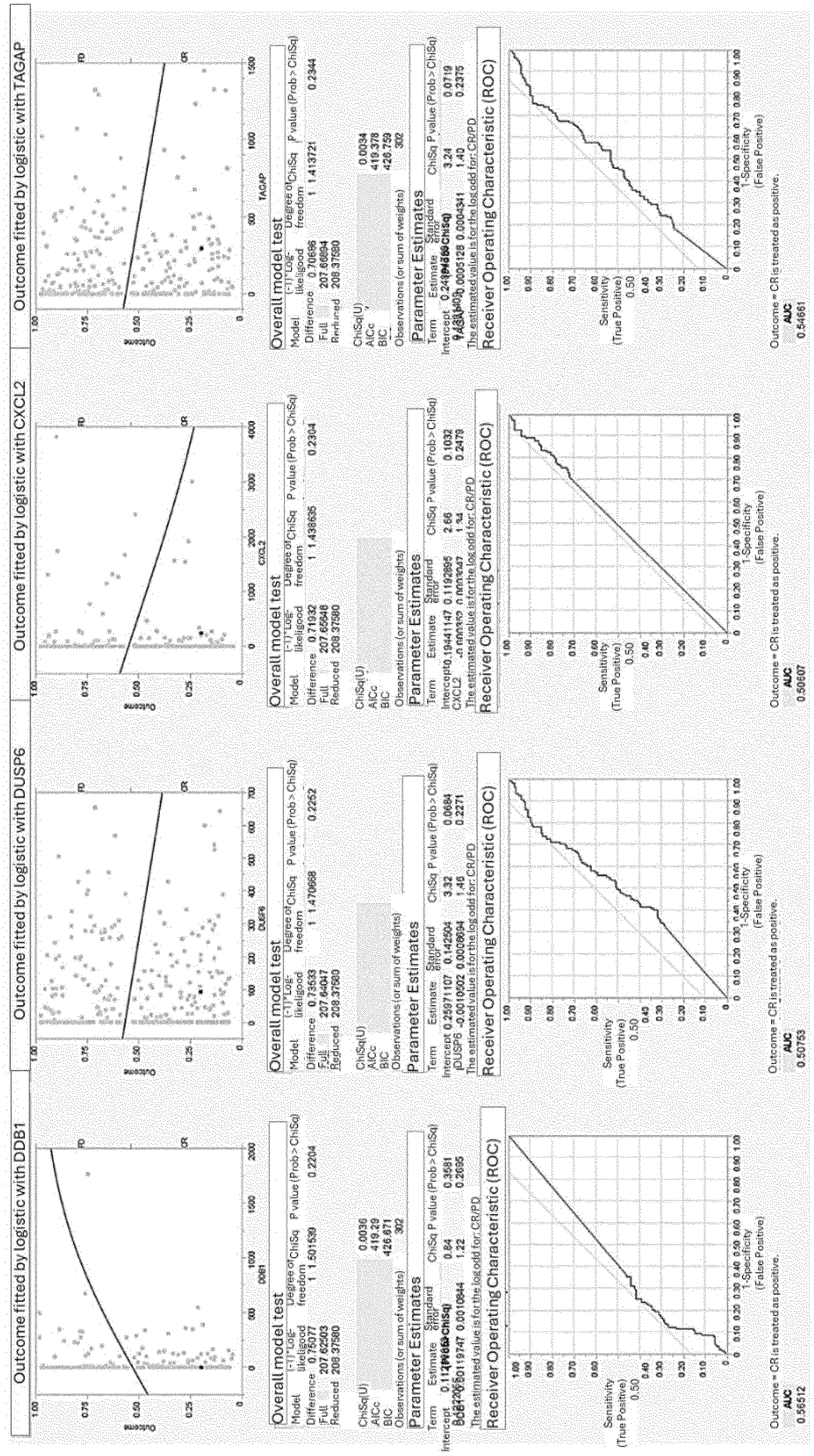
Outcome fitted by logistic with DDB1
Outcome fitted by logistic with DUSP6
Outcome fitted by logistic with CXCL2
Outcome fitted by logistic with TAGAP
Overall model test
Parameter Estimates
Receiver Operating Characteristic (ROC)
Outcome = CR is treated as positive.

FIG. 4GG

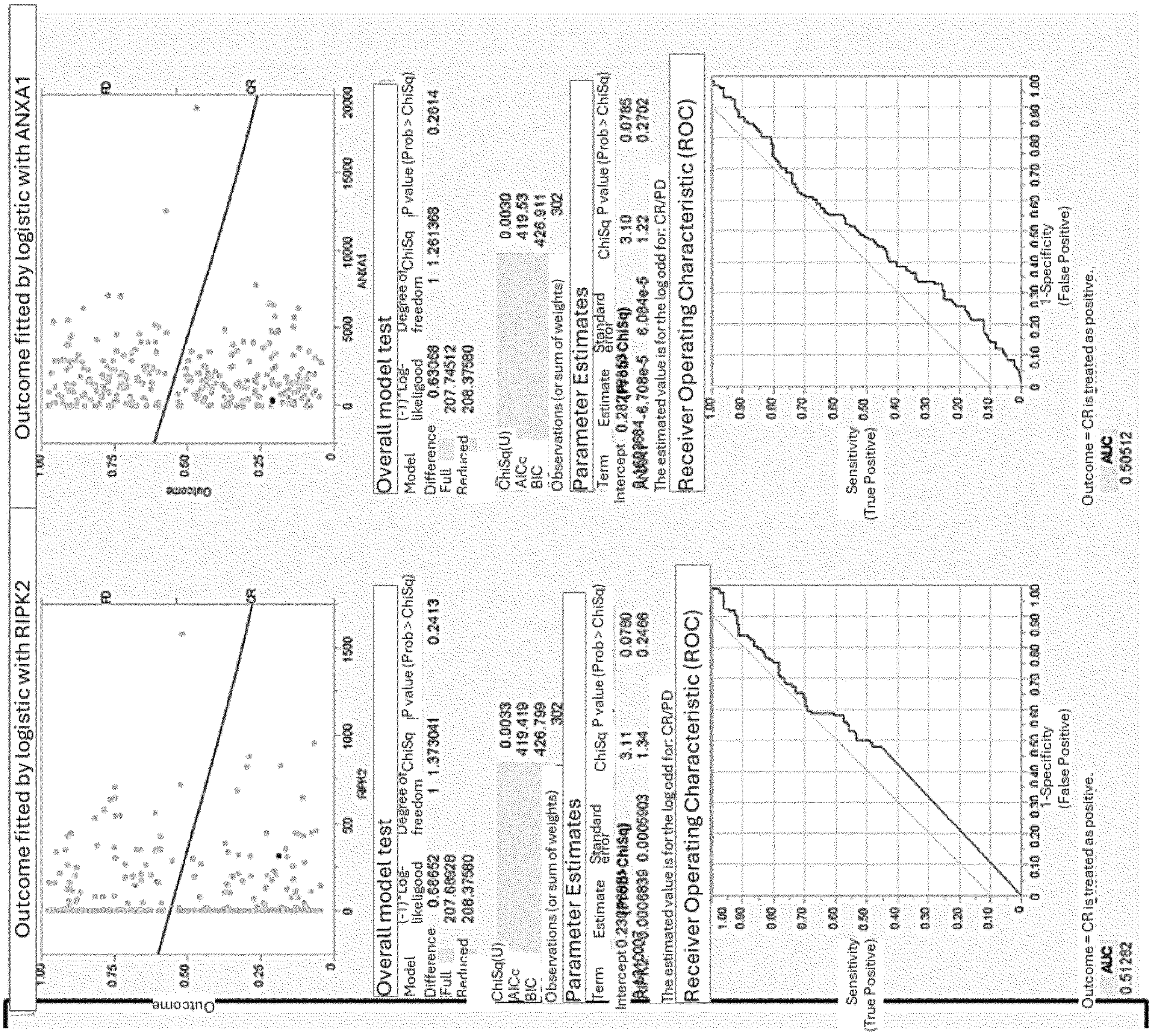
Outcome fitted by logistic with RIPK2
Outcome
RIPK2
Overall model test
Model (-1)*Log-likelihood Degree of freedom ChiSq P value (Prob > ChiSq)
Difference 0.68652 1 1.373041 0.2413
Full 207.68928
Reduced 208.37580
ChiSq(U) 0.0033
AICc 419.419
BIC 426.799
Observations (or sum of weights) 302
Parameter Estimates
Term Estimate Standard error ChiSq P value (Prob > ChiSq)
The estimated value is for the log odd for: CR/PD
Receiver Operating Characteristic (ROC)
Sensitivity (True Positive)
1-Specificity (False Positive)
Outcome = CR is treated as positive.
AUC
0.51282
Outcome fitted by logistic with ANXA1
Outcome
ANXA1
Overall model test
Model (-1)*Log-likelihood Degree of freedom ChiSq P value (Prob > ChiSq)
Difference 0.63068 1 1.261368 0.2614
Full 207.74512
Reduced 208.37580
ChiSq(U) 0.0030
AICc 419.53
BIC 426.911
Observations (or sum of weights) 302
Parameter Estimates
Term Estimate Standard error ChiSq P value (Prob > ChiSq)
The estimated value is for the log odd for: CR/PD
Receiver Operating Characteristic (ROC)
Sensitivity (True Positive)
1-Specificity (False Positive)
Outcome = CR is treated as positive.
AUC
0.50512

FIG. 4HH

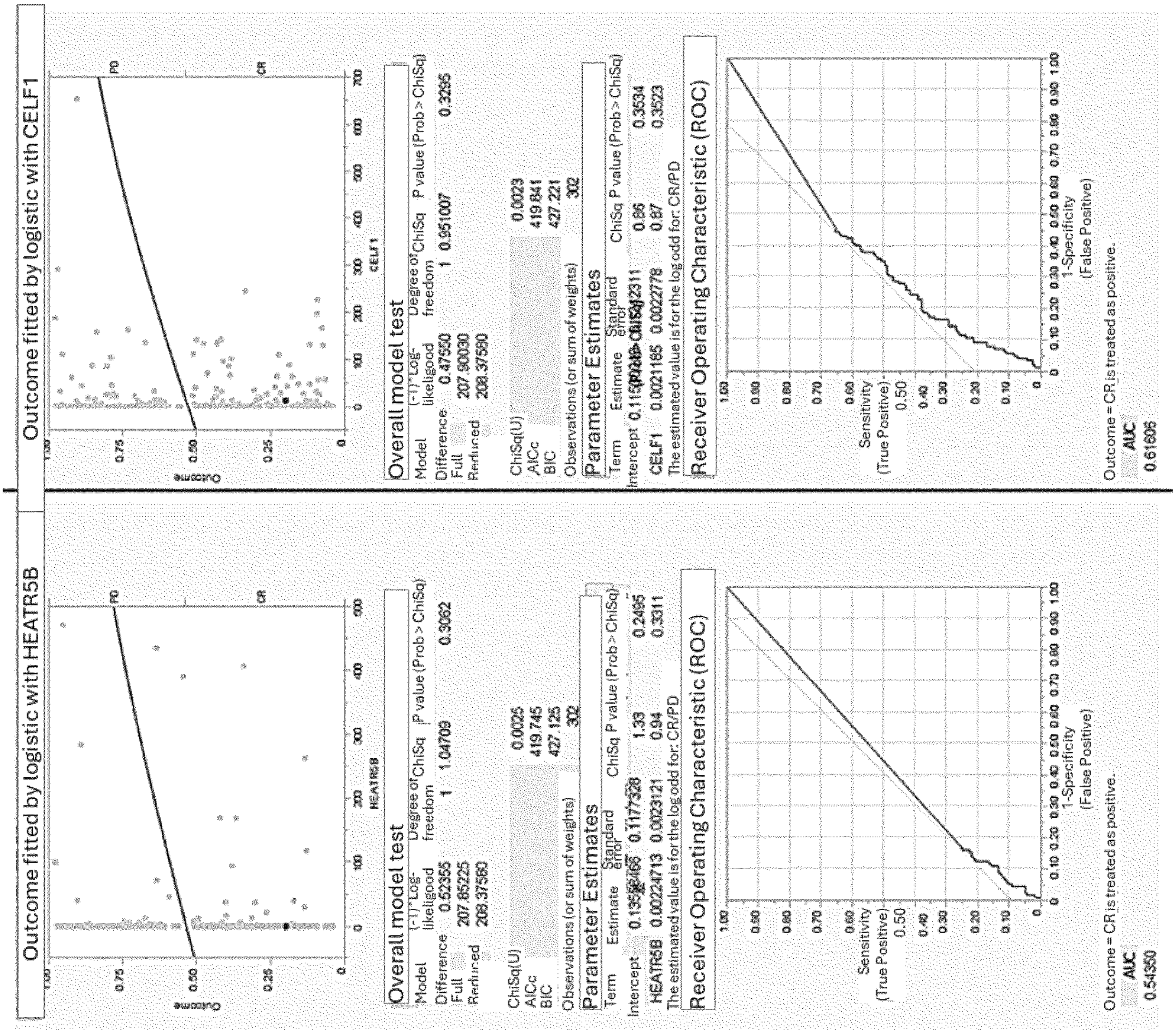
Outcome fitted by logistic with HEATR5B
Outcome
HEATR5B
PD
CR
Overall model test
Model (-1)*Log-likeligood Degree of freedom ChiSq P value (Prob > ChiSq)
Difference 0.52355 1 1.04709 0.3062
Full 207.85225
Reduced 208.37580
ChiSq(U) 0.0025
AICc 419.745
BIC 427.125
Observations (or sum of weights) 302
Parameter Estimates
Term Estimate Standard error ChiSq P value (Prob > ChiSq)
Intercept 0.13556466 0.1177328 1.33 0.2495
HEATR5B 0.00224713 0.0023121 0.94 0.3311
The estimated value is for the log odd for: CR/PD
Receiver Operating Characteristic (ROC)
Sensitivity (True Positive)
1-Specificity (False Positive)
Outcome = CR is treated as positive.
AUC
0.54350
Outcome fitted by logistic with CELF1
Outcome
CELF1
PD
CR
Overall model test
Model (-1)*Log-likeligood Degree of freedom ChiSq P value (Prob > ChiSq)
Difference 0.47550 1 0.951007 0.3295
Full 207.90030
Reduced 208.37580
ChiSq(U) 0.0023
AICc 419.841
BIC 427.221
Observations (or sum of weights) 302
Parameter Estimates
Term Estimate Standard error ChiSq P value (Prob > ChiSq)
Intercept 0.1158 0.1242311 0.86 0.3534
CELF1 0.0021185 0.0022778 0.87 0.3523
The estimated value is for the log odd for: CR/PD
Receiver Operating Characteristic (ROC)
Sensitivity (True Positive)
1-Specificity (False Positive)
Outcome = CR is treated as positive.
AUC
0.61606

FIG. 4II

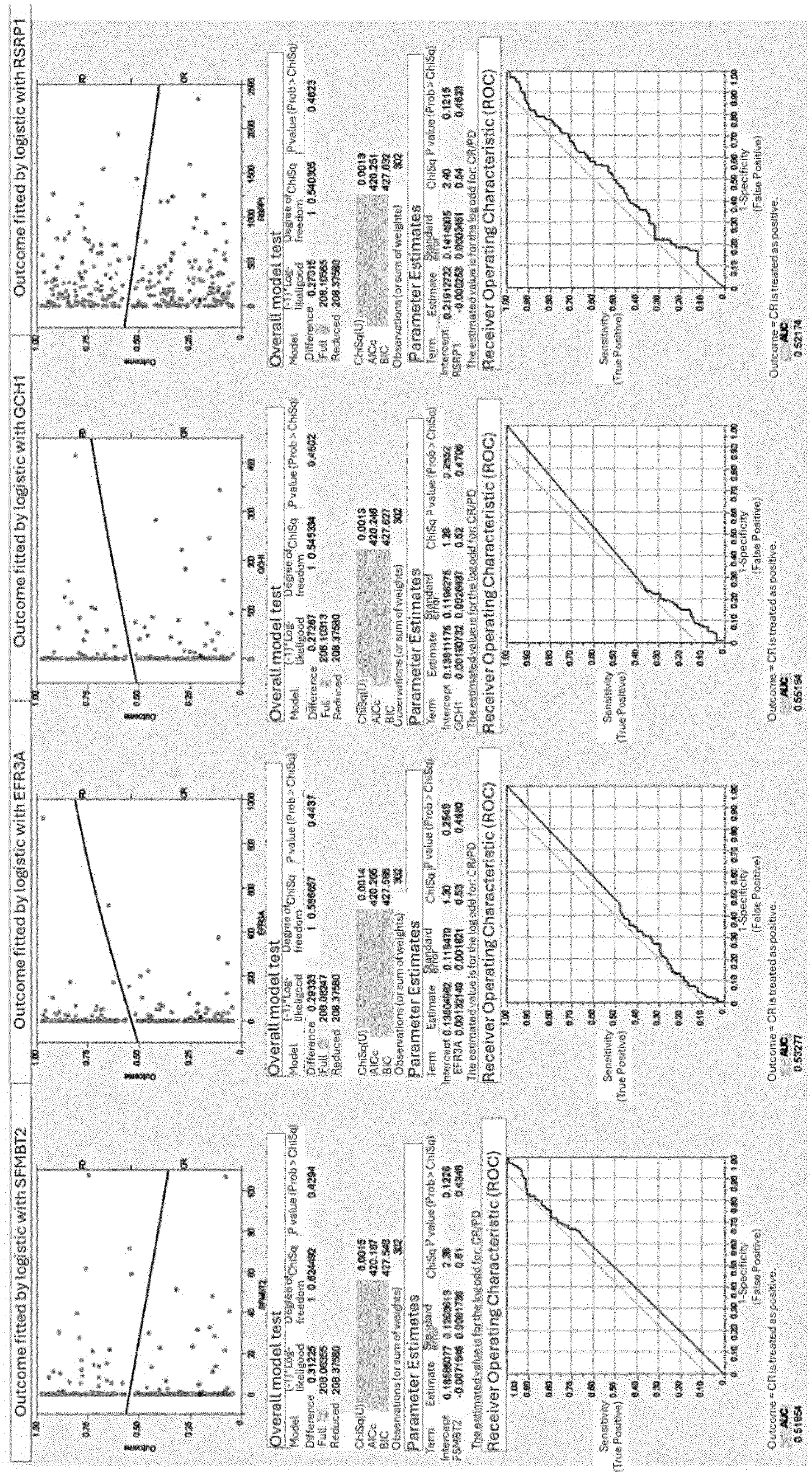
Outcome fitted by logistic with SFMBT2
Outcome fitted by logistic with EFR3A
Outcome fitted by logistic with GCH1
Outcome fitted by logistic with RSRP1
Overall model test
Parameter Estimates
Receiver Operating Characteristic (ROC)
Outcome = CR is treated as positive.
AUC
0.51654
0.53277
0.55164
0.52174

FIG. 4JJ

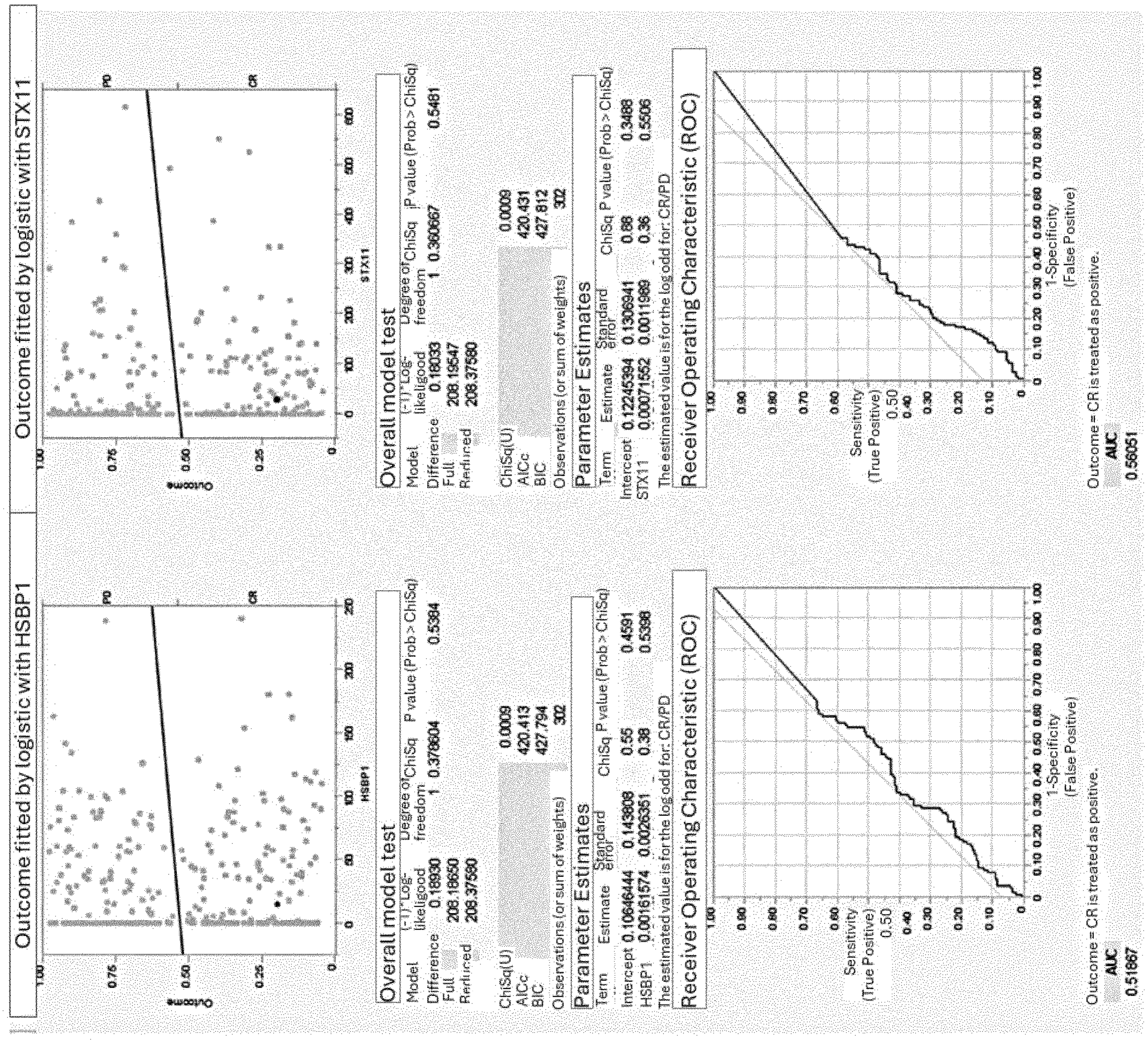
Outcome fitted by logistic with HSBP1
Overall model test
Model (-1)*Log-likelihood Degree of freedom ChiSq P value (Prob > ChiSq)
Difference 0.18930 1 0.378604 0.5384
Full 208.18650
Reduced 208.37580
ChiSq(U) 0.0009
AICc 420.413
BIC 427.794
Observations (or sum of weights) 302
Parameter Estimates
Term Estimate Standard error ChiSq P value (Prob > ChiSq)
Intercept 0.10646444 0.143808 0.55 0.4591
HSBP1 0.00161574 0.0026351 0.38 0.5398
The estimated value is for the log odd for: CR/PD
Receiver Operating Characteristic (ROC)
Sensitivity (True Positive)
1-Specificity (False Positive)
Outcome = CR is treated as positive.
AUC
0.51867
Outcome fitted by logistic with STX11
Overall model test
Model (-1)*Log-likelihood Degree of freedom ChiSq P value (Prob > ChiSq)
Difference 0.18033 1 0.360667 0.5481
Full 208.19547
Reduced 208.37580
ChiSq(U) 0.0009
AICc 420.431
BIC 427.812
Observations (or sum of weights) 302
Parameter Estimates
Term Estimate Standard error ChiSq P value (Prob > ChiSq)
Intercept 0.12245394 0.1306941 0.88 0.3488
STX11 0.00071552 0.0011989 0.36 0.5506
The estimated value is for the log odd for: CR/PD
Receiver Operating Characteristic (ROC)
Sensitivity (True Positive)
1-Specificity (False Positive)
Outcome = CR is treated as positive.
AUC
0.56051

FIG. 4KK

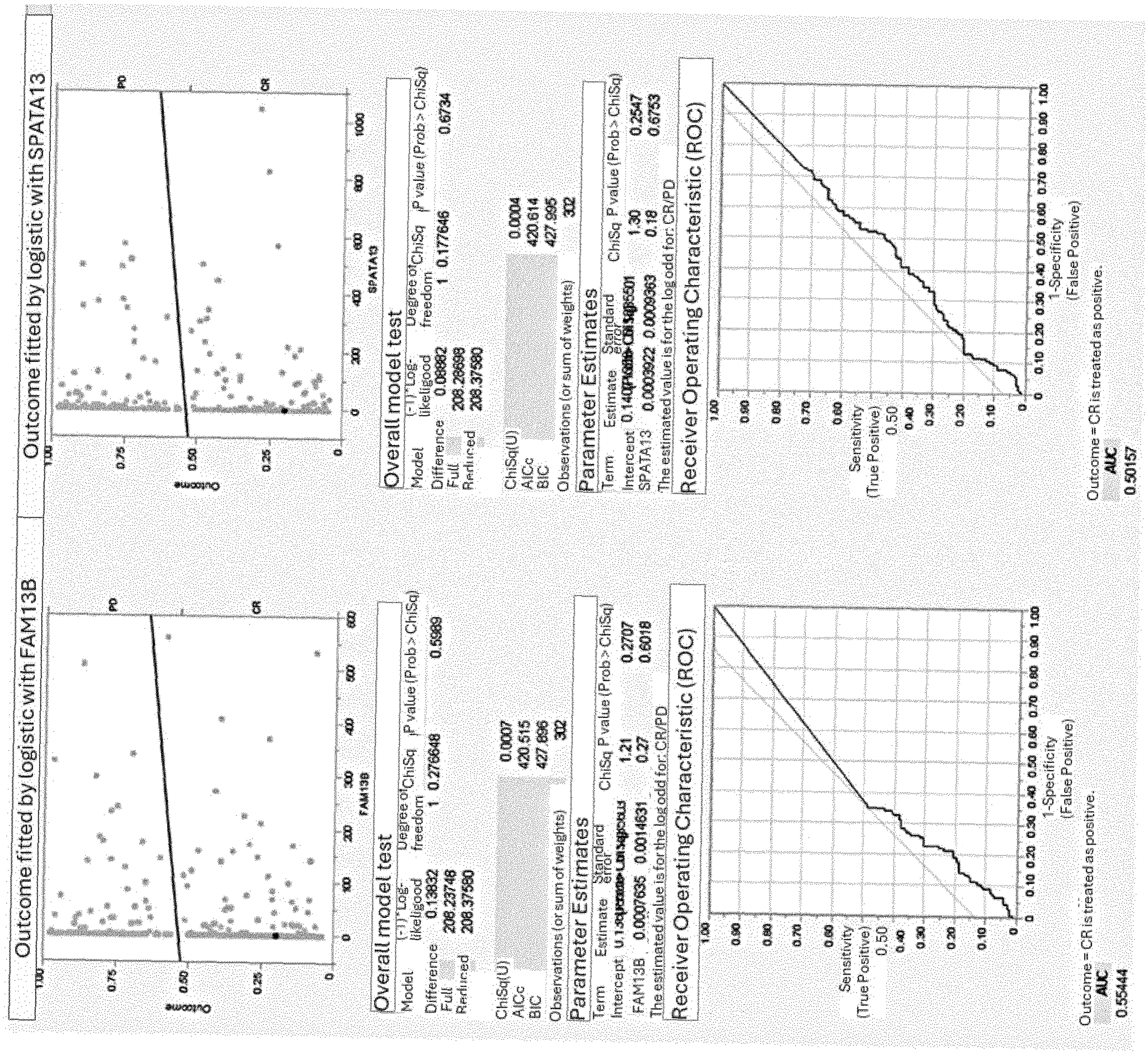
Outcome fitted by logistic with FAM13B
Overall model test
Model (-1)*Log-likeligood Degree of freedom ChiSq P value (Prob > ChiSq)
Difference 0.13832 1 0.276648 0.5989
Full 208.23748
Reduced 208.37580
ChiSq(U) 0.0007
AICc 420.515
BIC 427.896
Observations (or sum of weights) 302
Parameter Estimates
Term Estimate Standard error ChiSq P value (Prob > ChiSq)
FAM13B 0.0007635 0.0014631 0.27 0.6018
The estimated value is for the log odd for: CR/PD
Receiver Operating Characteristic (ROC)
Sensitivity (True Positive)
1-Specificity (False Positive)
Outcome = CR is treated as positive.
AUC
0.55444
Outcome fitted by logistic with SPATA13
Overall model test
Model (-1)*Log-likeligood Degree of freedom ChiSq P value (Prob > ChiSq)
Difference 0.08882 1 0.177646 0.6734
Full 208.28698
Reduced 208.37580
ChiSq(U) 0.0004
AICc 420.614
BIC 427.995
Observations (or sum of weights) 302
Parameter Estimates
Term Estimate Standard error ChiSq P value (Prob > ChiSq)
Intercept 1.30 0.2547
SPATA13 0.0003922 0.0009363 0.18 0.6753
The estimated value is for the log odd for: CR/PD
Receiver Operating Characteristic (ROC)
Sensitivity (True Positive)
1-Specificity (False Positive)
Outcome = CR is treated as positive.
AUC
0.50157

FIG. 4LL

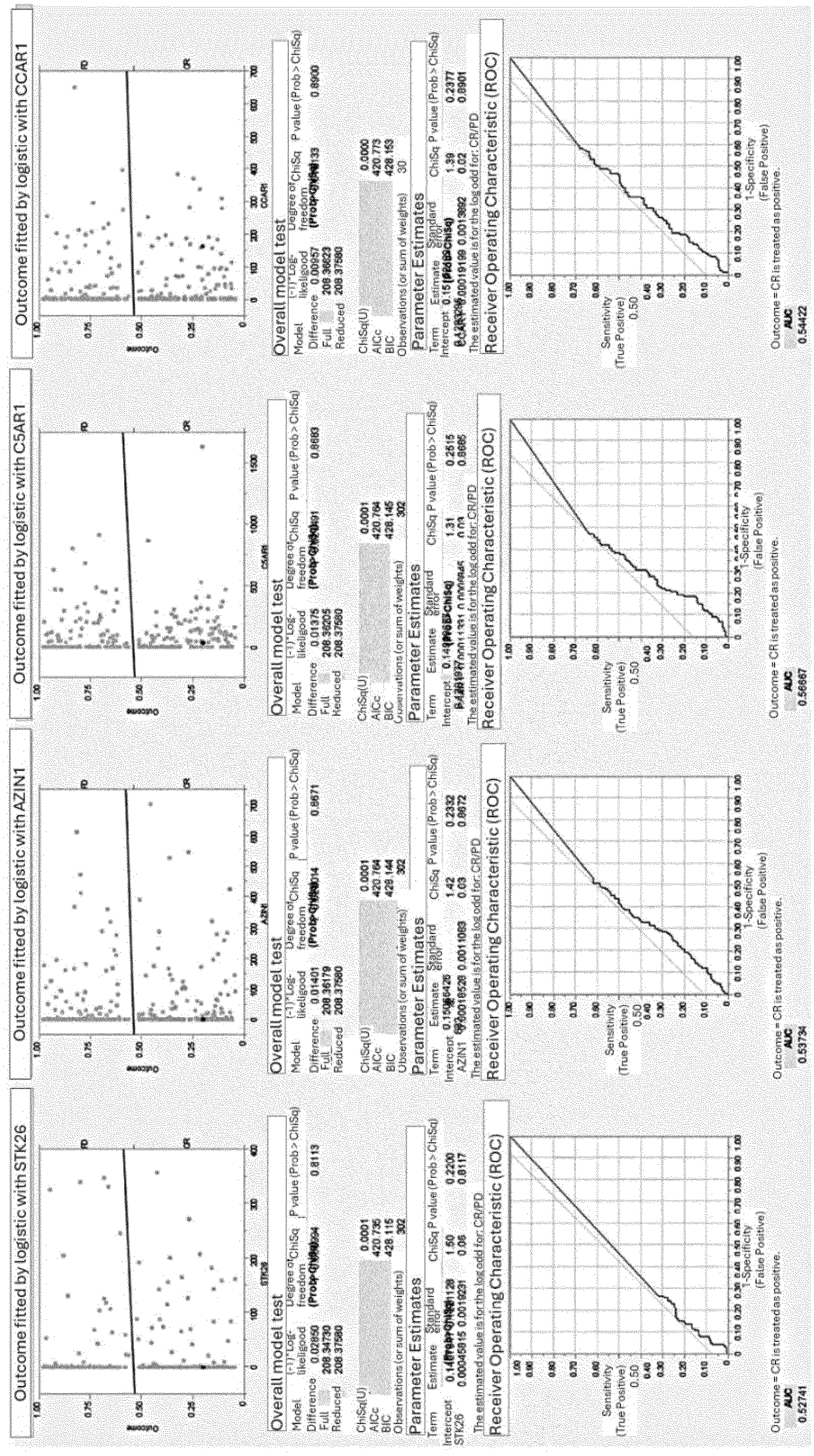
Outcome fitted by logistic with STK26
Outcome fitted by logistic with AZIN1
Outcome fitted by logistic with C5AR1
Outcome fitted by logistic with CCAR1
Overall model test
Parameter Estimates
Receiver Operating Characteristic (ROC)

FIG. 4MM

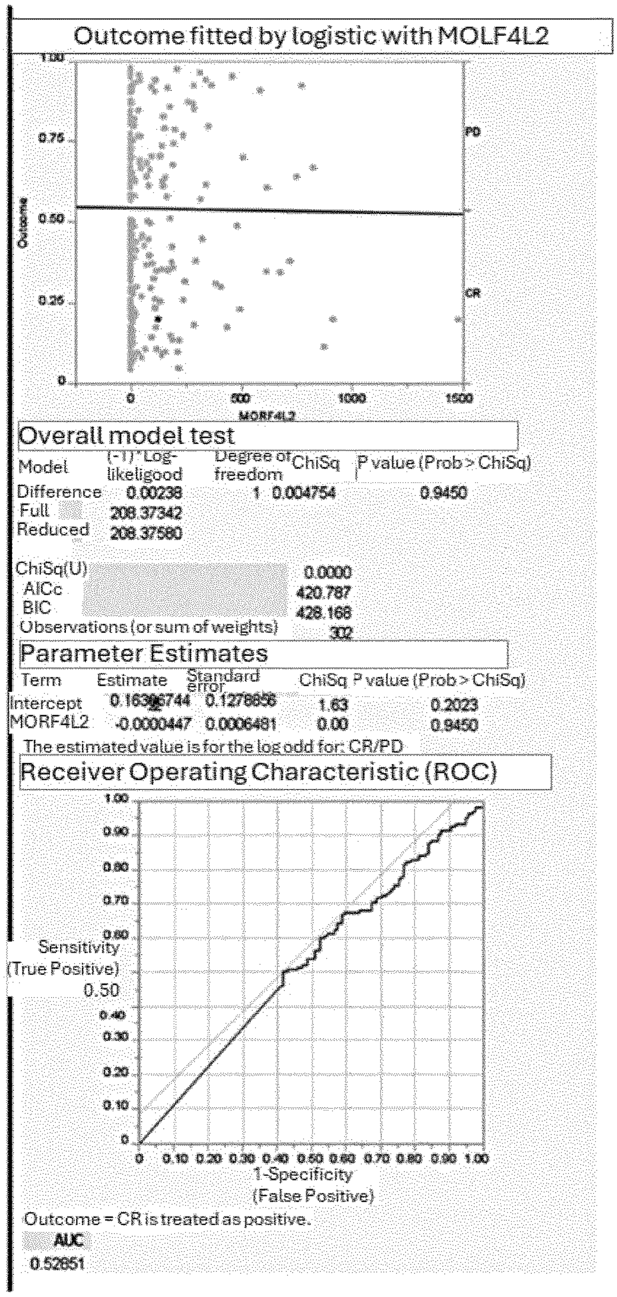
Outcome fitted by logistic with MOLF4L2
1.00
0.75
0.50
0.25
0
Outcome
PD
CR
0
500
1000
1500
MORF4L2
Overall model test
Model
(-1)*Log-likeligood
Degree of freedom
ChiSq
P value (Prob > ChiSq)
Difference 0.00238 1 0.004754 0.9450
Full 208.37342
Reduced 208.37580
ChiSq(U) 0.0000
AICc 420.787
BIC 428.168
Observations (or sum of weights) 302
Parameter Estimates
Term
Estimate
Standard error
ChiSq
P value (Prob > ChiSq)
Intercept 0.16306744 0.1278656 1.63 0.2023
MORF4L2 -0.0000447 0.0006481 0.00 0.9450
The estimated value is for the log odd for: CR/PD
Receiver Operating Characteristic (ROC)
1.00
0.90
0.80
0.70
0.60
Sensitivity
(True Positive)
0.50
0.40
0.30
0.20
0.10
0
0 0.10 0.20 0.30 0.40 0.50 0.60 0.70 0.80 0.90 1.00
1-Specificity
(False Positive)
Outcome = CR is treated as positive.
AUC
0.52651

FIG. 4NN

## Logistic regression analysis

**Estimated parameter**

| Term | Estimate | Standard error | ChiSq | P value (Prob > ChiSq) |
|---|---|---|---|---|
| Intercept | 0.14279648 | 0.1567033 | 0.83 | 0.3622 |
| TMEM176A | 0.00448154 | 0.0018934 | 5.60 | 0.0179* |
| TMEM176B | 0.00112643 | 0.000467 | 5.82 | 0.0159* |
| CLEC12A | -0.003981 | 0.0006911 | 33.19 | <.0001* |

The estimated value is for the log odd for: PD/CR

**Receiver Operating Characteristic (ROC)**

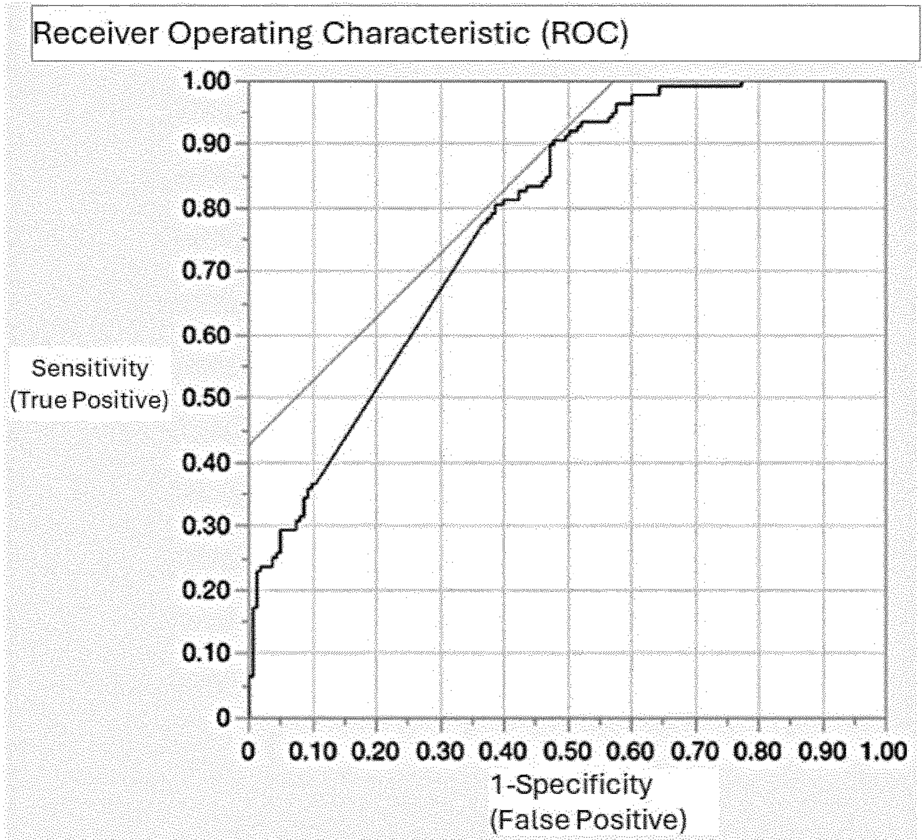


Outcome = PD is treated as positive.

| AUC |
|---|
| 0.78133 |

| | |
|---|---|
| ChiSq(U) | 0.2345 |
| AICc | 327.156 |
| BIC | 341.863 |
| Observations (or sum of weights) | 302 |

FIG. 5

## Logistic regression analysis

### Estimated parameter

| Term | Estimate | Standard error | ChiSq | P value (Prob > ChiSq) |
|---|---|---|---|---|
| Intercept | 0.62653668 | 0.2824323 | 4.92 | 0.0265* |
| TMEM176A | -0.0106474 | 0.0020982 | 25.75 | <.0001* |
| CLEC12A | 0.00233919 | 0.0007361 | 10.10 | 0.0015* |
| S100B | -0.008976 | 0.0065092 | 1.90 | 0.1679 |
| CD52 | 0.00028065 | 0.0001039 | 7.30 | 0.0069* |
| SH2D2A | -0.0021032 | 0.0008047 | 6.83 | 0.0090* |
| SIDT2 | 0.01913039 | 0.007094 | 7.27 | 0.0070* |
| GTF3C1 | -0.0063053 | 0.0026089 | 5.84 | 0.0157* |
| ZNF266 | -0.0199105 | 0.007164 | 7.72 | 0.0054* |
| MS4A7 | 9.11986e-5 | 0.0006984 | 0.02 | 0.8961 |
| LST1 | -0.0002628 | 0.0001412 | 3.46 | 0.0627 |

The estimated value is for the log odd for: CR/PD

### Receiver Operating Characteristic (ROC)

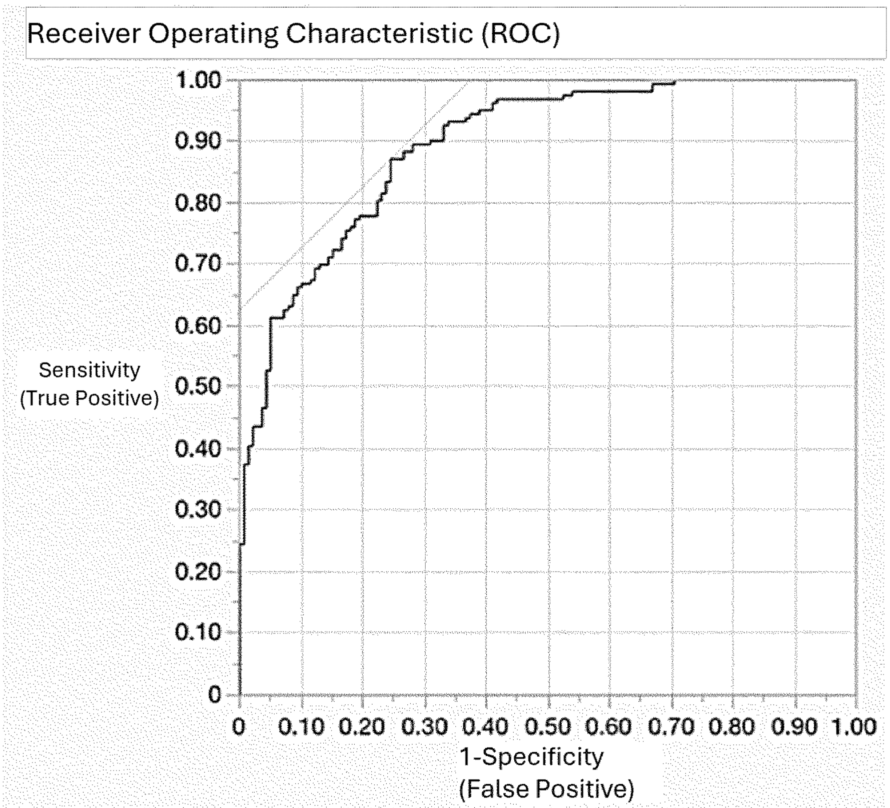


Outcome = CR is treated as positive.

| AUC |
|---|
| 0.89398 |

| | |
|---|---|
| ChiSq(U) | 0.4212 |
| AICc | 264.143 |
| BIC | 304.047 |
| Observations (or sum of weights) | 302 |

FIG. 6

## Logistic regression analysis

### Estimated parameter

| Term | Estimate | Standard error | ChiSq | P value (Prob > ChiSq) |
|---|---|---|---|---|
| Intercept | -0.1985317 | 0.4030901 | 0.24 | 0.6223 |
| TMEM176A | 0.00746354 | 0.0021054 | 12.57 | 0.0004* |
| CLEC12A | -0.0034408 | 0.0009072 | 14.38 | 0.0001* |
| S100B | 0.00967981 | 0.0079798 | 1.47 | 0.2251 |
| CD52 | -0.000261 | 0.0001167 | 5.00 | 0.0253* |
| SH2D2A | 0.00241373 | 0.001279 | 3.56 | 0.0591 |
| SIDT2 | -0.0177782 | 0.007751 | 5.26 | 0.0218* |
| GTF3C1 | 0.00930914 | 0.0041081 | 5.13 | 0.0234* |
| ZNF266 | 0.02252576 | 0.009086 | 6.15 | 0.0132* |
| MS4A7 | 0.00023139 | 0.0008287 | 0.08 | 0.7801 |
| LST1 | 0.00014132 | 0.0002147 | 0.43 | 0.5104 |
| SH2B3 | -0.0133484 | 0.0063819 | 4.37 | 0.0365* |
| AIF1 | 1.56362e-5 | 0.0001495 | 0.01 | 0.9167 |
| LAIR2 | -0.0025397 | 0.0007176 | 12.52 | 0.0004* |
| RETN | 0.00096442 | 0.00063 | 2.34 | 0.1258 |
| LYPD2 | -0.0004236 | 0.0003268 | 1.68 | 0.1949 |
| RNF14 | -0.0128677 | 0.0064754 | 3.95 | 0.0469* |
| UBE2F | 0.0016002 | 0.0011275 | 2.01 | 0.1558 |
| CTSS | 0.00055923 | 0.0004101 | 1.86 | 0.1726 |
| FGL2 | -0.0017609 | 0.0029493 | 0.36 | 0.5505 |
| HAVCR2 | 0.00332874 | 0.001746 | 3.63 | 0.0566 |

The estimated value is for the log odd for: PD/CR

### Receiver Operating Characteristic (ROC)

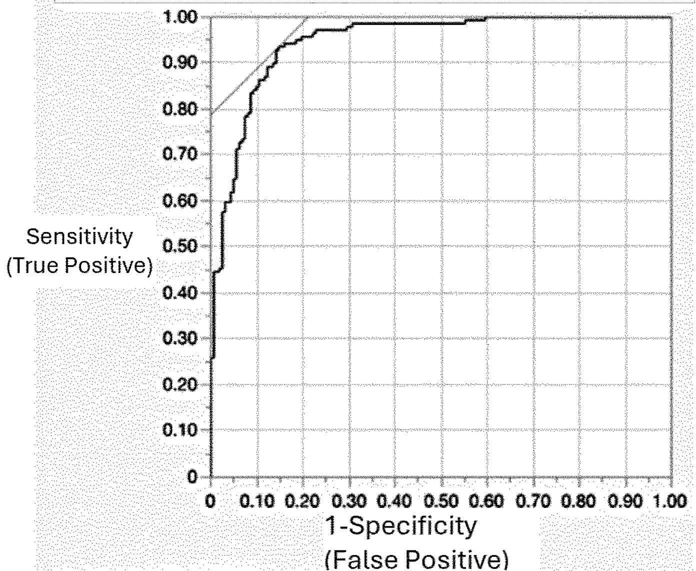


Outcome = CR is treated as positive.

| AUC |
|---|
| 0.94765 |

| | |
|---|---|
| ChiSq(U) | 0.5761 |
| AICc | 221.969 |
| BIC | 296.588 |
| Observations (or sum of weights) | 302 |

FIG. 7

## Logistic regression analysis

## 160 genes

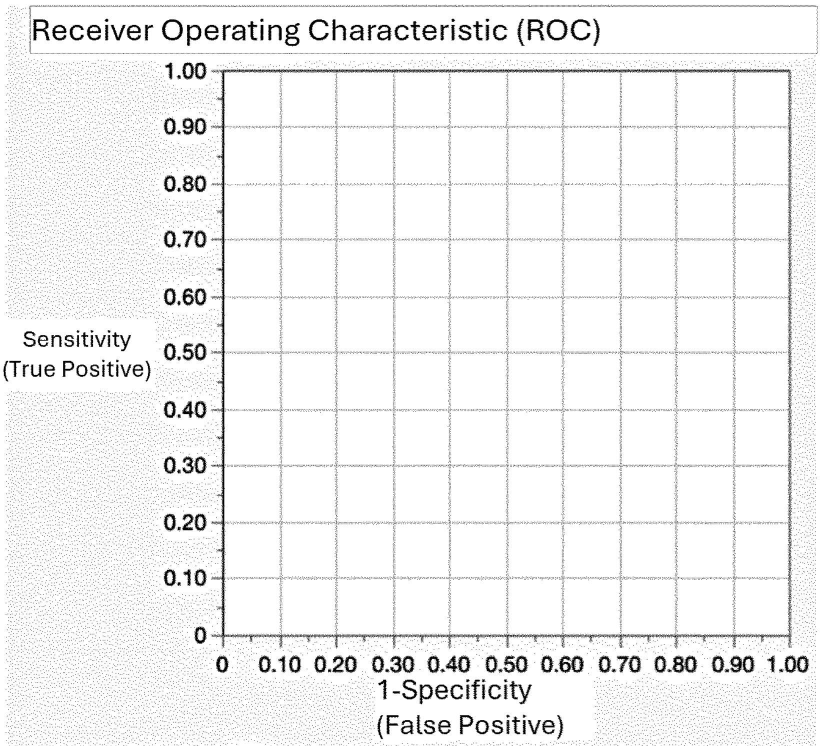


Outcome = CR is treated as positive.

| AUC |
|---|
| 1.00000 |

Training

| Actual value | Predicted value | Frequency |
|---|---|---|
| **Outcome** | **CR** | **PD** |
| CR | 163 | 0 |
| PD | 0 | 139 |

| Actual value | Predicted value | Ratio |
|---|---|---|
| **Outcome** | **CR** | **PD** |
| CR | 1.000 | 0.000 |
| PD | 0.000 | 1.000 |

| | |
|---|---|
| ChiSq(U) | 1.0000 |
| AICc | 694.6 |
| BIC | 919.379 |
| Observations (or sum of weights) | 302 |

FIG. 8

## Decision tree analysis

| Leaf label | CR | PD |
|---|---|---|
| CLEC12A<473.079041&LAIR2<1094.115234&HAVCR2>=5.301657&GTF3C1>=1.474105 | 0.0153 | 0.9847 |
| CLEC12A<473.079041&LAIR2<1094.115234&HAVCR2>=5.301657&GTF3C1<1.474105&STAB1>=1.124403 | 0.0280 | 0.9720 |
| CLEC12A<473.079041&LAIR2<1094.115234&HAVCR2>=5.301657&GTF3C1<1.474105&STAB1<1.124403&RSRP1>=161.716125 | 0.1107 | 0.8893 |
| CLEC12A<473.079041&LAIR2<1094.115234&HAVCR2>=5.301657&GTF3C1<1.474105&STAB1<1.124403&RSRP1<161.716125 | 0.6199 | 0.3801 |
| CLEC12A<473.079041&LAIR2<1094.115234&HAVCR2<5.301657&AIF1<7748.612305&CAPN7<0.278515&TMEM176B>=537.829712 | 0.0255 | 0.9745 |
| CLEC12A<473.079041&LAIR2<1094.115234&HAVCR2<5.301657&AIF1<7748.612305&CAPN7<0.278515&TMEM176B<537.829712&CELF1<0.3489&DIP2A>=0.414243 | 0.0178 | 0.9822 |
| CLEC12A<473.079041&LAIR2<1094.115234&HAVCR2<5.301657&AIF1<7748.612305&CAPN7<0.278515&TMEM176B<537.829712&CELF1<0.3489&DIP2A<0.414243 | 0.4604 | 0.5396 |
| CLEC12A<473.079041&LAIR2<1094.115234&HAVCR2<5.301657&AIF1<7748.612305&CAPN7<0.278515&TMEM176B<537.829712&CELF1>=0.3489&FAM91A1<0.157429 | 0.3746 | 0.6254 |
| CLEC12A<473.079041&LAIR2<1094.115234&HAVCR2<5.301657&AIF1<7748.612305&CAPN7<0.278515&TMEM176B<537.829712&CELF1>=0.3489&FAM91A1>=0.157429 | 0.8746 | 0.1254 |
| CLEC12A<473.079041&LAIR2<1094.115234&HAVCR2<5.301657&AIF1<7748.612305&CAPN7>=0.278515&RSRP1>=74.22364 | 0.3946 | 0.6054 |
| CLEC12A<473.079041&LAIR2<1094.115234&HAVCR2<5.301657&AIF1<7748.612305&CAPN7>=0.278515&RSRP1<74.22364&TMEM176B>=0.245295 | 0.7784 | 0.2216 |
| CLEC12A<473.079041&LAIR2<1094.115234&HAVCR2<5.301657&AIF1<7748.612305&CAPN7>=0.278515&RSRP1<74.22364&TMEM176B<0.245295 | 0.9708 | 0.0292 |
| CLEC12A<473.079041&LAIR2<1094.115234&HAVCR2<5.301657&AIF1>=7748.612305 | 0.9623 | 0.0377 |
| CLEC12A<473.079041&LAIR2>=1094.115234&FAM13B>=0.056771 | 0.8700 | 0.1300 |
| CLEC12A<473.079041&LAIR2>=1094.115234&FAM13B<0.056771 | 0.9731 | 0.0269 |
| CLEC12A>=473.079041&ADM>=87.210175 | 0.6127 | 0.3873 |
| CLEC12A>=473.079041&ADM<87.210175 | 0.9923 | 0.0077 |

| Metric | Training | Definition |
|---|---|---|
| Entropy R-squared | 0.6749 | 1-Loglike(model)/Loglike(0) |
| Generalized R-squared | 0.8097 | (1-(L(0)/L(model))^(2/n))/(1-L(0)^(2/n)) |
| Mean-logP | 0.2243 | ∑ -Log(ρ[j])/n |
| RASE | 0.2679 | √ ∑(y[j]-ρ[j])²/n |
| Mean absolute deviation | 0.1577 | ∑ \|y[j]-ρ[j]\|/n |
| Misclassification rate | 0.1093 | ∑ (ρ[j]≠ρMax)/n |
| N | 302 | n |

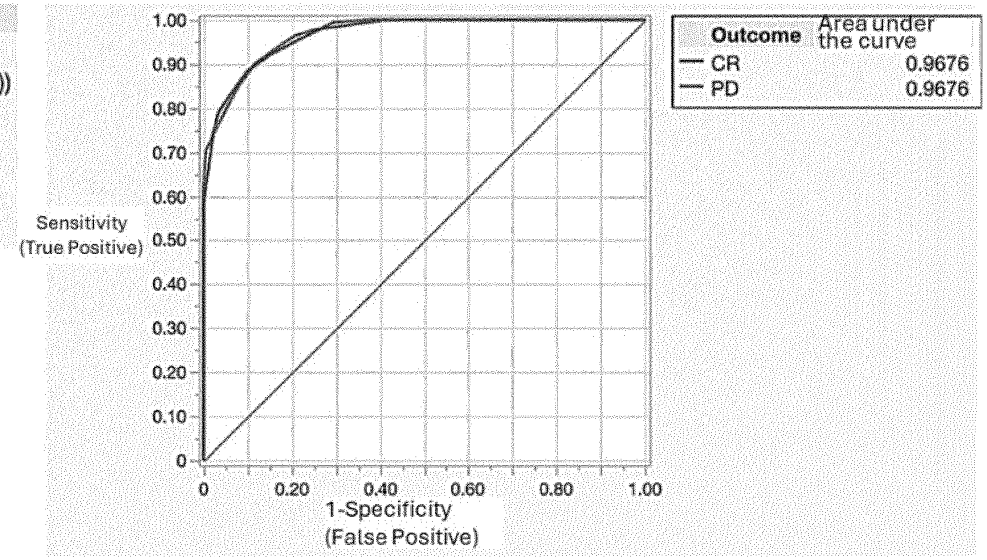


FIG. 9

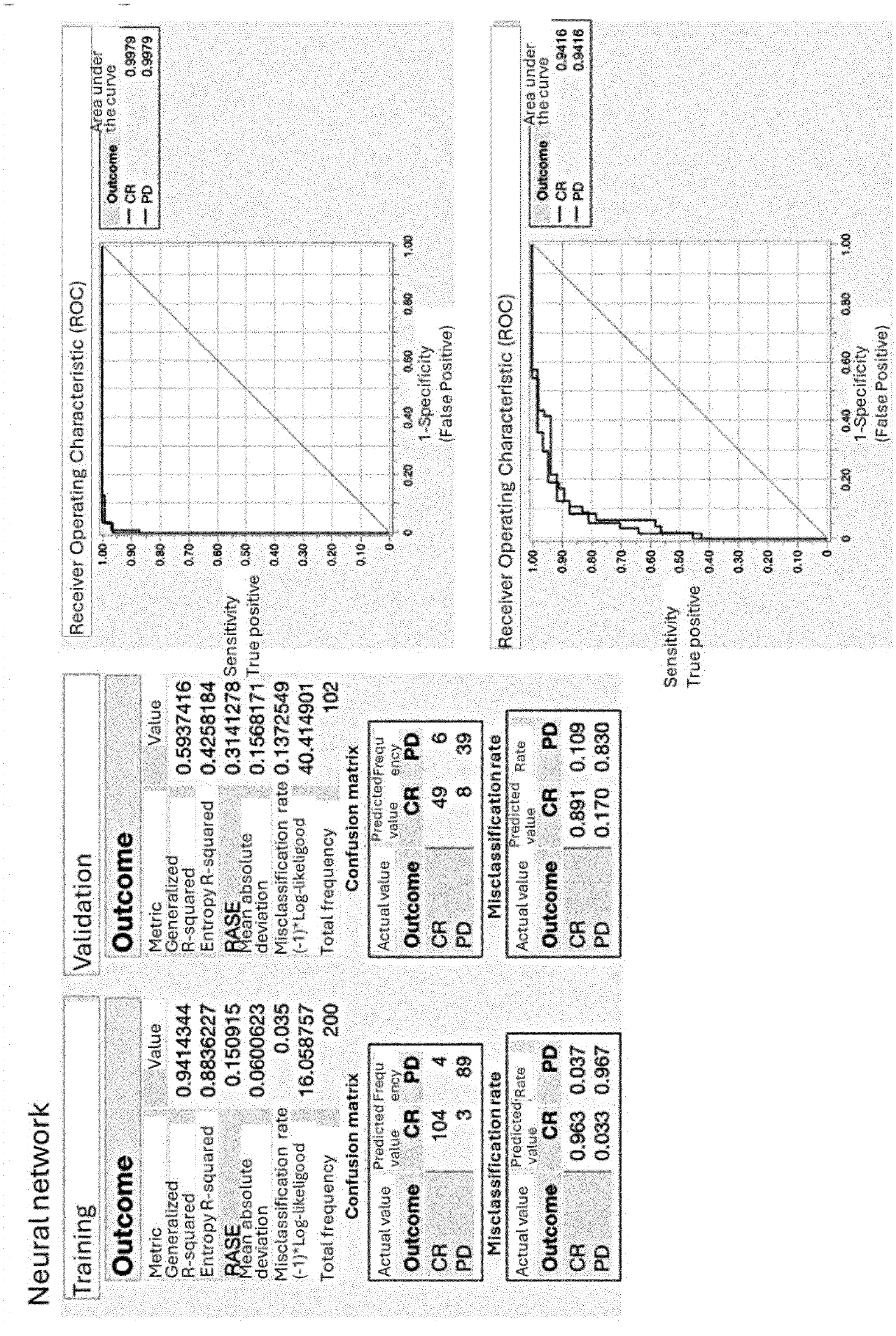
Neural network
Training
Outcome
Metric
Value
Generalized R-squared 0.9414344
Entropy R-squared 0.8836227
RASE 0.150915
Mean absolute deviation 0.0600623
Misclassification rate 0.035
(-1)*Log-likeligood 16.058757
Total frequency 200
Confusion matrix
Actual value
Predicted value
Frequency
Outcome CR PD
CR 104 4
PD 3 89
Misclassification rate
Actual value
Predicted value
Rate
Outcome CR PD
CR 0.963 0.037
PD 0.033 0.967
Validation
Outcome
Metric
Value
Generalized R-squared 0.5937416
Entropy R-squared 0.4258184
RASE 0.3141278
Mean absolute deviation 0.1568171
Misclassification rate 0.1372549
(-1)*Log-likeligood 40.414901
Total frequency 102
Confusion matrix
Actual value
Predicted value
Frequency
Outcome CR PD
CR 49 6
PD 8 39
Misclassification rate
Actual value
Predicted value
Rate
Outcome CR PD
CR 0.891 0.109
PD 0.170 0.830
Receiver Operating Characteristic (ROC)
Sensitivity
True positive
1-Specificity
(False Positive)
Outcome
CR
PD
Area under the curve
0.9979
0.9979
Receiver Operating Characteristic (ROC)
Sensitivity
True positive
1-Specificity
(False Positive)
Outcome
CR
PD
Area under the curve
0.9416
0.9416

FIG. 10

## Random forest

| Metric | Training | Definition |
|---|---|---|
| Entropy R-squared | 0.5085 | 1-Loglike(model)/Loglike(0) |
| Generalized R-squared | 0.6737 | (1-(L(0)/L(model))^(2/n))/(1-L(0)^(2/n)) |
| Mean-logP | 0.3392 | ∑ -Log(ρ[j])/n |
| RASE | 0.2992 | √ ∑(y[j]-ρ[j])²/n |
| Mean absolute deviation | 0.2788 | ∑ \|y[j]-ρ[j]\|/n |
| Misclassification rate | 0.0331 | ∑ (ρ[j]≠ρMax)/n |
| N | 302 | n |

Training

| Actual value | Predicted value | Frequency |
|---|---|---|
| **Outcome** | **CR** | **PD** |
| CR | 157 | 6 |
| PD | 4 | 135 |

| Actual value | Predicted value | Ratio |
|---|---|---|
| **Outcome** | **CR** | **PD** |
| CR | 0.963 | 0.037 |
| PD | 0.029 | 0.971 |

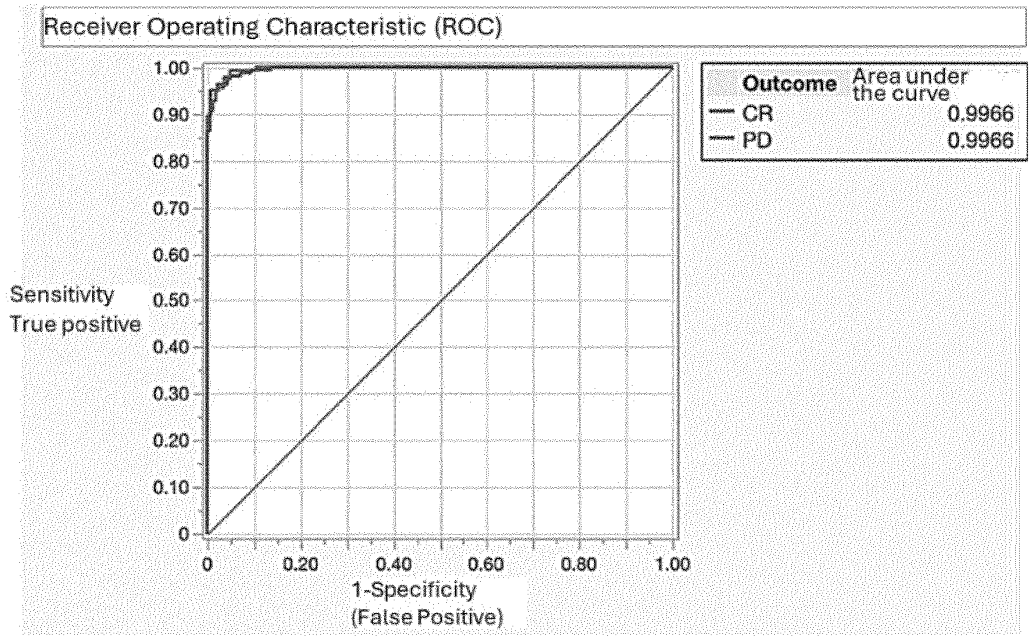


FIG. 11

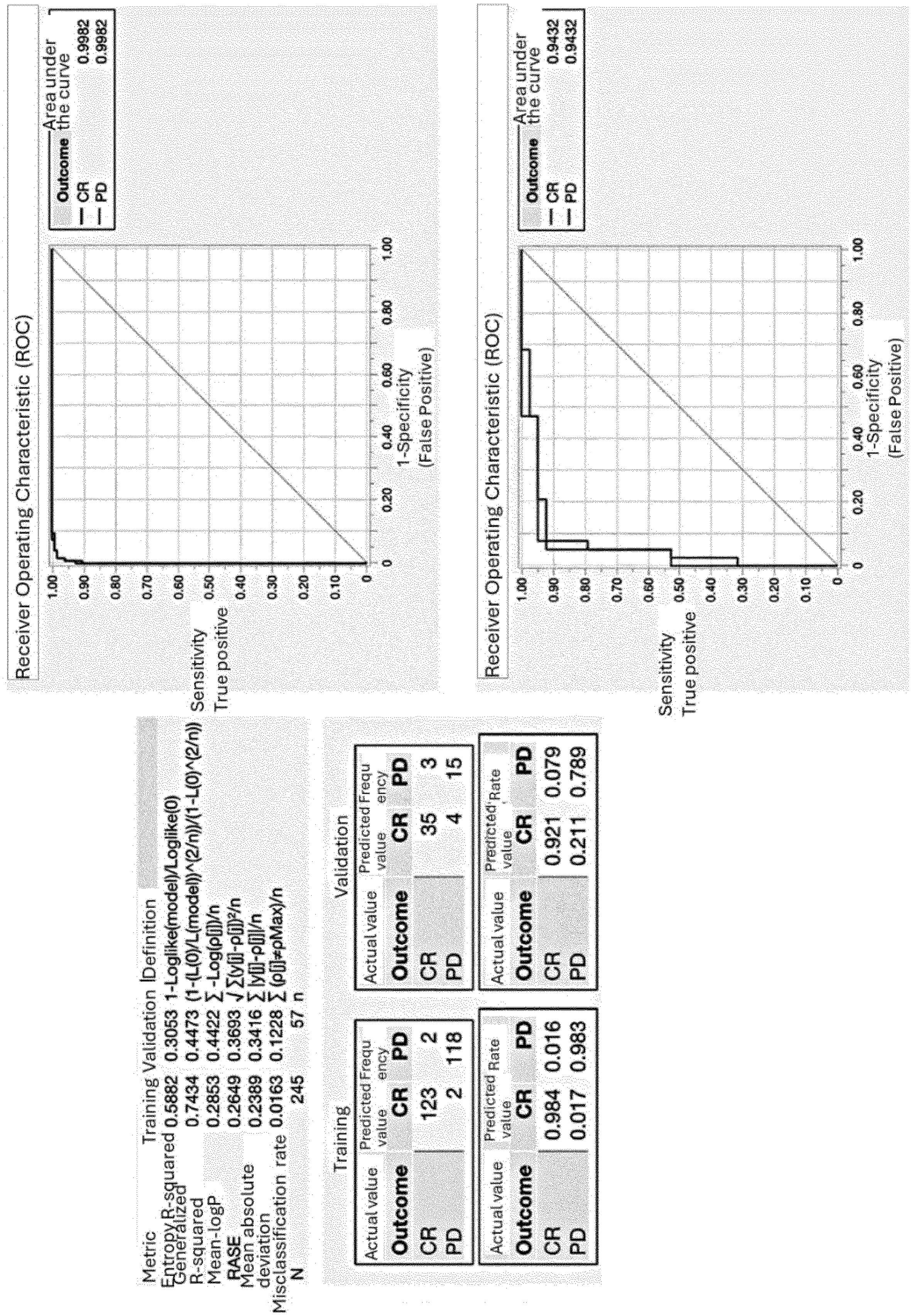
Receiver Operating Characteristic (ROC)
Sensitivity
True positive
1-Specificity
(False Positive)
Outcome
Area under the curve
CR 0.9982
PD 0.9982
Receiver Operating Characteristic (ROC)
Sensitivity
True positive
1-Specificity
(False Positive)
Outcome
Area under the curve
CR 0.9432
PD 0.9432
Metric Training Validation Definition
Entropy R-squared 0.5882 0.3053 1-Loglike(model)/Loglike(0)
Generalized R-squared 0.7434 0.4473 (1-(L(0)/L(model))^(2/n))/(1-L(0)^(2/n))
Mean-logP 0.2853 0.4422 ∑ -Log(ρ[j])/n
RASE 0.2649 0.3693 √ ∑(y[j]-ρ[j])²/n
Mean absolute deviation 0.2389 0.3416 ∑ |y[j]-ρ[j]|/n
Misclassification rate 0.0163 0.1228 ∑ (ρ[j]≠ρMax)/n
N 245 57 n
Training
Actual value
Outcome
Predicted value
Frequency
CR 123 2
PD 2 118
Predicted value
Rate
CR 0.984 0.016
PD 0.017 0.983
Validation
Actual value
Outcome
Predicted value
Frequency
CR 35 3
PD 4 15
Predicted value
Rate
CR 0.921 0.079
PD 0.211 0.789

FIG. 12

# Logistic regression analysis

## 30 genes

| | |
|---|---|
| ChiSq(U) | 0.6338 |
| AICc | 221.945 |
| BIC | 329.62 |
| Observations (or sum of weights) | 302 |

Receiver Operating Characteristic (ROC)

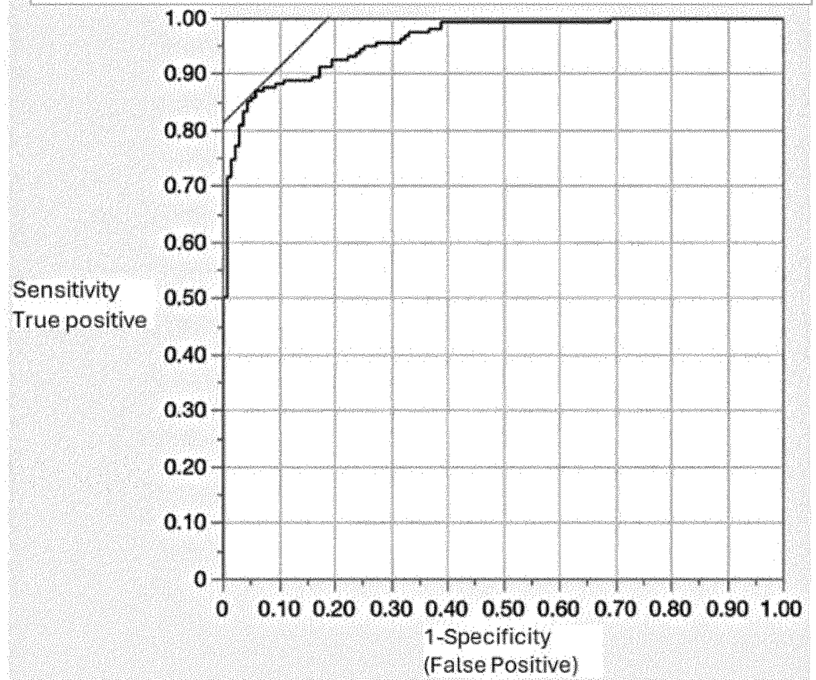


Outcome = CR is treated as positive.

**AUC**
0.96076

**Likelihood-ratio test for the effect**

| Factor | Number of parameters | Degree of freedom | Likelihood-Ratio Chi-Sq | P value (Prob > ChiSq) |
|---|---|---|---|---|
| CLEC12A | 1 | 1 | 12.3028594 | 0.0005* |
| TMEM176A | 1 | 1 | 2.72981858 | 0.0985 |
| CD52 | 1 | 1 | 3.37409869 | 0.0662 |
| S100B | 1 | 1 | 9.79112016 | 0.0018* |
| SH2D2A | 1 | 1 | 2.29852204 | 0.1295 |
| SIDT2 | 1 | 1 | 5.50501151 | 0.0190* |
| GTF3C1 | 1 | 1 | 4.98944074 | 0.0255* |
| ZNF266 | 1 | 1 | 7.01133112 | 0.0081* |
| MS4A7 | 1 | 1 | 0.98868509 | 0.3201 |
| LST1 | 1 | 1 | 0.61878135 | 0.4315 |
| SH2B3 | 1 | 1 | 5.94423165 | 0.0148* |
| AIF1 | 1 | 1 | 0.01131957 | 0.9153 |
| LAIR2 | 1 | 1 | 25.5393315 | <.0001* |
| RETN | 1 | 1 | 0.25490969 | 0.6136 |
| LYPD2 | 1 | 1 | 0.59515802 | 0.4404 |
| DIP2A | 1 | 1 | 2.11327866 | 0.1460 |
| RNF14 | 1 | 1 | 3.56039903 | 0.0592 |
| UBE2F | 1 | 1 | 1.78560517 | 0.1815 |
| CTSS | 1 | 1 | 0.06228519 | 0.8029 |
| FGL2 | 1 | 1 | 0.80331759 | 0.3701 |
| HAVCR2 | 1 | 1 | 2.58571583 | 0.1078 |
| LILRB2 | 1 | 1 | 0.07733244 | 0.7809 |
| TMEM176B | 1 | 1 | 8.61979628 | 0.0033* |
| CLU | 1 | 1 | 5.40192298 | 0.0201* |
| SELL | 1 | 1 | 4.13008873 | 0.0421* |
| PHTF2 | 1 | 1 | 1.90484288 | 0.1675 |
| PSAP | 1 | 1 | 2.85540097 | 0.0911 |
| SLA2 | 1 | 1 | 0.14326268 | 0.7051 |
| ID2 | 1 | 1 | 0.37536714 | 0.5401 |
| HCK | 1 | 1 | 0.00454899 | 0.9462 |

FIG. 13

## Logistic regression analysis

### 40 genes

| | |
|---|---|
| ChiSq(U) | 0.6813 |
| AICc | 228.082 |
| BIC | 366.963 |
| Observations (or sum of weights) 計) | 302 |

Receiver Operating Characteristic (ROC)

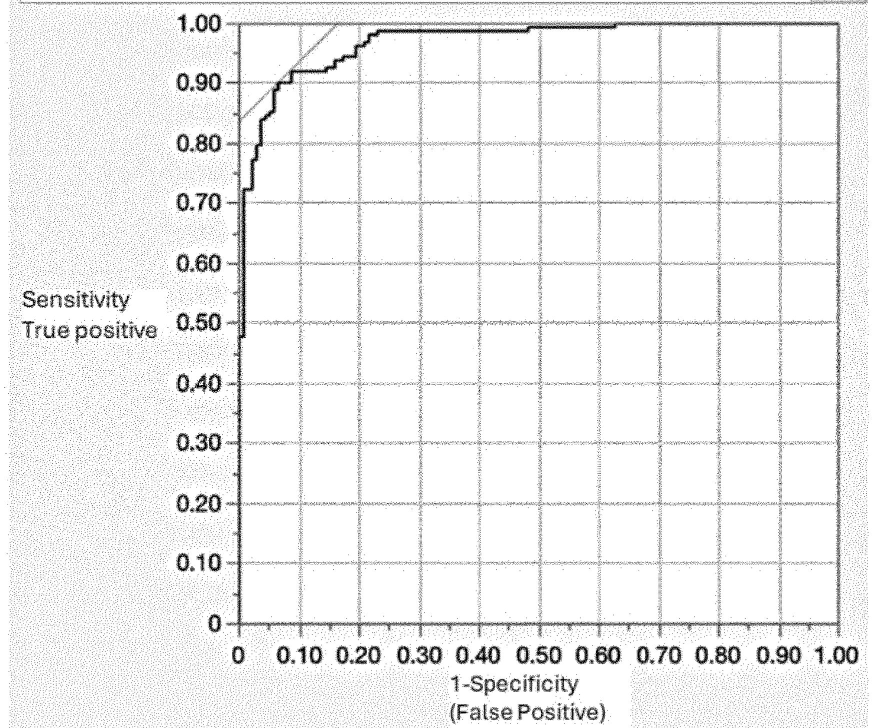


Outcome = CR is treated as positive.

| AUC |
|---|
| 0.97025 |

**Likelihood-ratio test for the effect**

| Factor | Number of parameters | Degree of freedom | Likelihood-Ratio Chi-Sq | P value (Prob > ChiSq) |
|---|---|---|---|---|
| CLEC12A | 1 | 1 | 11.35034 | 0.0008* |
| TMEM176A | 1 | 1 | 1.67876874 | 0.1951 |
| CD52 | 1 | 1 | 1.29865163 | 0.2545 |
| S100B | 1 | 1 | 9.30952378 | 0.0023* |
| SH2D2A | 1 | 1 | 3.09292634 | 0.0786 |
| SIDT2 | 1 | 1 | 5.42001255 | 0.0199* |
| GTF3C1 | 1 | 1 | 1.53097074 | 0.2160 |
| ZNF266 | 1 | 1 | 5.76351027 | 0.0164* |
| MS4A7 | 1 | 1 | 1.54212747 | 0.2143 |
| LST1 | 1 | 1 | 0.01395314 | 0.9060 |
| SH2B3 | 1 | 1 | 5.15305891 | 0.0232* |
| AIF1 | 1 | 1 | 0.61087705 | 0.4345 |
| LAIR2 | 1 | 1 | 16.4092996 | <.0001* |
| RETN | 1 | 1 | 0.3289141 | 0.5663 |
| LYPD2 | 1 | 1 | 0.8391549 | 0.3596 |
| DIP2A | 1 | 1 | 1.44700652 | 0.2290 |
| RNF14 | 1 | 1 | 1.51077421 | 0.2190 |
| UBE2F | 1 | 1 | 1.05215748 | 0.3050 |
| CTSS | 1 | 1 | 0.1760686 | 0.6748 |
| FGL2 | 1 | 1 | 1.38803727 | 0.2387 |
| HAVCR2 | 1 | 1 | 2.13869471 | 0.1436 |
| LILRB2 | 1 | 1 | 0.01572749 | 0.9002 |
| TMEM176B | 1 | 1 | 6.59195919 | 0.0102* |
| CLU | 1 | 1 | 4.1696449 | 0.0412* |
| SELL | 1 | 1 | 4.73885539 | 0.0295* |
| PHTF2 | 1 | 1 | 0.72171833 | 0.3956 |
| PSAP | 1 | 1 | 4.05232928 | 0.0441* |
| SLA2 | 1 | 1 | 9.79072e-7 | 0.9992 |
| ID2 | 1 | 1 | 0.07272465 | 0.7874 |
| HCK | 1 | 1 | 0.00088033 | 0.9763 |
| EGR1 | 1 | 1 | 1.85137223 | 0.1736 |
| CD247 | 1 | 1 | 0.69164056 | 0.4056 |
| GNLY | 1 | 1 | 1.33976543 | 0.2471 |
| SH2D1B | 1 | 1 | 2.85622732 | 0.0910 |
| PRF1 | 1 | 1 | 0.02942576 | 0.8638 |
| IL1RN | 1 | 1 | 2.63080702 | 0.1048 |
| PECAM1 | 1 | 1 | 0.08693986 | 0.7681 |
| KIR2DL3 | 1 | 1 | 3.53216463 | 0.0602 |
| UBE2G2 | 1 | 1 | 1.49444429 | 0.2215 |
| C1QA | 1 | 1 | 2.36936145 | 0.1237 |

FIG. 14

## Logistic regression analysis

### 45 genes

| | |
|---|---|
| ChiSq(U) | 0.7133 |
| AICc | 228.444 |
| BIC | 382.167 |
| Observations (or sum of weights) | 302 |

Receiver Operating Characteristic (ROC)

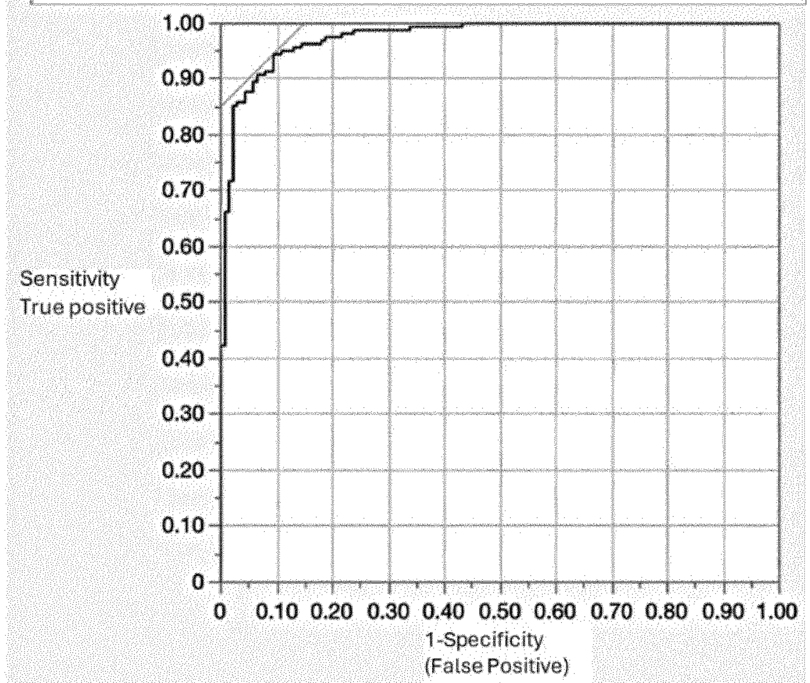


Outcome = CR is treated as positive.

| AUC |
|---|
| 0.97630 |

**Likelihood-ratio test for the effect**

| Factor | Number of parameters | Degree of freedom | Likelihood-Ratio Chi-Sq | P value (Prob > ChiSq) |
|---|---|---|---|---|
| CLEC12A | 1 | 1 | 7.5250552 | 0.0061* |
| TMEM176A | 1 | 1 | 0.68962907 | 0.4063 |
| CD52 | 1 | 1 | 1.80860425 | 0.1787 |
| S100B | 1 | 1 | 8.20929085 | 0.0042* |
| SH2D2A | 1 | 1 | 2.3354624 | 0.1265 |
| SIDT2 | 1 | 1 | 6.00576757 | 0.0143* |
| GTF3C1 | 1 | 1 | 2.1651799 | 0.1412 |
| ZNF266 | 1 | 1 | 3.93286304 | 0.0474* |
| MS4A7 | 1 | 1 | 1.70981992 | 0.1910 |
| LST1 | 1 | 1 | 0.09152258 | 0.7623 |
| SH2B3 | 1 | 1 | 4.23115831 | 0.0397* |
| AIF1 | 1 | 1 | 0.53626027 | 0.4640 |
| LAIR2 | 1 | 1 | 11.9004277 | 0.0006* |
| RETN | 1 | 1 | 0.70770363 | 0.4002 |
| LYPD2 | 1 | 1 | 0.78578749 | 0.3754 |
| DIP2A | 1 | 1 | 2.91667193 | 0.0877 |
| RNF14 | 1 | 1 | 0.5522598 | 0.4574 |
| UBE2F | 1 | 1 | 0.23608177 | 0.6271 |
| CTSS | 1 | 1 | 0.19142285 | 0.6617 |
| FGL2 | 1 | 1 | 0.29853363 | 0.5848 |
| HAVCR2 | 1 | 1 | 3.60830088 | 0.0575 |
| LILRB2 | 1 | 1 | 0.00124547 | 0.9718 |
| TMEM176B | 1 | 1 | 8.64469024 | 0.0033* |
| CLU | 1 | 1 | 7.08850187 | 0.0078* |
| SELL | 1 | 1 | 4.55321516 | 0.0329* |
| PHTF2 | 1 | 1 | 1.66420691 | 0.1970 |
| PSAP | 1 | 1 | 4.13665061 | 0.0420* |
| SLA2 | 1 | 1 | 0.00790341 | 0.9292 |
| ID2 | 1 | 1 | 0.05397005 | 0.8163 |
| HCK | 1 | 1 | 0.31055505 | 0.5773 |
| EGR1 | 1 | 1 | 1.15186547 | 0.2832 |
| CD247 | 1 | 1 | 0.16309294 | 0.6863 |
| GNLY | 1 | 1 | 0.02940158 | 0.8639 |
| SH2D1B | 1 | 1 | 2.51036911 | 0.1131 |
| PRF1 | 1 | 1 | 0.18648052 | 0.6659 |
| IL1RN | 1 | 1 | 2.11978604 | 0.1454 |
| PECAM1 | 1 | 1 | 0.02945469 | 0.8637 |
| KIR2DL3 | 1 | 1 | 2.7404117 | 0.0978 |
| UBE2G2 | 1 | 1 | 1.32039321 | 0.2505 |
| C1QA | 1 | 1 | 3.42862693 | 0.0641 |
| NR4A2 | 1 | 1 | 0.25440876 | 0.6140 |
| HK2 | 1 | 1 | 5.23156874 | 0.0222* |
| XRCC5 | 1 | 1 | 0.53724843 | 0.4636 |
| CXCR4 | 1 | 1 | 5.40259129 | 0.0201* |
| LYST | 1 | 1 | 0.60603911 | 0.4363 |

FIG. 15

## Logistic regression analysis

50 genes

| | |
|---|---|
| ChiSq(U) | 0.7349 |
| AICc | 233.694 |
| BIC | 401.71 |
| Observations (or sum of weights) | 302 |

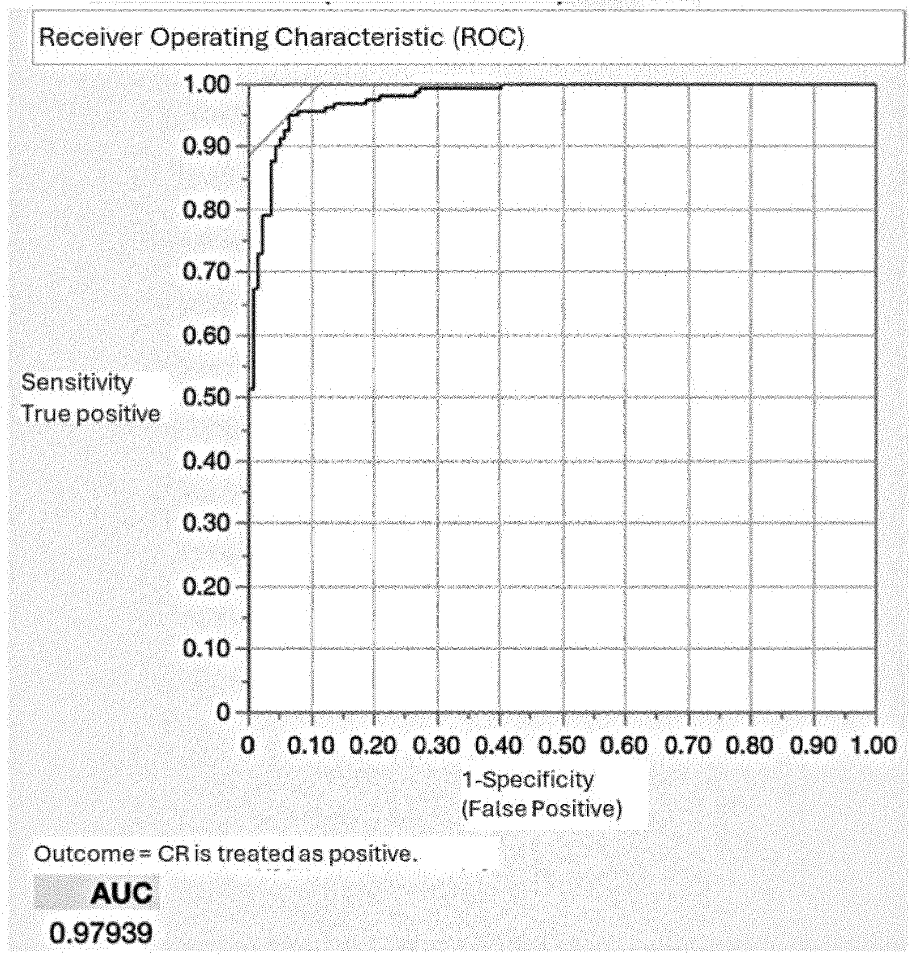


**Likelihood-ratio test for the effect**

| Factor | Number of parameters | Degree of freedom | Likelihood -Ratio Chi-Sq | P value (Prob > ChiSq) |
|---|---|---|---|---|
| CLEC12A | 1 | 1 | 8.52755229 | 0.0035* |
| TMEM176A | 1 | 1 | 0.5418003 | 0.4617 |
| CD52 | 1 | 1 | 1.46368502 | 0.2263 |
| S100B | 1 | 1 | 8.56028977 | 0.0034* |
| SH2D2A | 1 | 1 | 2.85197322 | 0.0913 |
| SIDT2 | 1 | 1 | 3.53533152 | 0.0601 |
| GTF3C1 | 1 | 1 | 2.13142723 | 0.1443 |
| ZNF266 | 1 | 1 | 3.80918909 | 0.0510 |
| MS4A7 | 1 | 1 | 1.94968646 | 0.1626 |
| LST1 | 1 | 1 | 0.71747679 | 0.3970 |
| SH2B3 | 1 | 1 | 3.8103568 | 0.0509 |
| AIF1 | 1 | 1 | 0.24863393 | 0.6180 |
| LAIR2 | 1 | 1 | 12.6737791 | 0.0004* |
| RETN | 1 | 1 | 0.56402749 | 0.4526 |
| LYPD2 | 1 | 1 | 0.57507164 | 0.4483 |
| DIP2A | 1 | 1 | 3.38292773 | 0.0659 |
| RNF14 | 1 | 1 | 1.31140882 | 0.2521 |
| UBE2F | 1 | 1 | 1.07177e-5 | 0.9974 |
| CTSS | 1 | 1 | 0.28301496 | 0.5947 |
| FGL2 | 1 | 1 | 0.23077316 | 0.6310 |
| HAVCR2 | 1 | 1 | 3.94502145 | 0.0470* |
| LILRB2 | 1 | 1 | 0.53452146 | 0.4647 |
| TMEM176B | 1 | 1 | 9.11377417 | 0.0025* |
| CLU | 1 | 1 | 6.55241766 | 0.0105* |
| SELL | 1 | 1 | 4.10434303 | 0.0428* |
| PHTF2 | 1 | 1 | 2.15372919 | 0.1422 |
| PSAP | 1 | 1 | 6.47420291 | 0.0109* |
| SLA2 | 1 | 1 | 0.0535501 | 0.8170 |
| ID2 | 1 | 1 | 0.46628422 | 0.4947 |
| HCK | 1 | 1 | 0.02072113 | 0.8855 |
| EGR1 | 1 | 1 | 0.07038641 | 0.7908 |
| CD247 | 1 | 1 | 0.10140851 | 0.7501 |
| GNLY | 1 | 1 | 0.0002332 | 0.9878 |
| SH2D1B | 1 | 1 | 3.01404669 | 0.0825 |
| PRF1 | 1 | 1 | 0.16810166 | 0.6818 |
| IL1RN | 1 | 1 | 3.18570759 | 0.0743 |
| PECAM1 | 1 | 1 | 0.39873011 | 0.5277 |
| KIR2DL3 | 1 | 1 | 2.63164378 | 0.1048 |
| UBE2G2 | 1 | 1 | 0.90769134 | 0.3407 |
| C1QA | 1 | 1 | 3.28332995 | 0.0700 |
| NR4A2 | 1 | 1 | 1.21156937 | 0.2710 |
| HK2 | 1 | 1 | 4.27494129 | 0.0387* |
| XRCC5 | 1 | 1 | 1.16839803 | 0.2797 |
| CXCR4 | 1 | 1 | 6.49708139 | 0.0108* |
| LYST | 1 | 1 | 0.96167475 | 0.3268 |
| CSF1R | 1 | 1 | 0.04626225 | 0.8297 |
| ANKRD28 | 1 | 1 | 0.24802588 | 0.6185 |
| WARS | 1 | 1 | 0.7869678 | 0.3750 |
| HBEGF | 1 | 1 | 8.32464159 | 0.0039* |
| CAT | 1 | 1 | 0.00239542 | 0.9610 |

FIG. 16

# Logistic regression analysis

## 55 genes

| | |
|---|---|
| ChiSq(U) | 0.7565 |
| AICc | 239.524 |
| BIC | 421.251 |
| Observations (or sum of weights) | 302 |

Receiver Operating Characteristic (ROC)

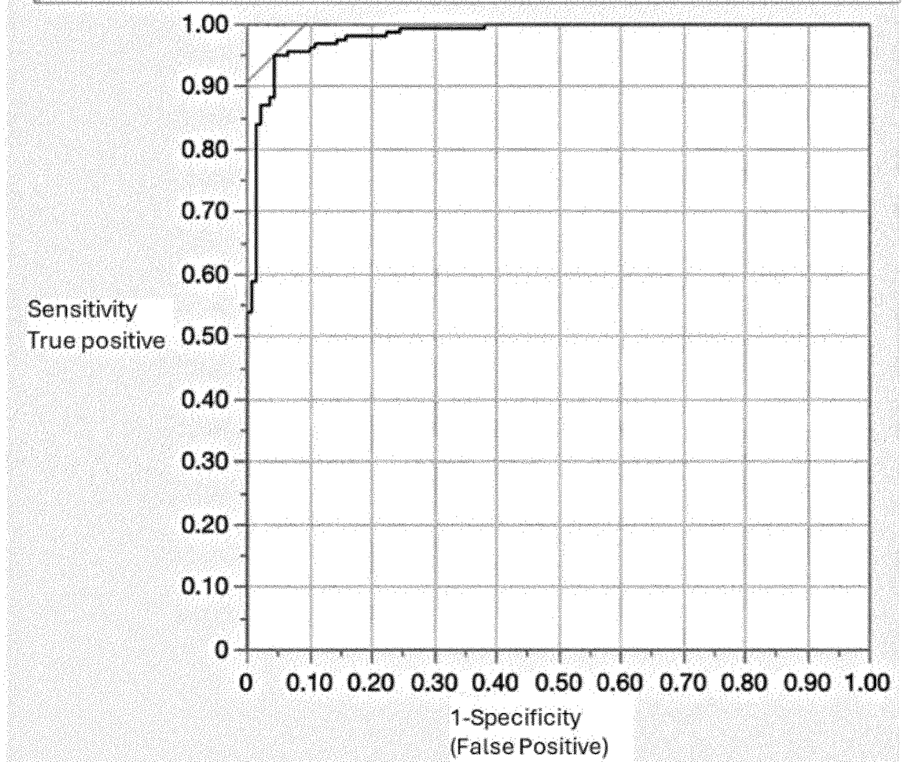


Outcome = CR is treated as positive.

**AUC**
0.98327

**Likelihood-ratio test for the effect**

| Factor | Number of parameters | Degree of freedom | Likelihood -Ratio Chi-Sq | P value (Prob > ChiSq) |
|---|---|---|---|---|
| CLEC12A | 1 | 1 | 4.8813288 | 0.0271* |
| TMEM176A | 1 | 1 | 1.81978577 | 0.1773 |
| CD52 | 1 | 1 | 1.41463263 | 0.2343 |
| S100B | 1 | 1 | 8.33508026 | 0.0039* |
| SH2D2A | 1 | 1 | 2.78672775 | 0.0950 |
| SIDT2 | 1 | 1 | 4.17430165 | 0.0410* |
| GTF3C1 | 1 | 1 | 2.85900842 | 0.0909 |
| ZNF266 | 1 | 1 | 3.59413086 | 0.0580 |
| MS4A7 | 1 | 1 | 2.83330263 | 0.0923 |
| LST1 | 1 | 1 | 0.63649169 | 0.4250 |
| SH2B3 | 1 | 1 | 4.39410765 | 0.0361* |
| AIF1 | 1 | 1 | 0.3868539 | 0.5340 |
| LAIR2 | 1 | 1 | 10.2040535 | 0.0014* |
| RETN | 1 | 1 | 0.82158156 | 0.3647 |
| LYPD2 | 1 | 1 | 2.16223995 | 0.1414 |
| DIP2A | 1 | 1 | 2.96598446 | 0.0850 |
| RNF14 | 1 | 1 | 0.57808535 | 0.4471 |
| UBE2F | 1 | 1 | 0.01986728 | 0.8879 |
| CTSS | 1 | 1 | 0.00078288 | 0.9777 |
| FGL2 | 1 | 1 | 0.18467143 | 0.6674 |
| HAVCR2 | 1 | 1 | 4.04092975 | 0.0444* |
| LILRB2 | 1 | 1 | 1.09666126 | 0.2950 |
| TMEM176B | 1 | 1 | 7.29854467 | 0.0069* |
| CLU | 1 | 1 | 8.84249044 | 0.0029* |
| SELL | 1 | 1 | 3.4947082 | 0.0616 |
| PHTF2 | 1 | 1 | 3.85282048 | 0.0497* |
| PSAP | 1 | 1 | 9.46411276 | 0.0021* |
| SLA2 | 1 | 1 | 0.01623391 | 0.8986 |
| ID2 | 1 | 1 | 0.30894031 | 0.5783 |
| HCK | 1 | 1 | 0.00829819 | 0.9274 |
| EGR1 | 1 | 1 | 0.03558245 | 0.8504 |
| CD247 | 1 | 1 | 0.00536041 | 0.9416 |
| GNLY | 1 | 1 | 0.0059556 | 0.9385 |
| SH2D1B | 1 | 1 | 2.75869063 | 0.0967 |
| PRF1 | 1 | 1 | 0.35188622 | 0.5530 |
| IL1RN | 1 | 1 | 2.84392731 | 0.0917 |
| PECAM1 | 1 | 1 | 0.00033453 | 0.9854 |
| KIR2DL3 | 1 | 1 | 2.96914935 | 0.0849 |
| UBE2G2 | 1 | 1 | 0.85390072 | 0.3555 |
| C1QA | 1 | 1 | 0.52702137 | 0.4679 |
| NR4A2 | 1 | 1 | 1.19684137 | 0.2740 |
| HK2 | 1 | 1 | 6.00461804 | 0.0143* |
| XRCC5 | 1 | 1 | 0.87144392 | 0.3506 |
| CXCR4 | 1 | 1 | 8.77262855 | 0.0031* |
| LYST | 1 | 1 | 1.3259128 | 0.2495 |
| CSF1R | 1 | 1 | 0.42234503 | 0.5158 |
| ANKRD28 | 1 | 1 | 0.27989469 | 0.5968 |
| WARS | 1 | 1 | 0.04146006 | 0.8387 |
| HBEGF | 1 | 1 | 7.51769468 | 0.0061* |
| CAT | 1 | 1 | 0.01073207 | 0.9175 |
| CYBB | 1 | 1 | 5.00524275 | 0.0253* |
| CTSL | 1 | 1 | 0.08529207 | 0.7702 |
| PRMT2 | 1 | 1 | 0.04939618 | 0.8241 |
| SLC39A8 | 1 | 1 | 4.4980346 | 0.0339* |
| FCGR3A | 1 | 1 | 0.01139162 | 0.9150 |

FIG. 17

## Logistic regression analysis
60 genes

| | |
|---|---|
| ChiSq(U) | 0.8040 |
| AICc | 235.192 |
| BIC | 430.012 |
| Observations (or sum of weights) | 302 |

Receiver Operating Characteristic (ROC)

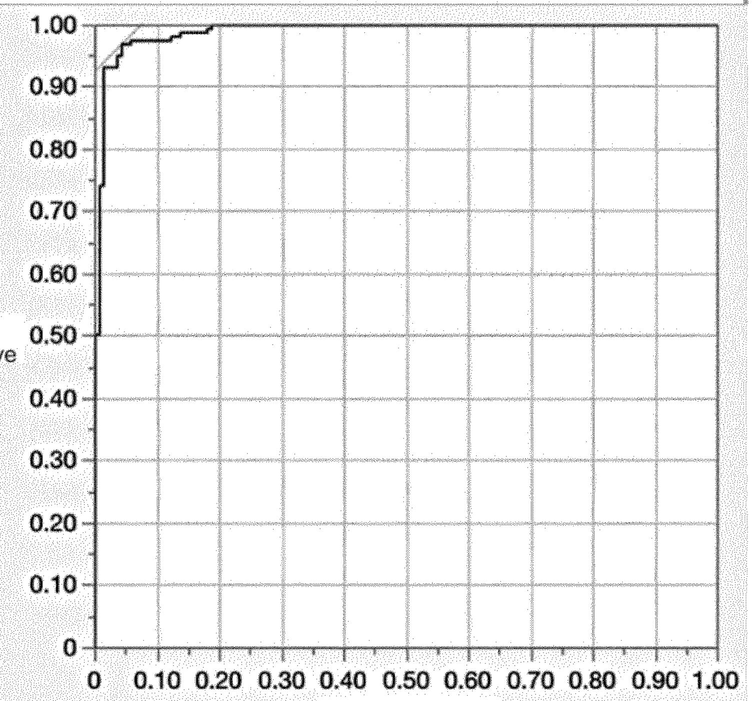


Outcome = CR is treated as positive.

| AUC |
|---|
| 0.98989 |

## Likelihood-ratio test for the effect

| Factor | Number of parameters | Degree of freedom | Likelihood -Ratio Chi-Sq | P value (Prob > ChiSq) |
|---|---|---|---|---|
| CLEC12A | 1 | 1 | 2.56836109 | 0.1090 |
| TMEM176A | 1 | 1 | 4.68978224 | 0.0303* |
| CD52 | 1 | 1 | 0.44022695 | 0.5070 |
| S100B | 1 | 1 | 15.2565749 | <.0001* |
| SH2D2A | 1 | 1 | 6.6690474 | 0.0098* |
| SIDT2 | 1 | 1 | 5.36814168 | 0.0205* |
| GTF3C1 | 1 | 1 | 3.63858759 | 0.0565 |
| ZNF266 | 1 | 1 | 3.42249565 | 0.0643 |
| MS4A7 | 1 | 1 | 7.47686332 | 0.0062* |
| LST1 | 1 | 1 | 1.42779909 | 0.2321 |
| SH2B3 | 1 | 1 | 5.92380711 | 0.0149* |
| AIF1 | 1 | 1 | 1.78311168 | 0.1818 |
| LAIR2 | 1 | 1 | 8.57455015 | 0.0034* |
| RETN | 1 | 1 | 0.96812878 | 0.3251 |
| LYPD2 | 1 | 1 | 1.49968773 | 0.2207 |
| DIP2A | 1 | 1 | 2.67532627 | 0.1019 |
| RNF14 | 1 | 1 | 0.21360958 | 0.6440 |
| UBE2F | 1 | 1 | 0.17734635 | 0.6737 |
| CTSS | 1 | 1 | 0.03259099 | 0.8567 |
| FGL2 | 1 | 1 | 3.42457284 | 0.0642 |
| HAVCR2 | 1 | 1 | 5.04550167 | 0.0247* |
| LILRB2 | 1 | 1 | 0.05149646 | 0.8205 |
| TMEM176B | 1 | 1 | 9.28526902 | 0.0023* |
| CLU | 1 | 1 | 5.21422034 | 0.0224* |
| SELL | 1 | 1 | 9.28154814 | 0.0023* |
| PHTF2 | 1 | 1 | 4.10528256 | 0.0427* |
| PSAP | 1 | 1 | 12.3340963 | 0.0004* |
| SLA2 | 1 | 1 | 0.06204083 | 0.8033 |
| ID2 | 1 | 1 | 1.67001021 | 0.1963 |
| HCK | 1 | 1 | 1.29501803 | 0.2551 |
| EGR1 | 1 | 1 | 0.04500068 | 0.8320 |
| CD247 | 1 | 1 | 0.34682387 | 0.5559 |
| GNLY | 1 | 1 | 0.52953767 | 0.4668 |
| SH2D1B | 1 | 1 | 3.14809503 | 0.0760 |
| PRF1 | 1 | 1 | 1.53495814 | 0.2154 |
| IL1RN | 1 | 1 | 2.24555202 | 0.1340 |
| PECAM1 | 1 | 1 | 0.13812972 | 0.7101 |
| KIR2DL3 | 1 | 1 | 3.83688218 | 0.0501 |
| UBE2G2 | 1 | 1 | 1.01459054 | 0.3138 |
| C1QA | 1 | 1 | 0.99401894 | 0.3188 |
| NR4A2 | 1 | 1 | 1.80690288 | 0.1789 |
| HK2 | 1 | 1 | 4.80698684 | 0.0283* |
| XRCC5 | 1 | 1 | 1.54035663 | 0.2146 |
| CXCR4 | 1 | 1 | 14.6167931 | 0.0001* |
| LYST | 1 | 1 | 0.03674741 | 0.8480 |
| CSF1R | 1 | 1 | 0.10906924 | 0.7412 |
| ANKRD28 | 1 | 1 | 0.07656255 | 0.7820 |
| WARS | 1 | 1 | 0.02737117 | 0.8686 |
| HBEGF | 1 | 1 | 3.53843628 | 0.0600 |
| CAT | 1 | 1 | 0.52780105 | 0.4675 |
| CYBB | 1 | 1 | 5.05034298 | 0.0246* |
| CTSL | 1 | 1 | 5.01515e-7 | 0.9994 |
| PRMT2 | 1 | 1 | 0.09417752 | 0.7589 |
| SLC39A8 | 1 | 1 | 7.28532792 | 0.0070* |
| FCGR3A | 1 | 1 | 0.06651579 | 0.7965 |
| GPR183 | 1 | 1 | 0.01894753 | 0.8905 |
| STAB1 | 1 | 1 | 6.82698878 | 0.0090* |
| CASP1 | 1 | 1 | 2.41474405 | 0.1202 |
| EGR2 | 1 | 1 | 1.77962533 | 0.1822 |
| FOLR3 | 1 | 1 | 11.8526053 | 0.0006* |

FIG. 18

# 302 cells (QC pass)

## Factor Selection

- 10 genes (Stepwise method)

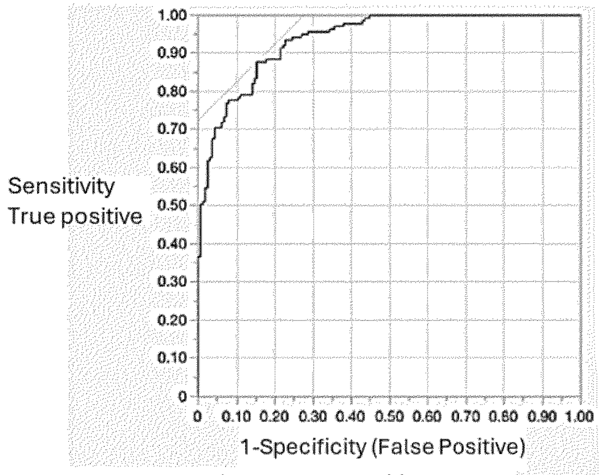


Outcome = PD is treated as positive.

| AUC |
|---|
| 0.93861 |

Overall model test

| Model | (-1)*Log-likeligood | Degree of freedom | ChiSq | P value (Prob > ChiSq) |
|---|---|---|---|---|
| Difference | 115.13953 | 10 | 230.2791 | <.0001* |
| Full | 93.23627 | | | |
| Reduced | 208.37580 | | | |

| | |
|---|---|
| ChiSq(U) | 0.5526 |
| AICc | 209.383 |
| BIC | 249.287 |
| Observations (or sum of weights) | 302 |

| Factor | Number of parameters | Degree of freedom | Likelihood -Ratio Chi-Sq | P value (Prob > ChiSq) |
|---|---|---|---|---|
| HEATR5B | 1 | 1 | 9.49457413 | 0.0021* |
| GTF3C1 | 1 | 1 | 21.8044622 | <.0001* |
| TMEM176B | 1 | 1 | 42.3328192 | <.0001* |
| CCL2 | 1 | 1 | 8.28290369 | 0.0040* |
| SH2B3 | 1 | 1 | 15.1396876 | <.0001* |
| HAVCR2 | 1 | 1 | 13.2575596 | 0.0003* |
| LAIR2 | 1 | 1 | 47.4072338 | <.0001* |
| CD52 | 1 | 1 | 17.705936 | <.0001* |
| CLEC12A | 1 | 1 | 55.0481999 | <.0001* |
| ZNF266 | 1 | 1 | 24.1827968 | <.0001* |

| Factor | Log value | P value |
|---|---|---|
| CLEC12A | 12.930 | 0.00000 |
| LAIR2 | 11.239 | 0.00000 |
| TMEM176B | 10.114 | 0.00000 |
| ZNF266 | 6.057 | 0.00000 |
| GTF3C1 | 5.520 | 0.00000 |
| CD52 | 4.589 | 0.00003 |
| SH2B3 | 4.001 | 0.00010 |
| HAVCR2 | 3.566 | 0.00027 |
| HEATR5B | 2.686 | 0.00206 |
| CCL2 | 2.398 | 0.00400 |

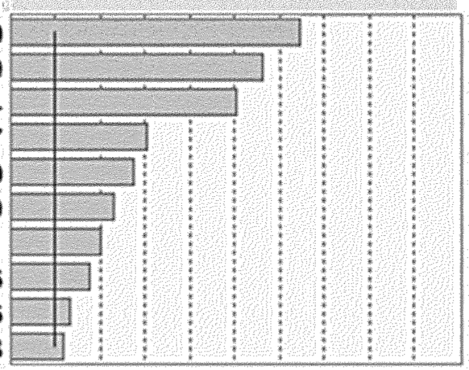

FIG. 19

302 cells (QC pass)

Factor Selection

- 47 genes (LASSO regression)

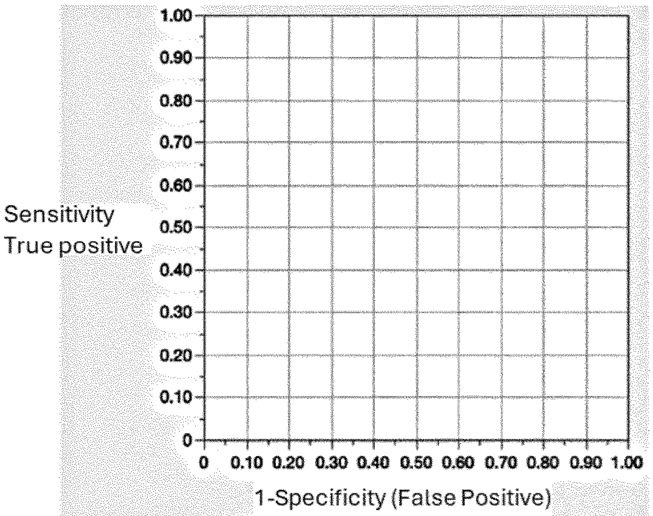


Outcome = Non-PD is treated as positive.

| AUC |
|---|
| 1.00000 |

Overall model test

| Model | (-1)*Log-likeligood | Degree of freedom | ChiSq | P value (Prob > ChiSq) |
|---|---|---|---|---|
| Difference | 208.37580 | 47 | 416.7516 | <.0001* |
| Full | 3.88536e-9 | | | |
| Reduced | 208.37580 | | | |

| | |
|---|---|
| ChiSq(U) | 1.0000 |
| AICc | 114.593 |
| BIC | 274.1 |
| Observations (or sum of weights) | 302 |

| Factor | Number of parameters | Degree of freedom | Wald Chi-Sq | P value (Prob > ChiSq) |
|---|---|---|---|---|
| LAIR2 | 1 | 1 | 79.139061 | <.0001* |
| YWHAQ | 1 | 1 | 54.732936 | <.0001* |
| CRY2 | 1 | 1 | 49.271334 | <.0001* |
| CXCR4 | 1 | 1 | 39.944242 | <.0001* |
| S100B | 1 | 1 | 38.710867 | <.0001* |
| UBQLN1 | 1 | 1 | 37.780637 | <.0001* |
| SH2D2A | 1 | 1 | 30.552925 | <.0001* |
| HEATR5B | 1 | 1 | 26.495888 | <.0001* |
| CLEC12A | 1 | 1 | 25.975397 | <.0001* |
| KIR2DL3 | 1 | 1 | 24.176177 | <.0001* |
| TMEM176B | 1 | 1 | 22.444913 | <.0001* |
| RPN2 | 1 | 1 | 22.209084 | <.0001* |
| HK2 | 1 | 1 | 22.199321 | <.0001* |
| CLU | 1 | 1 | 21.594782 | <.0001* |
| CD63 | 1 | 1 | 21.138569 | <.0001* |
| ZNF266 | 1 | 1 | 20.724734 | <.0001* |
| CCL2 | 1 | 1 | 19.940064 | <.0001* |
| HAVCR2 | 1 | 1 | 18.264705 | <.0001* |
| CLEC4F | 1 | 1 | 18.026442 | <.0001* |
| CUL5 | 1 | 1 | 17.554004 | <.0001* |
| MGAT1 | 1 | 1 | 17.157644 | <.0001* |
| CCAR1 | 1 | 1 | 16.605943 | <.0001* |
| FAM91A1 | 1 | 1 | 16.336978 | <.0001* |
| HERC3 | 1 | 1 | 16.102443 | <.0001* |
| PRF1 | 1 | 1 | 15.991493 | <.0001* |
| EGR2 | 1 | 1 | 15.845518 | <.0001* |
| TMEM176A | 1 | 1 | 15.208001 | <.0001* |
| SH2D1B | 1 | 1 | 14.61852 | 0.0001* |
| FGL2 | 1 | 1 | 14.170948 | 0.0002* |
| FOLR3 | 1 | 1 | 13.499617 | 0.0002* |
| FOXO1 | 1 | 1 | 10.036688 | 0.0015* |
| STX12 | 1 | 1 | 9.5945964 | 0.0020* |
| RNF14 | 1 | 1 | 9.5016978 | 0.0021* |
| ANXA1 | 1 | 1 | 9.0440517 | 0.0026* |
| STAT1 | 1 | 1 | 7.3363453 | 0.0068* |
| SH2B3 | 1 | 1 | 7.208169 | 0.0073* |
| SIDT2 | 1 | 1 | 7.1966826 | 0.0073* |
| SELL | 1 | 1 | 6.7416998 | 0.0094* |
| GTF2B | 1 | 1 | 6.4420754 | 0.0111* |
| IL1RN | 1 | 1 | 5.7409337 | 0.0166* |
| RSRP1 | 1 | 1 | 5.4281119 | 0.0198* |
| PSAP | 1 | 1 | 5.3236497 | 0.0210* |
| UBE2F | 1 | 1 | 5.1570615 | 0.0232* |
| CD52 | 1 | 1 | 5.0546305 | 0.0246* |
| TOPORS | 1 | 1 | 4.9223077 | 0.0265* |
| GTF3C1 | 1 | 1 | 4.6281824 | 0.0315* |
| C5AR1 | 1 | 1 | 4.0179752 | 0.0450* |

FIG. 20

Factor Selection

- 27 genes (LASSO regression) with only P<0.0001

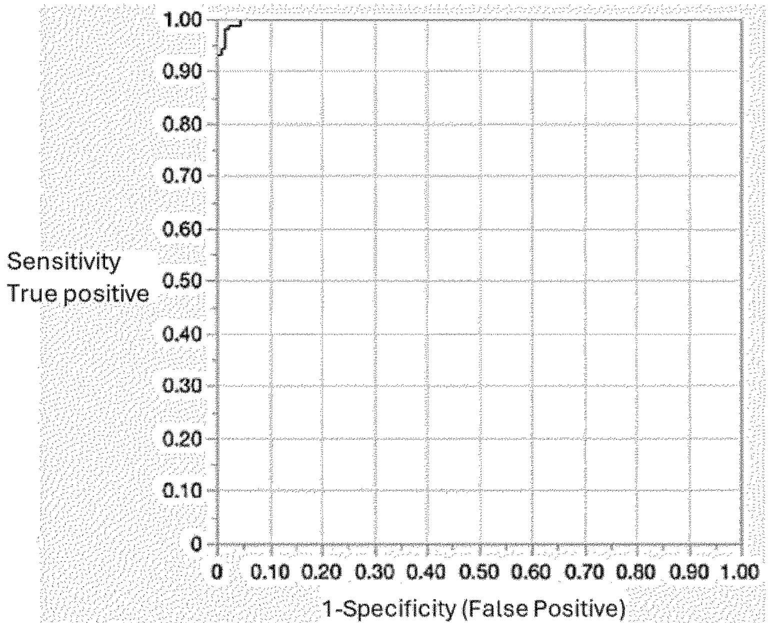


Outcome = Non-PD is treated as positive.

**AUC**

0.99872

Overall model test

| Model | (-1)*Log-likeligood | Degree of freedom | ChiSq | P value (Prob > ChiSq) |
|---|---|---|---|---|
| Difference | 195.03682 | 27 | 390.0736 | <.0001* |
| Full | 13.33898 | | | |
| Reduced | 208.37580 | | | |

| | |
|---|---|
| ChiSq(U) | 0.9360 |
| AICc | 88.6267 |
| BIC | 186.57 |
| Observations (or sum of weights) | 302 |

| Factor | Number of parameters | Degree of freedom | Wald Chi-Sq | P value (Prob > ChiSq) |
|---|---|---|---|---|
| LAIR2 | 1 | 1 | 79.139061 | <.0001* |
| YWHAQ | 1 | 1 | 54.732936 | <.0001* |
| CRY2 | 1 | 1 | 49.271334 | <.0001* |
| CXCR4 | 1 | 1 | 39.944242 | <.0001* |
| S100B | 1 | 1 | 38.710867 | <.0001* |
| UBQLN1 | 1 | 1 | 37.780637 | <.0001* |
| SH2D2A | 1 | 1 | 30.552925 | <.0001* |
| HEATR5B | 1 | 1 | 26.495888 | <.0001* |
| CLEC12A | 1 | 1 | 25.975397 | <.0001* |
| KIR2DL3 | 1 | 1 | 24.176177 | <.0001* |
| TMEM176B | 1 | 1 | 22.444913 | <.0001* |
| RPN2 | 1 | 1 | 22.209084 | <.0001* |
| HK2 | 1 | 1 | 22.199321 | <.0001* |
| CLU | 1 | 1 | 21.594782 | <.0001* |
| CD63 | 1 | 1 | 21.138569 | <.0001* |
| ZNF266 | 1 | 1 | 20.724734 | <.0001* |
| CCL2 | 1 | 1 | 19.940064 | <.0001* |
| HAVCR2 | 1 | 1 | 18.264705 | <.0001* |
| CLEC4F | 1 | 1 | 18.026442 | <.0001* |
| CUL5 | 1 | 1 | 17.554004 | <.0001* |
| MGAT1 | 1 | 1 | 17.157644 | <.0001* |
| CCAR1 | 1 | 1 | 16.605943 | <.0001* |
| FAM91A1 | 1 | 1 | 16.336978 | <.0001* |
| HERC3 | 1 | 1 | 16.102443 | <.0001* |
| PRF1 | 1 | 1 | 15.991493 | <.0001* |
| EGR2 | 1 | 1 | 15.845518 | <.0001* |
| TMEM176A | 1 | 1 | 15.208001 | <.0001* |

FIG. 21

# 302 cells (QC pass)

## Factor Selection

- 45 genes (Elastic net regression)

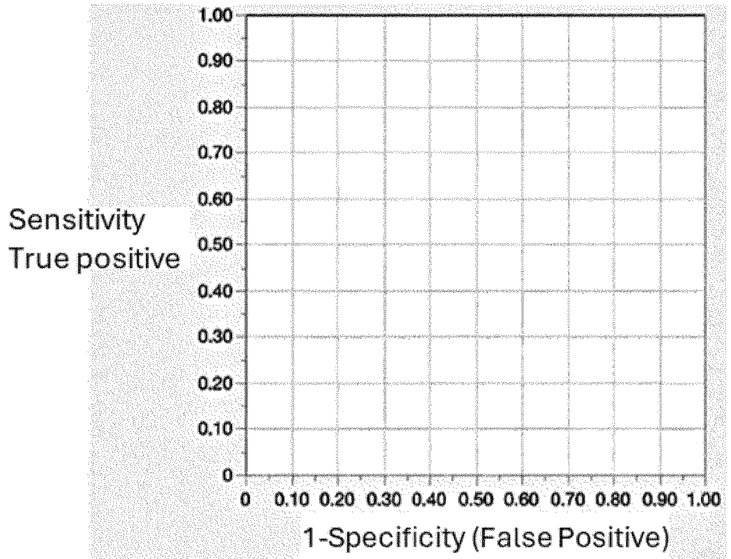


Outcome = Non-PD is treated as positive.

| AUC |
|---|
| 1.00000 |

**Overall model test**

| Model | (-1)*Log-likeligood | Degree of freedom | ChiSq | P value (Prob > ChiSq) |
|---|---|---|---|---|
| Difference | 208.37580 | 45 | 416.7516 | <.0001* |
| Full | 4.20893e-9 | | | |
| Reduced | 208.37580 | | | |

| | |
|---|---|
| ChiSq(U) | 1.0000 |
| AICc | 108.957 |
| BIC | 262.68 |
| Observations (or sum of weights) | 302 |

| Factor | Number of parameters | Degree of freedom | Wald Chi-Sq | P value (Prob > ChiSq) |
|---|---|---|---|---|
| LAIR2 | 1 | 1 | 59.826616 | <.0001* |
| YWHAQ | 1 | 1 | 42.119244 | <.0001* |
| CRY2 | 1 | 1 | 35.588035 | <.0001* |
| S100B | 1 | 1 | 31.849869 | <.0001* |
| CXCR4 | 1 | 1 | 25.682183 | <.0001* |
| UBQLN1 | 1 | 1 | 25.664738 | <.0001* |
| SH2D2A | 1 | 1 | 24.791586 | <.0001* |
| KIR2DL3 | 1 | 1 | 21.456172 | <.0001* |
| CLEC12A | 1 | 1 | 19.169614 | <.0001* |
| CLU | 1 | 1 | 18.931096 | <.0001* |
| CD63 | 1 | 1 | 18.560726 | <.0001* |
| HEATR5B | 1 | 1 | 18.182978 | <.0001* |
| HK2 | 1 | 1 | 17.981712 | <.0001* |
| TMEM176B | 1 | 1 | 17.028167 | <.0001* |
| CUL5 | 1 | 1 | 16.248564 | <.0001* |
| CLEC4F | 1 | 1 | 15.954007 | <.0001* |
| CCL2 | 1 | 1 | 15.62412 | <.0001* |
| PRF1 | 1 | 1 | 14.86745 | 0.0001* |
| RPN2 | 1 | 1 | 14.317199 | 0.0002* |
| ZNF266 | 1 | 1 | 12.910292 | 0.0003* |
| MGAT1 | 1 | 1 | 12.680645 | 0.0004* |
| FAM91A1 | 1 | 1 | 12.399838 | 0.0004* |
| HERC3 | 1 | 1 | 11.961984 | 0.0005* |
| SH2D1B | 1 | 1 | 11.629843 | 0.0006* |
| EGR2 | 1 | 1 | 11.321417 | 0.0008* |
| TMEM176A | 1 | 1 | 11.252658 | 0.0008* |
| CCAR1 | 1 | 1 | 11.157232 | 0.0008* |
| FOLR3 | 1 | 1 | 10.566175 | 0.0012* |
| FGL2 | 1 | 1 | 10.488524 | 0.0012* |
| RNF14 | 1 | 1 | 9.4314867 | 0.0021* |
| HAVCR2 | 1 | 1 | 9.1872965 | 0.0024* |
| FOXO1 | 1 | 1 | 8.5948002 | 0.0034* |
| ANXA1 | 1 | 1 | 7.6561587 | 0.0057* |
| SIDT2 | 1 | 1 | 7.6385538 | 0.0057* |
| STX12 | 1 | 1 | 7.4751469 | 0.0063* |
| GTF2B | 1 | 1 | 6.626159 | 0.0100* |
| SELL | 1 | 1 | 6.5315042 | 0.0106* |
| SH2B3 | 1 | 1 | 6.4606509 | 0.0110* |
| STAT1 | 1 | 1 | 5.6225024 | 0.0177* |
| IL1RN | 1 | 1 | 5.1857884 | 0.0228* |
| C5AR1 | 1 | 1 | 4.4981957 | 0.0339* |
| GTF3C1 | 1 | 1 | 4.3143297 | 0.0378* |
| RSRP1 | 1 | 1 | 4.053334 | 0.0441* |
| CD52 | 1 | 1 | 4.0279023 | 0.0448* |
| UBE2F | 1 | 1 | 3.922616 | 0.0476* |

FIG. 22

Factor Selection

- 17 genes (Elastic net regression) with p<0.0001

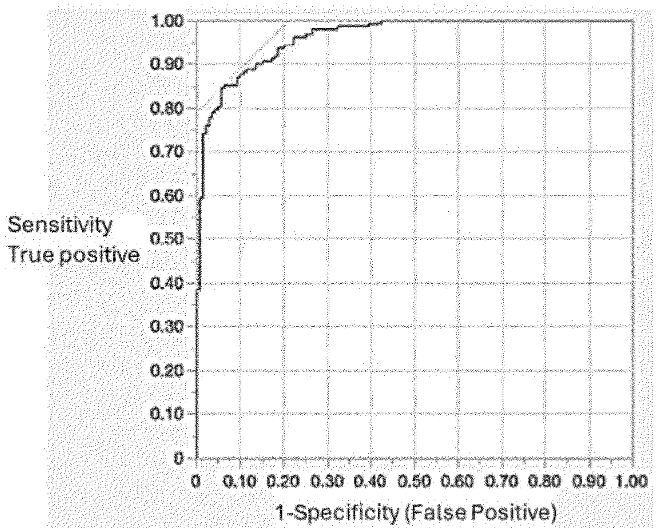


Outcome = Non-PD is treated as positive.

AUC
0.96248

Overall model test

| Model | (-1)*Log-likeligood | Degree of freedom | ChiSq | P value (Prob > ChiSq) |
|---|---|---|---|---|
| Difference | 135.03330 | 17 | 270.0666 | <.0001* |
| Full | 73.34250 | | | |
| Reduced | 208.37580 | | | |

| | |
|---|---|
| ChiSq(U) | 0.6480 |
| AICc | 185.102 |
| BIC | 249.473 |
| Observations (or sum of weights) | 302 |

| Factor | Number of parameters | Degree of freedom | Wald Chi-Sq | P value (Prob > ChiSq) |
|---|---|---|---|---|
| LAIR2 | 1 | 1 | 59.826616 | <.0001* |
| YWHAQ | 1 | 1 | 42.119244 | <.0001* |
| CRY2 | 1 | 1 | 35.588035 | <.0001* |
| S100B | 1 | 1 | 31.849869 | <.0001* |
| CXCR4 | 1 | 1 | 25.682183 | <.0001* |
| UBQLN1 | 1 | 1 | 25.664738 | <.0001* |
| SH2D2A | 1 | 1 | 24.791586 | <.0001* |
| KIR2DL3 | 1 | 1 | 21.456172 | <.0001* |
| CLEC12A | 1 | 1 | 19.169614 | <.0001* |
| CLU | 1 | 1 | 18.931096 | <.0001* |
| CD63 | 1 | 1 | 18.560726 | <.0001* |
| HEATR5B | 1 | 1 | 18.182978 | <.0001* |
| HK2 | 1 | 1 | 17.981712 | <.0001* |
| TMEM176B | 1 | 1 | 17.028167 | <.0001* |
| CUL5 | 1 | 1 | 16.248564 | <.0001* |
| CLEC4F | 1 | 1 | 15.954007 | <.0001* |
| CCL2 | 1 | 1 | 15.62412 | <.0001* |

FIG. 23

## INTERNATIONAL SEARCH REPORT

International application No.
**PCT/JP2024/011202**

**A. CLASSIFICATION OF SUBJECT MATTER**

***C12Q 1/6851***(2018.01)i; ***A61K 39/395***(2006.01)i; ***A61K 45/00***(2006.01)i; ***A61P 35/00***(2006.01)i; ***C07K 16/28***(2006.01)i; ***C12Q 1/686***(2018.01)i; ***C12Q 1/6869***(2018.01)i; ***G01N 33/50***(2006.01)i; ***G16H 20/10***(2018.01)i
FI: C12Q1/6851 Z; C12Q1/686 Z; C12Q1/6869 Z; A61K45/00; A61P35/00; A61K39/395 T; A61K39/395 U; A61K39/395 E; G01N33/50 P; G16H20/10; C07K16/28

According to International Patent Classification (IPC) or to both national classification and IPC

**B. FIELDS SEARCHED**

Minimum documentation searched (classification system followed by classification symbols)

C12Q1/6851; A61K39/395; A61K45/00; A61P35/00; C07K16/28; C12Q1/686; C12Q1/6869; G01N33/50; G16H20/10

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | WO 2023/001874 A1 (ASYLIA DIAGNOSTICS BV) 26 January 2023 (2023-01-26) claims | 1-13 |
| X | WIESWEG, M. et al. Machine learning-based predictors for immune checkpoint inhibitor therapy of non-small-cell lung cancer. Annals of Oncology. 2019, vol. 30, issue 4, pp. 655-657 entire text | 1-13 |
| X | AHMED, Y. B. et al. Genomic and transcriptomic predictors of response to immune checkpoint inhibitors in melanoma patients: A machine learning approach. Cancers. 2022, vol. 14, article 5605 summary | 1-13 |
| X | POLANO, M. et al. A pan-cancer approach to predict responsiveness to immune checkpoint inhibitors by machine learning. Cancers. 2019, vol. 11, article 1562 summary, 4.2 | 1-13 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **15 May 2024** | **28 May 2024** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (July 2022)

**INTERNATIONAL SEARCH REPORT**

International application No.
**PCT/JP2024/011202**

**C. DOCUMENTS CONSIDERED TO BE RELEVANT**

| Category* | Citation of document, with indication, where appropriate, of the relevant passages | Relevant to claim No. |
|---|---|---|
| A | JP 2022-538924 A (INTELLEXON GMBH) 06 September 2022 (2022-09-06) | 1-13 |
| A | JP 2022-518249 A (GENINUS INC.) 14 March 2022 (2022-03-14) | 1-13 |

Form PCT/ISA/210 (second sheet) (July 2022)

**INTERNATIONAL SEARCH REPORT**
**Information on patent family members**

International application No.
**PCT/JP2024/011202**

| Patent document cited in search report | Publication date (day/month/year) | Patent family member(s) | Publication date (day/month/year) |
|---|---|---|---|
| WO 2023/001874 A1 | 26 January 2023 | (Family: none) | |
| JP 2022-538924 A | 06 September 2022 | US 2022/0316014 A1 | |
| | | WO 2021/005002 A1 | |
| JP 2022-518249 A | 14 March 2022 | US 2022/0081727 A1 | |
| | | WO 2020/149523 A1 | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2019230919 A
- WO 2018231772 A
- US 9260753 B